(19)

Europäisches
Patentamt

European
Patent Office

Office européen
des brevets

(11) **EP 4 693 325 A1**

(12) **EUROPEAN PATENT APPLICATION**

(43) Date of publication:
**11.02.2026 Bulletin 2026/07**

(21) Application number: **25194155.5**

(22) Date of filing: **05.08.2025**

(51) International Patent Classification (IPC):
**G16H 20/17** *(2018.01)* **A61M 5/142** *(2006.01)*
**A61M 5/172** *(2006.01)* A61B 5/00 *(2006.01)*

(52) Cooperative Patent Classification (CPC):
**G16H 20/17; A61M 5/142; A61M 5/14244;
A61M 5/1723;** A61B 5/14503; A61B 5/14532;
A61M 5/16886; A61M 2005/14208;
A61M 2005/14268; A61M 2005/2026; G16H 40/63

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **07.08.2024 US 202463680325 P
28.07.2025 US 202519282335**

(71) Applicant: **Medtronic MiniMed, Inc.
Northridge, CA 91325-1219 (US)**

(72) Inventors:
• **TROCK, Adam S.
Northridge, 91325 (US)**
• **TIECK, Ruth M.
Northridge, 91325 (US)**
• **DELIJANI, Fatemeh
Northridge, 91325 (US)**

(74) Representative: **Zimmermann & Partner
Patentanwälte mbB
P.O. Box 330 920
80069 München (DE)**

(54) **METHOD OF BASAL, TEMP BASAL, AND SQUARE BOLUS DELIVERY FOR INCREASED
ENERGY EFFICIENCY AND PREDICTABILITY**

(57)      A processor-implemented method comprises determining a requested delivery rate of a request for fluid delivery using a fluid delivery device; determining a corresponding number of delivery events per hour for the request based on a range that the requested delivery rate falls in; determining, based on the requested delivery rate and the number of delivery events, a delivery volume of each delivery event of the number of delivery events and an event interval between adjacent deliver events of the number of delivery events; and causing performance of the number of delivery events according to the delivery volume and the event interval.

**EP 4 693 325 A1**

## Description

CROSS-REFERENCE TO RELATED APPLICATION

This application claims the benefit of and priority to U.S. Provisional Application No. 63/680,325, filed August 7, 2024, entitled "METHOD OF BASAL, TEMP BASAL, AND SQUARE BOLUS DELIVERY FOR IN-CREASED ENERGY EFFICIENCY AND PREDICT-ABILITY," which is assigned to the assignee hereof and is hereby incorporated by reference in its entirety for all purposes.

TECHNICAL FIELD

Techniques disclosed herein relate generally to fluid delivery devices, such as insulin pumps.

BACKGROUND

Fluid delivery devices are ubiquitously used for delivering medication to patients. Fluid delivery devices may include syringes, pens, pumps, and the like. For example, a fluid delivery pump may include a pump drive system that may include a motor and drive train components (e.g., including a slide and/or a sleeve). The drive train components may convert rotational motor motion to a translational displacement of a plunger (or stopper) in a fluid reservoir that holds medication fluid. In this way, the medication fluid may be displaced from the reservoir to a patient's body via a fluid path between the reservoir and the body of a patient, such as a flexible tube and/or a cannular that is at least partially inserted into the user. Many fluid delivery pumps may be portable or wearable, and thus may be powered by batteries. In one example, a fluid delivery device (e.g., an insulin pump) may be worn by a diabetes patient to automatically or semi-automatically administer insulin to the patient.

The pancreas of a healthy person can produce and release insulin into the blood stream in response to elevated blood glucose levels. More specifically, beta cells ($\beta$-cells) in the pancreas can produce and secrete insulin into the blood stream as needed. If $\beta$-cells become incapacitated or die, a condition known as Type 1 diabetes mellitus, insulin may need to be provided to the diabetic patient's body using, for example, a syringe, a pen, or an infusion pump of a blood glucose level management system, to maintain health or life. Some glucose level management systems may require manual administration of insulin based on dosage determined by an insulin calculator. Some glucose level management systems may include an insulin pump controlled to deliver insulin to the patient automatically or semi-automatically. For example, an insulin pump may be operated in a mode where basal insulin is delivered at a fixed rate or a variable rate determined for the user based on, for example, glucose level measurements obtained from a patient-worn glucose sensor, such as a continuous glucose monitoring (CGM) sensor. An insulin pump may also be used to control the delivery of meal boluses to account for meal intake, and/or the delivery of boluses to account for rising glucose trends, a sudden spike in detected glucose level, and the like. Therefore, such a glucose level management system that includes a CGM sensor and an insulin pump may monitor and regulate a user's blood or interstitial glucose level in a substantially continuous and autonomous manner, for example, overnight while the patient is sleeping.

SUMMARY

This disclosure relates generally to fluid delivery devices, such as insulin pumps. The techniques disclosed herein may be practiced in a variety of ways, such as using a processor-implemented method, a system comprising one or more processors and one or more processor-readable media, and/or one or more (non-transitory) processor-readable media.

According to certain embodiments, a processor-implemented method may include determining a requested delivery rate of a request for fluid delivery using a fluid delivery device; determining a corresponding number of delivery events per hour for the request based on a range that the requested delivery rate falls in; determining, based on the requested delivery rate and the number of delivery events, a delivery volume of each delivery event of the number of delivery events and an event interval between adjacent deliver events of the number of delivery events; and causing performance of the number of delivery events according to the delivery volume and the event interval.

According to certain embodiments, a system may include one or more processors and one or more processor-readable storage media storing instructions, which, when executed by the one or more processors, cause performance of operations comprising: determining a requested delivery rate of a request for fluid delivery; determining a corresponding number of delivery events per hour for the request based on a range that the requested delivery rate falls in; determining, based on the requested delivery rate and the number of delivery events, a delivery volume of each delivery event of the number of delivery events and an event interval between adjacent deliver events of the number of delivery events; and causing performance of the number of delivery events according to the delivery volume and the event interval.

According to certain embodiments, a fluid delivery system may include a reservoir including a plunger and barrel for storing fluid, a drive system including a motor and configured to linearly translate the plunger, a motor position sensor configured to measure positions of the motor, one or more processors electrically coupled to the motor and the motor position sensor, and one or more processor-readable storage media. The one or more processor-readable storage media store instructions which, when executed by the one or more processors, cause the one or more processors to perform operations including: determining a requested delivery rate of a request for fluid delivery; determining a corresponding number of delivery events per hour for the request based on a range that the requested delivery rate falls in; determining, based on the requested delivery rate and the number of delivery events, a delivery volume of each delivery event of the number of delivery events and an event interval between adjacent deliver events of the number of delivery events; and causing performance of the number of delivery events according to the delivery volume and the event interval.

Further disclosed herein is a processor-implemented method comprising determining a requested delivery rate of a request for fluid delivery using a fluid delivery device; determining a corresponding number of delivery events per hour for the request based on a range that the requested delivery rate falls in; determining, based on the requested delivery rate and the number of delivery events, a delivery volume of each delivery event of the number of delivery events and an event interval between adjacent deliver events of the number of delivery events; and causing performance of the number of delivery events according to the delivery volume and the event interval.

This summary is provided to introduce a selection of concepts in a simplified form that are further described below in the detailed description. This summary is not intended to identify key features or essential features of the claimed subject matter, nor is it intended to be used as an aid in determining the scope of the claimed subject matter.

BRIEF DESCRIPTION OF THE DRAWINGS

Illustrative embodiments are described in detail below with reference to the following figures.

FIG. 1 illustrates an example of a glucose level management system according to certain embodiments.

FIG. 2 is a block diagram of an example of a glucose level management system according to certain embodiments.

FIG. 3 illustrates an example of a fluid delivery device suitable for use in a glucose level management system according to certain embodiments.

FIG. 4 is an exploded perspective view of an example of the fluid delivery device of FIG. 4 according to certain embodiments.

FIG. 5 is a cross-sectional view of the fluid delivery device of FIG. 4 with a reservoir inserted in the fluid delivery device.

FIG. 6 is a block diagram of an example of an infusion system according to certain embodiments.

FIG. 7 includes a flowchart illustrating an example of a process of calibrating and utilizing a fluid delivery device according to certain embodiments.

FIG. 8 includes a flowchart illustrating an example of a process of manufacturing calibration of a fluid delivery device according to certain embodiments.

FIG. 9 includes a flowchart illustrating an example of a process of determining a baseline coefficient of variation of motor rotation time with no load applied in a fluid delivery system according to certain embodiments.

FIG. 10 includes a flowchart illustrating an example of a process of detecting a plunger of a reservoir in a fluid delivery system according to certain embodiments.

FIG. 11 includes a flowchart illustrating an example of a process of measuring and compensating friction between a plunger and a barrel of a fluid reservoir in a fluid delivery system according to certain embodiments.

FIG. 12 includes a flowchart illustrating an example of a process of determining a baseline coefficient of variation of delivery rate of a fluid delivery device according to certain embodiments.

FIG. 13 includes a flowchart illustrating an example of a process of scheduling and delivering fluid using a fluid delivery system according to certain embodiments.

FIG. 14 includes a flowchart illustrating an example of a process of controlling a fluid delivery system for fluid delivery according to certain embodiments.

FIG. 15 includes a flowchart illustrating an example of a process of determining the stroke size for the next stroke based on the remaining amount of fluid to be delivered according to certain embodiments.

FIG. 16 includes a flowchart illustrating an example of a process of scheduling and delivering basal insulin using a fluid delivery system according to certain embodiments.

FIG. 17 includes a flowchart illustrating an example of a process of scheduling and delivering temporary basal insulin using a fluid delivery system according to certain embodiments.

FIG. 18 includes a flowchart illustrating an example of a process of scheduling and delivering square bolus insulin using a fluid delivery system according to certain embodiments.

FIG. 19A is a diagram illustrating an example of selecting the number of delivery events for a square bolus based on the total amount of the square bolus according to certain embodiments.

FIG. 19B illustrates an example of delivering an amount of square bolus in four events within one hour according to some examples.

FIG. 20 includes a flowchart illustrating an example of a process of detecting occlusion during fluid delivery using a fluid delivery system according to certain embodiments.

FIG. 21 includes a flowchart illustrating an example of a process of detecting occlusion of a fluid delivery system while the operating temperature changes according to certain embodiments.

FIG. 22 includes a graph illustrating examples of parameters used in an example of a process of detecting occlusion of a fluid delivery system while the operating temperature changes according to certain embodiments.

FIG. 23 is a block diagram of an example of an electronic device that may implement some of the examples disclosed herein.

The figures depict embodiments of the present disclosure for purposes of illustration only. One skilled in the art will readily recognize from the following description that alternative embodiments of the structures and methods illustrated may be employed without departing from the principles, or benefits touted, of this disclosure.

In the appended figures, similar components and/or features may have the same reference label. Further, various components of the same type may be distinguished by following the reference label by a dash and a second label that distinguishes among the similar components. If only the first reference label is used in the specification, the description is applicable to any one of the similar components having the same first reference label irrespective of the second reference label.

DETAILED DESCRIPTION

This disclosure relates generally to fluid delivery devices (e.g., insulin pumps) and methods of calibrating and operating the fluid delivery devices. For purposes of illustration and explanation, various examples described in this disclosure are related to insulin delivery. However, techniques disclosed herein can be used in fluid delivery systems for automatically or semi-automatically delivering other types of fluid, such as other types of medication, and thus are not limited to insulin delivery using insulin pumps.

Diabetes mellitus is a disease of the glucose regulatory system of a patient, where the naturally produced insulin in the body may not be sufficient to control the glucose level in the blood stream of the patient due to insufficient production of insulin and/or insulin resistance. Therefore, a diabetic patient may need to receive insulin from a pump or another delivery device, such as an injection or infusion device (e.g., a syringe or pen), to control the glucose level in the patient's blood stream. A diabetic patient's therapy routine may generally include dosages of basal insulin and bolus insulin. Basal insulin, also referred to as background insulin, may include continuous or constant release of small amounts of insulin to keep blood glucose levels at consistent levels during long time periods. Bolus insulin may be administered, for example, before, at, or after mealtimes or other times where there may be a rapid increase in the blood glucose level. The dose of insulin to be delivered may be determined based on, for example, the carbohydrate count of a meal, and/or the glucose levels of the patient measured using a glucose monitor, such as a fingerstick blood glucose measurement device or a continuous glucose monitoring (CGM) sensor. The insulin dose may also be influenced by, for example, measurements of a physiological condition (e.g., temperature) of the patient, and activities of the patient (e.g., exercises, food intake activities, etc.). Thus, the rate of infusion can be pre-determined, or may vary as the difference between a current measurement value and the target measurement value fluctuates. In a closed-loop or automatic operating mode, dosage commands may be generated based on a difference between a current (or most recent) measurement of the interstitial fluid glucose level in the body of the user and a target (or reference) glucose setpoint value, and may be generated in an automated manner in accordance with the delivery control scheme associated with a particular operating mode.

It is desirable that the insulin delivered, including the basal insulin and bolus insulin, can maintain a diabetes patient's glucose level within a desired range. Managing the patient's glucose level may be complicated by variations in, for example, the patient's individual physiological response to insulin and various types of food, and potentially other factors, such as the patient's daily activities (e.g., exercise). Thus, the delivery of insulin to the patient may need to be precisely controlled to occur at time instants and in amounts that are determined based on, for example, the amount of carbohydrates taken by a patient and/or real-time measurements of glucose levels (e.g., interstitial or blood glucose level) by a glucose sensor, such as a CGM sensor.

A fluid delivery device for automatically or semi-automatically delivering fluid to a patient may include a motor and/or other actuation arrangements that are operable to linearly displace a plunger (or stopper) of a fluid reservoir provided within the fluid delivery device to deliver a dosage of fluid, such as insulin, to the body of the patient. The fluid delivery device, such as an insulin pump, may be worn by a user to deliver fluid (e.g., insulin) in a substantially continuous and autonomous manner, for example, overnight while the patient is sleeping. The fluid reservoir may be disposable, replaceable, or refillable, whereas other portions of the fluid delivery device may be reuseable. Different fluid reservoirs may have different frictions between the plunger and the barrel, and may be filled with fluid of different volumes. The drive system, such as the motor and the drive train components, in different fluid delivery devices may also have different performance. Therefore, when a new or refilled reservoir is placed into the fluid delivery device, the volume of the fluid in the reservoir may need to be measured, and the fluid delivery device may need to be calibrated to compensate for variations in the drive system and reservoir, such as determining the appropriate drive voltage level and/or drive signal duty cycle and the corresponding delivery speed, in order to precisely control the amount of fluid delivered to the user. In addition, since many fluid delivery devices such as insulin pumps may be portable and wearable and may generally be powered by batteries, it may be desirable that the fluid delivery device (e.g., an insulin pump) employs a safe, reliable, accurate, power efficient, and relatively simple and fast calibration and control schemes.

An insulin pump may utilize a motor for controlled delivery of insulin. To precisely control the dosing of insulin therapy and to make the insulin pump energy efficient for longer battery life, the motor may be controlled to perform pulsatile operations as opposed to continuous motion. For example, some insulin pumps may utilize a set of motor stroke sizes (e.g., 3 different stroke sizes), where each stroke may be used for delivering a set amount of insulin. The amount of insulin in each request for insulin delivery may be divided into one or more strokes. In each stroke, the motor may be driven at a constant voltage for a specified amount of time and then allowed to coast to a stop. In some examples, the drive time for each stroke may be adjusted based on the feedback from a motor position sensor to compensate for under or over delivery that may occur due to, for example, changes in fluidic back pressure and drive system friction. This driving technique may result in energy inefficiency due to, for example, a combination of short motor on times (which may result in high peak motor power) and long processor and sensor on times for each stroke (e.g., in order to capture the long coasting motion). In addition, this driving technique may result in variations of insulin delivery (and therefore errors) from stroke to stroke due to the indirect relationship between the motor drive time and the amount of insulin delivered. Furthermore, additional force, strain, or pressure sensors (which may be collectively referred to herein as strain sensors) may need to be used to detect the back-pressure generated from the fluid in the fluid reservoir, in order to detect the initial plunger position (and the amount of insulin in a reservoir) and possible occlusion during the fluid delivery. The inclusion of the additional strain sensor(s) in a fluid delivery device may significantly increase the design and control complexity, increase the cost, and reduce the reliability of the infusion pump due to, for example, the sensor fragility.

According to certain embodiments disclosed herein, the motor of a fluid delivery device may be controlled to perform pulsatile motor operations, where the set of stroke sizes may include many (e.g., more than 10 or more than 20) different stroke sizes. Each stroke may include a plurality of steps, where the number of steps in the stroke may be selected from many different values, depending on the maximum stroke size used by the fluid delivery device and the amount of fluid to be delivered using the stroke. Rather than controlling the different delivery amounts based on different motor drive time durations, step-by-step position control may be utilized to more precisely control the delivery amount. Active motor braking (instead of coasting) may also be used to improve energy efficiency of the motor, position sensor, and controller. The motor and other components of the drive system of each fluid delivery device may be driven at a specific voltage level that is calibrated for a specific stall load during production and further tuned for a specific reservoir during reservoir setup. These techniques allow plunger detection and occlusion detection to be performed using the motor speed (or rotation time and/or delivery rate) measured using motor position sensors that are also used for other purposes, rather than using the additional force, strain, or pressure sensors. As such, the driving techniques disclosed herein may significantly reduce energy consumption, improve delivery accuracy, and enable the detection of both the plunger during reservoir setup and possible occlusion during insulin delivery using a motor position sensor instead of an additional strain sensor. As such, the fluid delivery device may have a longer battery life, may use a smaller battery, or may use more energy for functions other than driving the motor. Improved stroke to stroke accuracy may result in more predictable therapy. The ability to detect both the plunger during reservoir setup and possible occlusion during insulin delivery using the motor position sensor instead of an additional pressure sensor may result in reduced mechanical design and control complexity, improved product reliability, and reduced product cost.

An insulin pump may use an insulin reservoir or cartridge for storing insulin to be delivered. Some insulin pump may integrate the reservoir within the body of the insulin pump, and thus may be disposable after the reservoir is empty. But many insulin pumps are reusable pumps, where the insulin reservoir may be replaceable (or refillable) by a user, such that the reservoir may be replaced after the patient consumes all insulin in the reservoir (e.g., within 3-7 days) or after the reservoir has been in the insulin pump for a long time period, to provide continuous insulin delivery and to prevent insulin degradation or potential infection. Many other portions of the insulin pump, such as the housing, the controller, the motor, the drive train components, the user interface, and the communication interface, may be reused to reduce the overall cost in a long term.

Many different types of insulin reservoirs or cartridges may be used in insulin pumps. For example, some insulin pumps may use syringe-type reservoirs that may include a hollow plastic barrel with one open end, a septum on the closed end, and a plunger (also referred to as a stopper) with rubber O-rings within the barrel. The plunger may be pushed linearly by the motor and the drive train of the insulin pump from the open end, and the linear motion of the plunger may displace the fluid from the reservoir to cause fluidic delivery through a needle piercing the septum (e.g., from an infusion set). The motor may be driven to rotate around an axis, and the drive train may convert the rotational motion of the motor to linear motion of the plunger, and may include, for example, a slide for pushing the plunger and a sleeve that causes the slide to move linearly.

Different users may use different total daily doses (TDDs) of insulin, and thus may need different amounts of insulin for a same time period. As such, some reservoirs and fluid delivery devices may allow the patients to fill the reservoirs to any volume that may be suitable for them. Therefore, the insulin pumps may need to be able to automatically determine the initial location of the plunger within the reservoir barrel and the volume of the insulin stored in each reservoir during reservoir setup. Some insulin pumps may determine the fill volume of the reservoir and the plunger position in the reservoir by driving the motor forward at a high speed and measuring the resultant back pressure using a strain sensor. For example, when the slide reaches the plunger position, the back pressure may increase drastically and may be detected by the strain sensor to indicate the detection of the plunger. As discussed above, incorporating a strain sensor into an insulin pump may increase the mechanical complexity as the entire drive system may need to "float" to allow the back pressure generated by the fluid due to the pressure applied to the fluid by the slide through the plunger or the back pressure caused by an occlusion to be detected by the strain sensor. The presence of the strain sensor may also reduce the reliability of the insulin pump due to, for example, the sensor fragility and the inability to fully secure the drive system, which may cause the insulin pump to be more susceptible to damage from shock and drop. In addition, strain sensors with sufficient accuracy and resolution for plunger and occlusion detection may be expensive, and thus may significantly increase the cost of an insulin pump.

According to certain embodiments disclosed herein, the plunger detection during reservoir setup may be performed using signals from motor position sensors (e.g., Hall effect sensors) that are also used for determining the delivery amount and/or speed of fluid delivery motor, instead of using a separate strain sensor or other sensors. For example, during the reservoir setup, the motor may be driven at a voltage level determined during the factory calibration procedure, and the motor rotational speed or motor rotation time may be determined using the motor position sensor. A baseline coefficient of variation (CV) of the motor rotation time (or motor rotational speed) may be determined during a first plurality of rotations of the motor when no load is applied to the motor slide. After the baseline CV of the motor rotation time is established, subsequent motor speed or rotation time measurements may be filtered by a series of mathematical and statistical filters (e.g., a maximum value filter) to generate a moving coefficient of variation of the motor rotation time. The moving coefficient of variation may be compared against the baseline coefficient of variation to detect the plunger. For example, when the moving coefficient of variation exceeds the baseline CV by a specified amount, the plunger may be detected. In this way, plunger detection may be performed during reservoir setup without the need for a separate strain sensor or another type of sensor, thereby reducing the design and control complexity, improving the reliability, and reducing the overall cost of the fluid delivery device.

In reusable insulin pumps that use replaceable insulin reservoirs, due to the high-volume manufacturing and lower cost of insulin reservoirs, the friction between the plastic barrel and the plunger (e.g., O-rings on the plunger) may vary from reservoir to reservoir. The variation in friction may make it difficult to accurately and timely determine whether the back pressure is due to the reservoir friction or a flow blockage, and thus may make it difficult to detect an occlusion quickly and accurately. The variation in friction may also cause variation in the fluid delivery rate under a same motor drive voltage, which may affect the accuracy of the amount of insulin delivered.

According to certain embodiments disclosed herein, the variation in the friction between the plunger (and the O-rings) and the reservoir barrel may be measured and compensated during the reservoir setup, such that appropriate respective motor drive voltages and duty cycles may be used to drive the specific reservoirs to achieve a more constant or consistent delivery rate, thereby enabling more accurate and timely detection of a real occlusion based on the changes in the fluid delivery rate measured by the motor position sensors. In one example, a reference motor speed (or a reference fluid delivery rate) at no load and under a motor drive voltage level and a motor drive duty cycle that have been calibrated against a known load during manufacturing calibration of each insulin pump may be measured during the manufacturing calibration, and may be saved to the insulin pump. After an insulin reservoir is positioned in the insulin pump, the motor may be driven using a series of strokes (e.g., each including a plurality of steps). A controller may obtain data from a motor position sensor to determine a motor speed (or rotation time) or fluid delivery rate during a plurality of strokes, and compare the measured motor speed or fluid delivery rate against the reference speed or fluid delivery rate. If the measured fluid delivery rate is lower than the reference fluid delivery rate, the reservoir friction may cause the delivery rate reduction and the motor voltage may be increased to compensate for the delivery rate reduction. This process may be repeated until the measured delivery rate is equal to or greater than the reference delivery rate. The reservoir friction measurement and compensation may be performed in a small number of strokes while the tube connecting the reservoir to a cannular is being flushed. The reservoir friction measurement and compensation technique disclosed herein may allow the insulin pump to compensate for the unique friction presented by each insulin reservoir and allow more accurate and timely occlusion detection.

Fluid delivery devices such as insulin pumps may need to be able to detect flow blockage caused by occlusion in a timely manner during insulin delivery, because an occlusion would prevent the fluid from reaching the patient's subcutaneous tissue. Occlusion may cause a total loss of insulin therapy and thus may present a substantial risk to diabetic patients as they are likely to become hyperglycemic if insulin therapy is not restored in a timely manner. Thus, an insulin pump may need to be able to detect occlusion within, for example, 5 units of missed delivery of insulin, so that a notification can be provided to the patient as early as possible. As described above, some insulin pumps may use force, strain, or pressure sensors to detect the back-pressure generated from pumping insulin into an occluded infusion set, thereby inferring an occlusion based on the change of the measured back pressure. As also described above, incorporating a strain or pressure sensor into an insulin pump may increase the mechanical complexity as the entire drive system may need to "float" to allow the back pressure generated by the fluid due to the pressure applied to the fluid by the slide through the plunger or the back pressure caused by an occlusion to be detected by the strain sensor. The presence of the strain sensor may reduce the reliability of the insulin pump due to, for example, the sensor fragility and the inability to fully secure the drive system, which may cause the insulin pump to be more susceptible to damage from shock and drop. In addition, strain sensors with sufficient accuracy and resolution for plunger and occlusion detection may be significantly more expensive, and thus may significantly increase the cost of an insulin pump.

According to certain embodiments disclosed herein, occlusion of the fluid delivery device may be detected based on the fluid delivery rate measured using the motor position sensor (e.g., a Hall effect sensor) of the fluid delivery device, in combination with the calibration techniques disclosed herein. The calibration techniques may be used to compensate the variations in the motor torque constant and the drive system friction that may otherwise cause the occlusion detection sensitivity to vary widely and result in failure to meet the specified performance metrics. In one example, before the beginning of the therapy, for example, right after the friction between the plunger and a barrel of a reservoir is compensated to adjust the fluid delivery rate from the reservoir (e.g., by adjusting the motor drive voltage and/or duty cycle), motor speeds or fluid delivery rates during a plurality of strokes may be determined based on the positions of the motor measured by the motor position sensor during insulin delivery using the adjusted motor drive voltage and/or duty cycle. The measured motor speeds or fluid delivery rates may be used to generate a coefficient of variation of the delivery rates, which may be used as a baseline coefficient of variation for occlusion detection.

During fluid delivery, motor speeds or fluid delivery rates may be continuously determined based on the positions of the motor measured by the motor position sensor using the adjusted motor drive voltage and/or duty cycle. The measured motor speeds or fluid delivery rates may be filtered by a series of mathematical and statistical filters to generate a moving coefficient of variation of the delivery rates. The filters may include a minimum filter that may reduce the stroke-to-stroke variation while allowing a consistent decrease in speed or delivery rate that would occur during an occlusion to become evident. The moving coefficient of variation may be compared against the baseline coefficient of variation for occlusion detection. The results of the comparison may be scored against multiple thresholds, where the different thresholds are used to identify the probability that an actual occlusion has occurred.

For example, when the moving coefficient of variation exceeds the baseline coefficient of variation and the moving coefficient of variation continues to increase (e.g., the rate of change of the moving CV is greater than a first threshold) for a specified amount of delivery (e.g., a first threshold number of steps), an occlusion may be declared. In some examples, a pre-occlusion threshold may be used to detect a pre-occlusion condition when the moving coefficient of variation exceeds the baseline coefficient of variation and continues to increase at a lower rate (e.g., the rate of change of the moving CV is greater than a second threshold) for a specified amount of delivery (e.g., a second threshold number of steps). In some examples, after a pre-occlusion is triggered, the current rate of change of the moving CV may be compared with a previous rate of change of the moving CV, and an occlusion may be detected if the current rate of change of the moving CV is greater than the previous rate of change of the moving CV by a threshold value and the number of delivery steps during which the fluid delivery device is in the pre-occlusion state is greater than a threshold number. If the number of delivery steps during which the fluid delivery device is in the pre-occlusion state is not greater than the threshold number, an occlusion may not have occurred. In some examples, the pre-occlusion threshold may be automatically adapted to changing motor load conditions during insulin therapy. For example, if a pre-occlusion state is declared but later undeclared because the number of subsequent steps during which the pre-occlusion condition is not met is greater than a threshold number, the pre-occlusion threshold may be increased.

In some examples, the occlusion detection techniques disclosed herein may use the operating temperature of the fluid delivery system as an input to control the detection sensitivity (e.g., the threshold for comparing with the moving coefficient of variation of the filtered delivery rates), thereby avoiding false occlusion detections caused by the impact of temperature drop on the delivery rate. For example, if the operating temperature of the fluid delivery system decreases by a value greater than a threshold, the baseline coefficient of variation may be multiplied by a desensitization factor that is greater than one, and an occlusion may not be detected if the moving coefficient of variation of the filtered delivery rates is less than the product of the baseline coefficient of variation and the desensitization factor.

The occlusion detection techniques disclosed herein do not use an additional strain sensor or another type of sensor, and thus may significantly reduce the design and control complexity and cost of the insulin pump and increase the reliability of the insulin pump. The occlusion detection techniques disclosed herein may increase the occlusion detection sensitivity to enable faster and more accurate occlusion detection, and may also provide increased robustness against false occlusion detections by automatically adapting to changing motor load conditions during insulin therapy. The improved occlusion detection performance may improve the user experience and clinical outcomes.

Insulin therapy using an insulin pump may include several modes of delivery, such as bolus insulin delivery and basal insulin delivery. Bolus insulin may be delivered at a larger dose around meal time or any other time when a patient's glucose level is significantly higher than the desired target. While a typical bolus may be delivered with a series of successive strokes in rapid succession, a basal insulin delivery and a square bolus insulin delivery may be scheduled as a series of strokes over a longer time period, such as 30 minutes or longer. Insulin pumps that use a limited set of stroke sizes may need to use a complex scheduling scheme for basal delivery, and may have a higher power consumption for the delivery because most basal rates may need to be achieved using small strokes every minute or every few minutes. While the scheduling scheme may ultimately result in the correct amount of insulin being delivered, each specific basal rate may result in a different series of strokes and time intervals between strokes, which may be difficult to predict.

According to certain embodiments disclosed herein, a series of multiple quick checks may be performed to determine how many delivery events need to be performed for each basal rate (including temporary basal rate) or square bolus rate, and the insulin delivery may be evenly divided across the calculated number of delivery events. This scheduling scheme may result in a uniform and predictable series of basal deliveries, and may also greatly reduce the number of deliveries performed for a given basal rate, which allows the delivery motor to operate more efficiently. Square bolus may also be scheduled and delivered in a similar manner. In each delivery event, the insulin may be delivered in one or more strokes, where the stroke size may be more flexible to accommodate different requested amounts. The size of each stroke may be selected to match the requested amount closely and also reduce the number of strokes, such that the power consumption for the delivery may be reduced.

Aspects and embodiments of the present disclosure can be practiced with one or more types of insulin (e.g., fast-acting insulin, intermediate-acting insulin, and/or slow-acting insulin). Unless indicated otherwise by the context, terms such as "dose," "insulin," "basal," and "bolus" may not denote a particular type of insulin. For example, fast-acting insulin may be used for both basal dosages and bolus dosages. The term "basal" can refer to and include insulin that is delivered in an amount and at a frequency that is intended to correspond to a healthy body's release of insulin between meals and during sleep. The term "bolus" may refer to and include insulin that is delivered in an amount and at a timing that is intended to correspond to a healthy body's release of insulin for counteracting a high glucose level, such as that caused by the consumption of a meal. A meal may include any type or amount of food or beverage consumption, including breakfast, lunch, dinner, snacks, and beverages, among others.

In the following description, for the purposes of explanation, specific details are set forth in order to provide a thorough understanding of examples of the disclosure. However, it will be apparent that various examples may be practiced without these specific details. For example, devices, systems, structures, assemblies, methods, and other components may be shown as components in block diagram form in order not to obscure the examples in unnecessary detail. In other instances, well-known devices, processes, systems, structures, operations, and techniques may be shown without necessary detail or may not be shown, in order to avoid obscuring the examples. The figures and description are not intended to be restrictive. The terms and expressions that have been employed in this disclosure are used as terms of description and not of limitation, and there is no intention in the use of such terms and expressions of excluding any equivalents of the features shown and described or portions thereof. The word "example" is used herein to mean "serving as an example, instance, or illustration." Any embodiment or design described herein as "example" is not necessarily to be construed as preferred or advantageous over other embodiments or designs.

Carbohydrates in food intake may be broken down into glucose (sugar) in the stomach and/or intestine, and may be absorbed at the small intestine and/or large intestine into the blood stream. The blood stream may transport glucose to the capillaries of the body, where some glucose may diffuse into the interstitial fluid between cells (e.g., fat or muscle cells), which may use the glucose for energy. The endocrine system of the human body that directs and regulates the functions and activities of the body (working with the nervous system) may secrete chemicals that transmit messages to tissues and organs. For example, endocrine glands or organs may release hormones into the blood stream for transportation to target cells with receptors. In one example, insulin may be made by the β-cells of pancreas, and may, for example, increase the glucose uptake, enhance glucose utilization, stop hepatic glucose production, stimulate glycogen formation in liver and skeletal muscle, promote protein synthesis, and increase fat storage.

Insulin may function as a key that can unlock cells and help glucose to move into cells where the glucose may be used for energy. Without insulin, glucose may not be able to enter cells to be used for energy, and may build up in the interstitial fluid and blood stream. A healthy pancreas may continuously release a small amount of regular human insulin 24 hours a day, including between meals and during sleep. The small amount pf insulin may match the liver's release of glucose. A healthy pancreas may also secrete a larger amount of insulin after food intake to match the amount of food intake.

The liver of a person may absorb excessive glucose from digestion and convert excessive glucose to glycogen for storage so that glucose may be available when needed. For example, when the blood glucose level is low, the α-cells of the pancreas may secrete glucagon. Glucagon may cause the liver to release the stored form of glucose (glycogen) into the blood stream to help increase the glucose level. The balance between insulin secreted by the β-cells and the glucagon secreted by the α-cells may help to maintain the normal blood glucose level, such as in the range of about 80-120 mg/dL before meals.

The naturally produced insulin in the body of a diabetic patient may not be sufficient to control the glucose level in the blood stream of the patient, due to insufficient production of insulin and/or insulin resistance. Therefore, a diabetic patient may need to receive insulin from a pump or another delivery device such as an injection or infusion device to control the glucose level in the patient's blood stream. To control the glucose level, a diabetic patient's therapy routine may generally include dosages of basal insulin and bolus insulin. Dosages of insulin to be delivered may be determined based on, for example, the carbohydrate count of a meal, and/or the glucose level of the patient measured using a glucose monitor, such as a continuous glucose monitor (CGM).

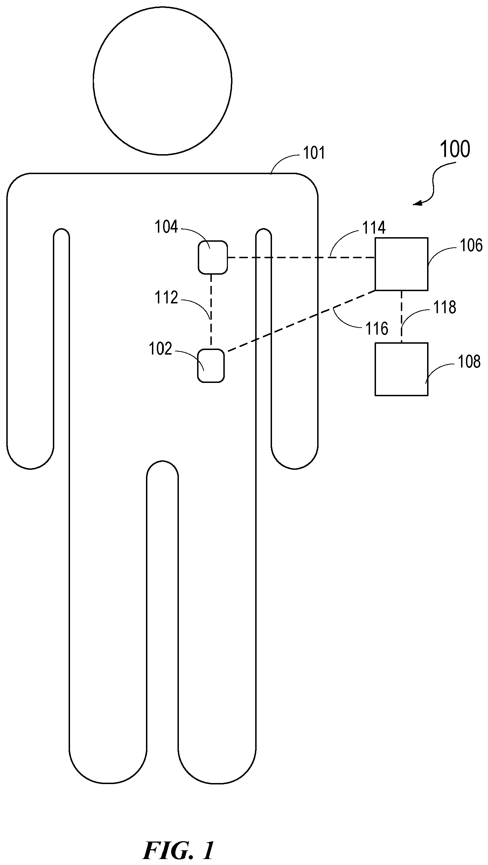

FIG. 1 illustrates an example of a glucose level management system 100 according to certain embodiments. Glucose level management system 100 may be used to monitor and regulate the blood glucose level of a patient 101. In the illustrated example, glucose level management system 100 may include a delivery device 102, a monitoring device 104, a computing device 106, and an optional remote/cloud computing system 108. Delivery device 102, monitoring device 104, and computing device 106 may be embodied in various ways, including being disposed in one or more device housings. For example, in some embodiments, all of devices 102-106 may be disposed in a single device housing. In some embodiments, each of devices 102-106 may be disposed in a separate device housing. In some embodiments, two or more of devices 102-106 may be disposed in the same device housing. In some embodiments, a single device 102, 104, or 106 may have two or more parts that are disposed in two or more housings. For example, monitoring device 104 may include an on-body part and a display and control part communicated with the on-body part through wires or wirelessly. Delivery device 102 may include an on-body site (e.g., including a cannula) and a part that includes a reservoir, a pump, and a control unit. These and other embodiments, and combinations thereof, are contemplated to be within the scope of the present disclosure.

Glucose level management system 100 may include a plurality of communication links, such as communication links 112-118. Communications links 112-118 may each be a wired connection and/or a wireless connection. In embodiments where two devices are located in a same housing, the communication link may include, for example, wires, cables, and/or communication buses on a printed circuit board, among other things. In embodiments where two devices are separate from each other in different device housings, the communication links may be wired and/or wireless connections. Wired connections may include, for example, an Ethernet connection, a Universal Serial Bus (USB) connection, and/or another type of physical connection. Wireless connections may include, for example, a cellular connection, a Wi-Fi connection, a Bluetooth® connection, a mesh network connection, and/or another type of connection using a wireless communication protocol. Some embodiments of communication links 112-118 may use direct connections, such as Bluetooth® connections, and/or may use connections that route through one or more networks or network devices (not shown), such as an Ethernet network, a Wi-Fi network, a cellular network, a satellite network, an intranet, an extranet, the Internet, and/or the Internet backbone, among other types of networks. Various combinations of wired and/or wireless connections may be used for communication links 112-118.

Delivery device 102 may be configured to deliver a therapeutic substance to patient 101. The therapeutic substance may include, for example, insulin, HIV drugs, drugs to treat pulmonary hypertension, iron chelation drugs, pain medications, anti-cancer treatments, medications, vitamins, hormones, a nutritional supplement, a dye, a tracing medium, a saline medium, a hydration medium, and the like. Delivery device 102 may be secured to patient 101 (e.g., to the body or clothing of patient 101) or may be at least partially implanted in the body of patient 101. In some embodiments, the delivery device 102 may include a reservoir, an actuator, a delivery mechanism, and a cannula (not shown). The reservoir may be configured to store an amount of the therapeutic substance. In some embodiments, the reservoir may be refillable or replaceable. The actuator may be configured to drive the delivery mechanism. In some examples, the actuator may include a motor, such as an electric motor. The delivery mechanism may be configured to move the therapeutic substance from the reservoir through the cannula. In some examples, the delivery mechanism may include a pump and/or a plunger. The cannula may facilitate a fluidic connection between the reservoir and the body of patient 101. The cannula and/or a needle may facilitate delivery of the therapeutic substance to a tissue layer, vein, interstitial fluid, or body cavity of patient 101. During operation, the actuator, in response to a signal (e.g., a command signal), may drive the delivery mechanism, thereby causing the therapeutic substance to move from the reservoir, through the cannula, and into the body of patient 101.

The components of delivery device 102 described above are merely provided as an example. Delivery device 102 may include other components, such as, without limitation, a power supply, a communication transceiver, one or more processors or other computing resources, memory devices, and/or user interfaces (e.g., buttons, keys, display, etc.), among other things. In some implementations, delivery device 102 may host an App (e.g., an insulin calculator) that may calculate the desired amount of therapeutic substance to be delivered to patient 101. Persons skilled in the art will recognize various implementations of delivery device 102 and the components of such implementations. All such implementations and components are contemplated to be within the scope of the present disclosure.

Monitoring device 104 may be configured to detect a physiological condition (e.g., a glucose concentration level) of patient 101 and may also be configured to detect other things. Monitoring device 104 may be secured to the body of patient 101 (e.g., to the skin of patient 101 via an adhesive) and/or may be at least partially implanted into the body of patient 101. Depending on the particular location or configuration, monitoring device 104 may be in contact with biological matter (e.g., interstitial fluid and/or blood) of patient 101. Monitoring device 104 may include one or more sensors (not shown), such as, without limitation, electrochemical sensors, electrical sensors, and/or optical sensors. As persons skilled in the art will understand, an electrochemical sensor may be configured to respond to the interaction or binding of a biological marker to electrodes by generating an electrical signal based on, for example, a potential, conductance, current, and/or impedance of an electrical path through the electrodes. The electrodes may include a material selected to interact with a particular biomarker, such as glucose. The potential, current, conductance, and/or impedance may correlate with a concentration of the particular biomarker.

In some implementations, a glucose sensor may produce signals through a series of electrochemical reactions when the sensor is inserted and exposed to glucose and oxygen diffused from the interstitial fluid. In one example, the electrochemical sensor may include a glucose limiting membrane (GLM) that limits the amount of glucose and oxygen delivered to a glucose oxidase (GOx) layer of a working electrode of the sensor to ensure that the reactions are glucose limited. The GOx layer or another active enzyme layer on the working electrode of the sensor may break down glucose and oxygen into gluconic acid and hydrogen peroxide. The generated peroxide molecules may interact with the working electrode to break down hydrogen peroxide into two hydrogen ions, oxygen, and two electrons at the surface of the working electrode, when a voltage signal is supplied to the working electrode. The electrical charges may be forced to move between electrodes (e.g., between the working electrode and counter electrode), thereby generating a sensor current signal (Isig) that can be measured by sensor electronics. Other signals such as the counter voltage (Vcntr, the voltage potential difference between the counter electrode and the working electrode), electrochemical impedance spectroscopy (EIS) at different frequencies, and the like, may also be measured. The signals measured using the sensor, including the Isig, Vcntr, and EIS, may be processed (e.g., filtered or transformed) to generate some other signals or parameters, such as filtered Isig signals, real and imaginary impedance at various frequencies, and the like. These signals and/or the processed parameters may be used in one or more sensor glucose (SG) models (e.g., machine learning models or mathematical models) to determine SG values that may be estimations of the blood glucose (BG) levels of the patient.

In some embodiments, monitoring device 104 may include other types of sensors that may be worn, carried, or coupled to patient 101 to measure activity of patient 101 that may influence the glucose levels or glycemic response of patient 101. As an example, the sensors may include an acceleration sensor configured to detect an acceleration of patient 101 or a portion of the patient 101, such as the person's hands or feet, the position changes of which may be associated with an activity of patient 101. For example, the acceleration or movement (or lack thereof) of the body portion of patient 101 may be indicative of exercise, sleep, or food/beverage consumption activity of patient 101, which may influence the glycemic response of patient 101. In some embodiments, the sensors may measure heart rate and/or body temperature, which may indicate an amount of physical exertion experienced by patient 101. In some embodiments, the sensors may include a Global Positioning System (GPS) receiver which may detect GPS signals to determine a location of patient 101.

The sensors described above are merely provided as examples. Other sensors or types of sensors for monitoring physiological condition, activity, and/or location, among other things, will be recognized by persons skilled in the art and are contemplated to be within the scope of the present disclosure. For any sensor, the signal provided by a sensor may be referred to herein as a "sensor signal." As used herein, the term "sensed data" may mean and include the information represented by a sensor signal or by a pre-processed sensor signal. In some embodiments, sensed data may include glucose levels of patient 101, acceleration of a part of patient 101, heart rate of patient 101, temperature of patient 101, and/or geolocation (e.g., GPS location) of patient 101, among other things. Monitoring device 104 may communicate sensed data to delivery device 102 via communication link 112 and/or to computing device 106 via communication link 114. Use of sensed data by delivery device 102 and/or by computing device 106 is described in more detail below.

In some embodiments, monitoring device 104 may include components and/or circuitry configured to pre-process sensor signals. Pre-processing may include, for example, amplification, filtering, attenuation, scaling, isolation, normalization, transformation, sampling, and/or analog-to-digital conversion, among other things. In some embodiments, monitoring device 104 may host an App for process the sensor signals. In some embodiments, monitoring device 104 may include a wired or wireless transceiver as described above for transmitting the sensor signals or receiving commands or instructions. Persons skilled in the art will recognize various implementations for such pre-processing, including, without limitation, implementations using processors, controllers, integrated circuits, application specific integrated circuits (ASICs), hardware, firmware, programmable logic devices, and/or machine-executable instructions, among others. The types of pre-processing and their implementations are merely provided as examples. Other types of pre-processing and implementations are contemplated to be within the scope of the present disclosure. In some embodiments, monitoring device 104 may not perform pre-processing.

Computing device 106 may provide processing capabilities and may be implemented in various ways. In some embodiments, computing device 106 may be a consumer device, such as a smartphone, a computerized wearable device (e.g., a smartwatch), a tablet computer, a laptop computer, or a desktop computer, among others, or may be a special purpose device (e.g., a portable control device) provided by, for example, the manufacturer of delivery device 102. In some embodiments, computing device 106 may be processing circuitry that may be integrated with another device, such as delivery device 102. In some embodiments, computing device 106 may be secured to patient 101 (e.g., to the body or clothing of patient 101), may be at least partially implanted into the body of patient 101, and/or may be held by patient 101.

For each of the embodiments of computing device 106, computing device 106 may include various types of logic circuitry, including, but not limited to, microprocessors, controllers, digital signal processors (DSPs), application specific integrated circuits (ASICs), field-programmable gate arrays (FPGAs), central processing units (CPU), graphics processing units (GPU), programmable logic devices, memory (e.g., random access memory, volatile memory, non-volatile memory, etc.), or other discrete or integrated logic circuitry, as well as combinations of such components. The term "processing circuitry" may generally refer to any of the foregoing logic circuitry, alone or in combination with other logic circuitry, or any other circuitry for performing computations.

Some aspects of delivery device 102, monitoring device 104, and computing device 106 have been described above. One or more of devices 102-106 may include a user interface (not shown) that presents information to patient 101 and/or receives information from patient 101. The user interface may include a graphical user interface (GUI), a display device, a keyboard, a touchscreen, a speaker, a microphone, a vibration motor, buttons, switches, and/or other types of user interfaces. Persons skilled in the art will recognize various types of user interfaces that may be used, and all such user interfaces are contemplated to be within the scope of the present disclosure. For example, where computing device 106 is a consumer device such as a smart phone, tablet computer, laptop computer, or the like, the user interfaces would include a display device, a physical and/or virtual keyboard, and/or audio speakers provided by such consumer devices, among other things. In some embodiments, a user interface may notify patient 101 of sensed data (e.g., glucose level) and/or insulin delivery data (e.g., rates of historic, current, or future insulin delivery) and may present alerts to patient 101. In some embodiments, a user interface may receive inputs from patient 101, which may include, for example, a requested change in insulin delivery setting and/or a meal indication, among other things. The descriptions and embodiments above regarding user interfaces are merely provided as examples, and other types and other uses of user interfaces are contemplated to be within the scope of the present disclosure.

In one specific example, the communications between devices 102-106 and cooperation between devices 102-106 may be used for insulin delivery. As depicted in FIG. 1 and as described above, devices 102-106 may communicate with each other via communication links 112-116. In some embodiments, computing device 106 may control operations of delivery device 102 and/or monitoring device 104. For example, computing device 106 may generate one or more signals (e.g., a command signal) that cause delivery device 102 to deliver insulin to patient 101, for example, as a basal dosage and/or a bolus dosage. In some embodiments, computing device 106 may receive data associated with insulin delivery (e.g., insulin delivery data) from delivery device 102 and/or receive sensed data (e.g., glucose levels) from monitoring device 104, and may perform computations based on the insulin delivery data, the sensed data, and/or other data to control delivery device 102. Insulin delivery data may include, but is not limited to, the type of insulin being delivered, historical insulin delivery rates and/or amounts, current insulin delivery rate and/or amount, insulin delivery time, and/or user inputs affecting insulin delivery. As persons skilled in the art will understand, in a closed-loop operating mode, computing device 106 may communicate dosage commands to delivery device 102 based on, for example, a difference between a current glucose level in the body of patient 101 (e.g., received from monitoring device 104) and a target glucose level (e.g., determined by computing device 106 or set on delivery device 102). The dosage commands may indicate an amount of insulin to be delivered and/or a rate (or time) of insulin delivery, and may regulate the current glucose level toward the target glucose level.

Remote/cloud computing system 108 may be any proprietary remote/cloud computing system or a commercial cloud computing system including one or more server computing devices. Remote/cloud computing system 108 may provide alternative or additional computing resources as needed when the computing resources of a client computing device (e.g., computing device 106) are not sufficient. Computing device 106 and remote/cloud computing system 108 may communicate with each other through a communication link 118, which may traverse one or more communication networks (not shown). The communication networks may include, for example, an Ethernet network, a Wi-Fi network, a cellular network, a satellite network, an intranet, an extranet, the Internet, and/or the Internet backbone, among other types of networks. Persons skilled in the art will recognize implementations for remote/cloud computing system 108 and how to interface with such systems through various types of networks. For example, remote/cloud computing system 108 may include an array of processing circuitry and may execute machine-readable instructions. Such implementations, interfaces, and networks are contemplated to be within the scope of the present disclosure.

In some embodiments, remote/cloud computing system 108 may make a therapy determination (e.g., an insulin amount or adjusted insulin amount), and may communicate the therapy to delivery device 102 via computing device 106. In some embodiments, computing device 106 may make the therapy determination and communicate it to delivery device 102. In some embodiments, monitoring device 104 may make the therapy determination and communicate it to delivery device 102 either directly or through an intermediary such as computing device 106.

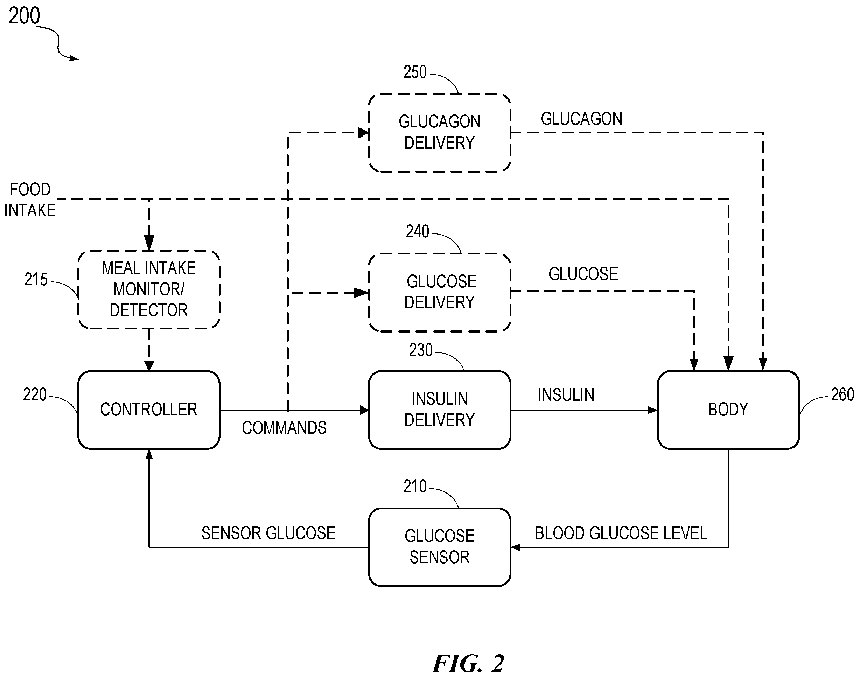

FIG. 2 is a block diagram of an example of a glucose level management system 200 according to certain embodiments. In the illustrated example, glucose level management system 200 may include a glucose sensor subsystem 210, a controller 220, an insulin delivery subsystem 230, a glucose delivery subsystem 240, and a glucagon delivery subsystem 250. Glucose sensor subsystem 210 may generate sensor glucose (SG) signals (e.g., SG levels) that may be the estimations of blood glucose levels in a body 260, and may provide the SG signals to controller 220. Controller 220 may receive the SG signals and generate commands to insulin delivery subsystem 230, and, in some implementations, glucose delivery subsystem 240 and/or glucagon delivery subsystem 250. Insulin delivery subsystem 230 may receive commands from controller 220 and deliver insulin to body 260 according to the commands. In some embodiments, glucose delivery subsystem 240 may receive commands from controller 220 and provide glucose into body 260 according to the commands. In some embodiments, glucagon delivery subsystem 250 may receive commands from controller 220 and deliver glucagon into body 260 according to the commands.

In some implementations, glucose sensor subsystem 210 may include a glucose sensor, sensor electronics configured to generate SG signals, a sensor communication system configured to send the SG signals to controller 220, and a housing for the sensor electronics and the sensor communication system. The glucose sensor may measure blood glucose levels, for example, directly from a blood stream, or indirectly via interstitial fluid using a subcutaneous sensor as described in more detail below.

Controller 220 may include electrical components and software to generate commands for insulin delivery subsystem 230, glucose delivery subsystem 240, and/or glucagon delivery subsystem 250. Controller 220 may include a controller communication system to receive the sensor signal and provide the commands to insulin delivery subsystem 230, glucose delivery subsystem 240, and/or glucagon delivery subsystem 250. In some implementations, controller 220 may implement a glucose calculator. In some implementations, controller 220 may include a user interface and/or operator interface (not shown) comprising a data input device and/or a data output device. Such a data output device may, for example, generate signals to initiate an alarm and/or include a display or printer for showing status of controller 220 and/or a patient's vital indicators. Such a data input device may include dials, buttons, pointing devices, manual switches, alphanumeric keys, a touch-sensitive display, combinations thereof, and/or the like for receiving user and/or operator inputs. Such a data input device may be used for scheduling and/or initiating insulin bolus injections for meals, for example. It should be understood, however, that these are merely examples of input and output devices that may be a part of an operator and/or user interface and that claimed subject matter is not limited in these respects.

Insulin delivery subsystem 230 may include, for example, an infusion device and/or an infusion tube to infuse insulin into body 260. Similarly, glucose delivery subsystem 240 may include, for example, an infusion device and/or an infusion tube to infuse glucose into body 260. Likewise, glucagon delivery subsystem 250 may include, for example, an infusion device and/or an infusion tube to infuse glucagon into body 260. In some embodiments, the insulin, glucagon, and/or glucose may be infused into body 260 using a shared delivery system and/or infusion tube. In some embodiments, the insulin, glucagon, and/or glucose may be infused using an intravenous system for providing fluids to a patient (e.g., in a hospital or other medical environment). It should be understood, however, that certain example embodiments may include an insulin delivery subsystem 230 without a glucagon delivery subsystem 250 and/or without a glucose delivery subsystem 240. In some embodiments, each of insulin delivery subsystem 230, glucose delivery subsystem 240, and glucagon delivery subsystem 250 may include infusion electrical components to activate an infusion motor according to the commands from controller 220, an infusion communication system to receive commands from controller 220, and a delivery subsystem housing.

In some embodiments, controller 220 may be housed in a delivery subsystem housing, and an infusion communication system may comprise an electrical trace or a wire that carries the commands from controller 220 to the delivery subsystem. In some embodiments, controller 220 may be housed in a sensor system housing, and a sensor communication system may comprise an electrical trace or a wire that carries the sensor signal from sensor electrical components to controller electrical components. In some embodiments, controller 220 may have its own housing or may be included in a supplemental device. In some embodiments, controller 220 may be co-located with a delivery subsystem and a sensor system within a single housing. In some embodiments, a sensor, a controller, and/or infusion communication systems may utilize a cable; a wire; a fiber optic line; RF, IR, or ultrasonic transmitters and receivers; combinations thereof; and/or the like instead of electrical traces, just to name a few examples.

In some embodiments, glucose level management system 200 may also include a meal intake monitoring subsystem 215. Meal intake monitoring subsystem 215 may be used to log the amount of user food intake, or may automatically detect the amount of user food intake. For example, in some implementations, the user may enter the food items and/or the estimated amount of carbohydrates in a meal at the meal time. In some implementations, meal intake monitoring subsystem 215 may include sensors (e.g., cameras or accelerators) that may automatically detect a meal event, the food items in a meal, and/or the estimated amount of carbohydrates in the meal. The estimated amount of carbohydrates in the meal may be sent to controller 220, which may determine an appropriate amount of meal bolus and generate a command for insulin delivery subsystem 230 to deliver the meal bolus. In some implementations, meal intake monitoring subsystem 215 may not be used in glucose level management system 200, and the dosage for the meal bolus may be determined based on the measured glucose level.

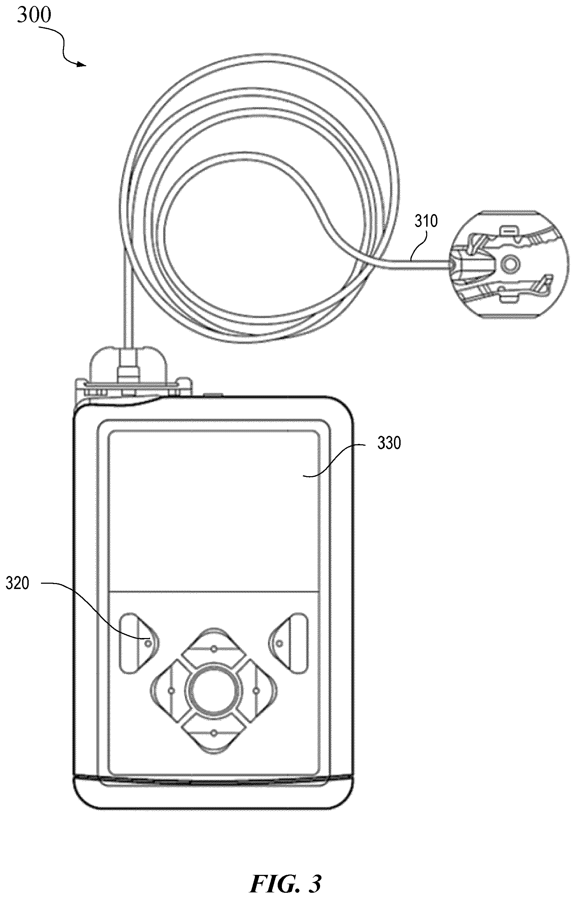

FIG. 3 depicts an example of a fluid delivery device 300, which may implement a delivery device described above (e.g., delivery device 102 or insulin delivery subsystem 230). In an insulin delivery device implemented using fluid delivery device 300, insulin delivery may be performed based on internal communication between a central computing module (e.g., a microprocessor of fluid delivery device 300) and an insulin delivery module (e.g., including a microcontroller, a motor, and a pump). For instance, insulin delivery may be caused by the central computing module communicating a delivery command in the form of an electrical signal that travels via a communication fabric to the insulin delivery module. The central computing module may also be configured to communicate (e.g., via a transceiver) with a computing device (e.g., computing device 106 of FIG. 1) communicatively coupled to a remote or cloud computing system (e.g., remote/cloud computing system 108 of FIG. 1). Fluid delivery device 300 may communicate various event data toward the remote or cloud computing system, which may communicate insulin delivery determinations toward fluid delivery device 300, in accordance with the techniques described herein.

Fluid delivery device 300 may provide insulin through a small tube 310 configured for fluidic connection with a cannula inserted subcutaneously. Fluid delivery device 300 may be configured to deliver two types of dosages-a basal dosage, which can be delivered periodically (e.g., every five minutes) in tiny amounts throughout the day and night, and a bolus dosage to cover an increase in blood glucose from meals and/or to correct high blood glucose levels. In the depicted example, fluid delivery device 300 includes a user interface having button elements 320 that can be manipulated to administer a bolus of insulin, to change therapy settings, to change user preferences, to select display features, and the like. Fluid delivery device 300 may also include a display device 330 that can be used to present various types of information or data to the user. In accordance with aspects of the present disclosure, a user of fluid delivery device 300 may use button elements 320 to input certain event data (e.g., event type, event start time, event details, etc.), and the user inputs can be confirmed using display device 330. Fluid delivery device 300 of FIG. 3 is merely provided by way of example, and other types of insulin delivery devices and other techniques different from those described above are contemplated to be within the scope of the present disclosure.

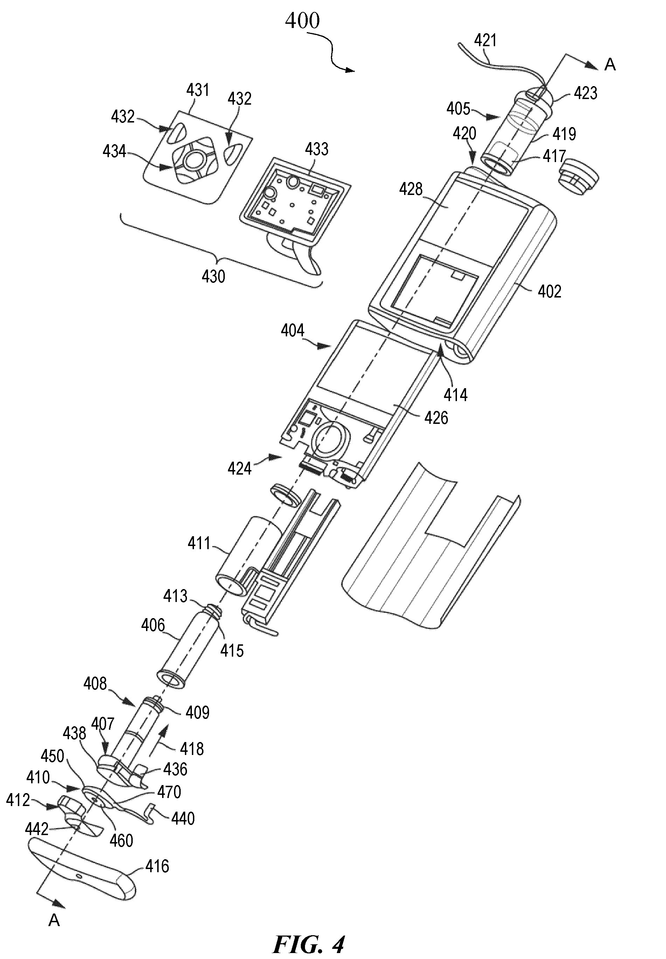

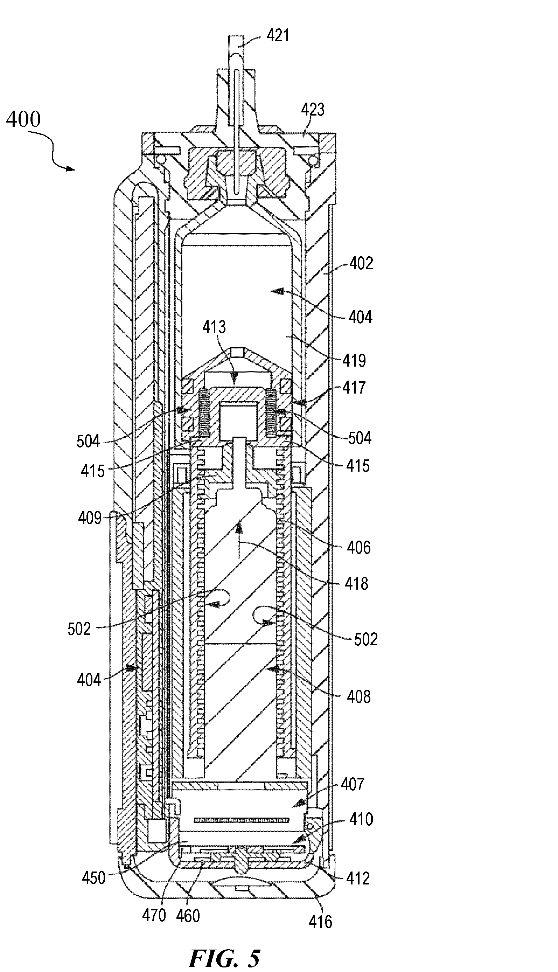

FIG. 4 and FIG. 5 illustrate an example of a fluid delivery device 400 (e.g., an infusion pump) suitable for use in an infusion system, such as, for example, delivery device 102 or insulin delivery subsystem 230. Fluid delivery device 400 may be an example of an implementation of fluid delivery device 300. For example, FIG. 4 may be an exploded perspective view of an example of fluid delivery device 300 of FIG. 3 according to certain embodiments. FIG. 5 may be a cross-sectional view of the fluid delivery device of FIG. 4 with a reservoir inserted in the fluid delivery device. Fluid delivery device 400 may be a portable medical device designed to be carried or worn by a patient (or user), and may leverage any number of conventional features, components, elements, and characteristics of existing fluid infusion devices, such as, for example, some of the features, components, elements, and/or characteristics described in United States Patent Nos. 5,485,465 and 7,621,893. It should be appreciated that FIGS. 4-5 depict some aspects of the fluid delivery device 400 in a simplified manner; in practice, the fluid delivery device 400 may include additional elements, features, or components that are not shown or described in detail herein.

As illustrated in FIGS. 4-5, fluid delivery device 400 may include a housing 402 adapted to receive a fluid-containing reservoir 405. An opening 420 in the housing 402 accommodates a fitting 423 (or cap) for the reservoir 405, with the fitting 423 being configured to mate or otherwise interface with tubing 421 of an infusion set 425 that provides a fluid path to/from the body of the user. In this manner, fluid communication from the interior of the reservoir 405 to the user is established via the tubing 421. The illustrated fluid delivery device 400 includes a human-machine interface (HMI) 430 (or user interface) that includes elements 432, 434 that can be manipulated by the user to administer a bolus of fluid (e.g., insulin), to change therapy settings, to change user preferences, to select display features, to enter and/or confirm BG measurement values, and the like. The infusion device also includes a display element 426, such as a liquid crystal display (LCD) or another suitable display element, that can be used to present various types of information or data to the user, such as, without limitation: the current glucose level of the patient; the time; a graph or chart of the patient's glucose level versus time; device status indicators; etc.

The housing 402 is formed from a substantially rigid material having a hollow interior 414 adapted to allow an electronics assembly 404, a sliding member (or slide) 406, a drive system 408, a sensor assembly 410, and a drive system capping member 412 to be disposed therein in addition to the reservoir 405, with the contents of the housing 402 being enclosed by a housing capping member 416. The opening 420, the slide 406, and the drive system 408 are coaxially aligned in an axial direction 418, whereby the drive system 408 facilitates linear displacement of the slide 406 in the axial direction 418 to dispense fluid from the reservoir 405 (after the reservoir 405 has been inserted into opening 420), with the sensor assembly 410 being configured to measure axial forces (e.g., forces aligned with the axial direction 418) exerted on the sensor assembly 410 responsive to operating the drive system 408 to displace the slide 406. In various embodiments, the sensor assembly 410 may be utilized to detect one or more of the following: an occlusion in a fluid path that slows, prevents, or otherwise degrades fluid delivery from the reservoir 405 to a user's body; when the reservoir 405 is empty; when the slide 406 is properly seated with the reservoir 405; when a fluid dose has been delivered; when the fluid delivery device 400 is subjected to shock or vibration; when the fluid delivery device 400 requires maintenance.

Depending on the embodiment, the fluid-containing reservoir 405 may be realized as a syringe, a vial, a cartridge, a bag, or the like. In certain embodiments, the infused fluid is insulin, although many other fluids may be administered through infusion such as, but not limited to, HIV drugs, drugs to treat pulmonary hypertension, iron chelation drugs, pain medications, anti-cancer treatments, medications, vitamins, hormones, or the like. As illustrated in FIGS. 4-5, the reservoir 405 typically includes a reservoir barrel 419 that contains the fluid and is concentrically and/or coaxially aligned with the slide 406 (e.g., in the axial direction 418) when the reservoir 405 is inserted into the fluid delivery device 400. The end of the reservoir 405 proximate the opening 420 may include or otherwise mate with the fitting 423, which secures the reservoir 405 in the housing 402 and prevents displacement of the reservoir 405 in the axial direction 418 with respect to the housing 402 after the reservoir 405 is inserted into the housing 402. As described above, the fitting 423 extends from (or through) the opening 420 of the housing 402 and mates with tubing 421 to establish fluid communication from the interior of the reservoir 405 (e.g., reservoir barrel 419) to the user via the tubing 421 and infusion set 425. The opposing end of the reservoir 405 proximate the slide 406 includes a plunger 417 (or stopper) positioned to push fluid from inside the barrel 419 of the reservoir 405 along a fluid path through tubing 421 to a user. The slide 406 is configured to mechanically couple or otherwise engage with the plunger 417, thereby becoming seated with the plunger 417 and/or reservoir 405. Fluid is forced from the reservoir 405 via tubing 421 as the drive system 408 is operated to displace the slide 406 in the axial direction 418 toward the opening 420 in the housing 402.

In the illustrated embodiment of FIGS. 4-5, the drive system 408 includes a motor assembly 407 and a drive screw 409. The motor assembly 407 includes a motor that is coupled to drive train components of the drive system 408 that are configured to convert rotational motor motion to a translational displacement of the slide 406 in the axial direction 418, and thereby engaging and displacing the plunger 417 of the reservoir 405 in the axial direction 418. In some embodiments, the motor assembly 407 may also be powered to translate the slide 406 in the opposing direction (e.g., the direction opposite axial direction 418) to retract and/or detach from the reservoir 405 to allow the reservoir 405 to be replaced. In exemplary embodiments, the motor assembly 407 includes a brushless DC (BLDC) motor having one or more permanent magnets mounted, affixed, or otherwise disposed on its rotor. However, the subject matter described herein is not necessarily limited to use with BLDC motors, and in alternative embodiments, the motor may be realized as a solenoid motor, an AC motor, a stepper motor, a piezoelectric caterpillar drive, a shape memory actuator drive, an electrochemical gas cell, a thermally driven gas cell, a bimetallic actuator, or the like. The drive train components may comprise one or more lead screws, cams, ratchets, jacks, pulleys, pawls, clamps, gears, nuts, slides, bearings, levers, beams, stoppers, plungers, sliders, brackets, guides, bearings, supports, bellows, caps, diaphragms, bags, heaters, or the like. In this regard, although the illustrated embodiment of the infusion pump utilizes a coaxially aligned drive train, the motor could be arranged in an offset or otherwise noncoaxial manner, relative to the longitudinal axis of the reservoir 405.

As shown in FIG. 5, the drive screw 409 mates with threads 502 internal to the slide 406. When the motor assembly 407 is powered and operated, the drive screw 409 rotates, and the slide 406 is forced to translate in the axial direction 418. In an exemplary embodiment, the fluid delivery device 400 includes a sleeve 411 to prevent the slide 406 from rotating when the drive screw 409 of the drive system 408 rotates. Thus, rotation of the drive screw 409 causes the slide 406 to extend or retract relative to the drive motor assembly 407. When the fluid infusion device is assembled and operational, the slide 406 contacts the plunger 417 to engage the reservoir 405 and control delivery of fluid from the fluid delivery device 400. In an exemplary embodiment, the shoulder portion 415 of the slide 406 contacts or otherwise engages the plunger 417 to displace the plunger 417 in the axial direction 418. In alternative embodiments, the slide 406 may include a threaded tip 413 capable of being detachably engaged with internal threads 504 on the plunger 417 of the reservoir 405, as described in detail in United States Patent Nos. 5,248,093 and 5,485,465, which are incorporated by reference herein.

As illustrated in FIG. 4, the electronics assembly 404 includes control electronics 424 coupled to the display element 426, with the housing 402 including a transparent window portion 428 that is aligned with the display element 426 to allow the display element 426 to be viewed by the user when the electronics assembly 404 is disposed within the interior 414 of the housing 402. The control electronics 424 generally represent the hardware, firmware, processing logic and/or software (or combinations thereof) configured to control operation of the motor assembly 407 and/or drive system 408, as described in greater detail below in the context of FIG. 4. The control electronics 424 is also suitably configured and designed to support various user interface, input/output, and display features of fluid delivery device 400. Whether such functionality is implemented as hardware, firmware, a state machine, or software depends upon the particular application and design constraints imposed on the embodiment. Those familiar with the concepts described here may implement such functionality in a suitable manner for each particular application, but such implementation decisions should not be interpreted as being restrictive or limiting. In an exemplary embodiment, the control electronics 424 includes one or more programmable controllers that may be programmed to control operation of the fluid delivery device 400.

The motor assembly 407 includes one or more electrical leads 436 adapted to be electrically coupled to the electronics assembly 404 to establish communication between the control electronics 424 and the motor assembly 407. In response to command signals from the control electronics 424 that operate a motor driver (e.g., a power converter) to regulate the amount of power supplied to the motor from a power supply, the motor actuates the drive train components of the drive system 408 to displace the slide 406 in the axial direction 418 to force fluid from the reservoir 405 along a fluid path (including tubing 421 and an infusion set), thereby administering doses of the fluid contained in the reservoir 405 into the user's body. The power supply may include one or more batteries contained within the housing 402. Alternatively, the power supply may be a solar panel, capacitor, AC or DC power supplied through a power cord, or the like. In some embodiments, the control electronics 424 may operate the motor of the motor assembly 407 and/or drive system 408 in a stepwise manner, typically on an intermittent basis; to administer discrete precise doses of the fluid to the user according to programmed delivery profiles.

Referring to FIGS. 4-5, as described above, the user interface 430 includes HMI elements, such as buttons 432 and a directional pad 434, that are formed on a graphic keypad overlay 431 that overlies a keypad assembly 433, which includes features corresponding to the buttons 432, directional pad 434 or other user interface items indicated by the graphic keypad overlay 431. When assembled, the keypad assembly 433 is coupled to the control electronics 424, thereby allowing the HMI elements 432, 434 to be manipulated by the user to interact with the control electronics 424 and control operation of the fluid delivery device 400, for example, to administer a bolus of insulin, to change therapy settings, to change user preferences, to select display features, to set or disable alarms and reminders, and the like. In this regard, the control electronics 424 maintains and/or provides information to the display element 426 regarding program parameters, delivery profiles, pump operation, alarms, warnings, statuses, or the like, which may be adjusted using the HMI elements 432, 434. In various embodiments, the HMI elements 432, 434 may be realized as physical objects (e.g., buttons, knobs, joysticks, and the like) or virtual objects (e.g., using touch-sensing and/or proximity-sensing technologies). For example, in some embodiments, the display element 426 may be realized as a touch screen or touch-sensitive display, and in such embodiments, the features and/or functionality of the HMI elements 432, 434 may be integrated into the display element 426 and the user interface 430 may not be present. In some embodiments, the electronics assembly 404 may also include alert generating elements coupled to the control electronics 424 and suitably configured to generate one or more types of feedback, such as, without limitation: audible feedback; visual feedback; haptic (physical) feedback; or the like.

Referring to FIGS. 4-5, in accordance with one or more embodiments, the sensor assembly 410 includes a back plate structure 450 and a loading element 460. The loading element 460 is disposed between the capping member 412 and a beam structure 470 that includes one or more beams having sensing elements disposed thereon that are influenced by compressive force applied to the sensor assembly 410 that deflects the one or more beams, as described in greater detail in United States Patent No. 8,474,332, which is incorporated by reference herein. In exemplary embodiments, the back plate structure 450 is affixed, adhered, mounted, or otherwise mechanically coupled to the bottom surface 438 of the drive system 408 such that the back plate structure 450 resides between the bottom surface 438 of the drive system 408 and the housing capping member 416. The drive system capping member 412 is contoured to accommodate and conform to the bottom of the sensor assembly 410 and the drive system 408. The drive system capping member 412 may be affixed to the interior of the housing 402 to prevent displacement of the sensor assembly 410 in the direction opposite the direction of force provided by the drive system 408 (e.g., the direction opposite axial direction 418). Thus, the sensor assembly 410 is positioned between the motor assembly 407 and secured by the capping member 412, which prevents displacement of the sensor assembly 410 in a downward direction opposite the direction of the arrow that represents the axial direction 418, such that the sensor assembly 410 is subjected to a reactionary compressive force when the drive system 408 and/or motor assembly 407 is operated to displace the slide 406 in the axial direction 418 in opposition to the fluid pressure in the reservoir 405. Under normal operating conditions, the compressive force applied to the sensor assembly 410 is correlated with the fluid pressure in the reservoir 405. As shown, electrical leads 440 are adapted to electrically couple the sensing elements of the sensor assembly 410 to the electronics assembly 404 to establish communication to the control electronics 424, wherein the control electronics 424 are configured to measure, receive, or otherwise obtain electrical signals from the sensing elements of the sensor assembly 410 that are indicative of the force applied by the drive system 408 in the axial direction 418.

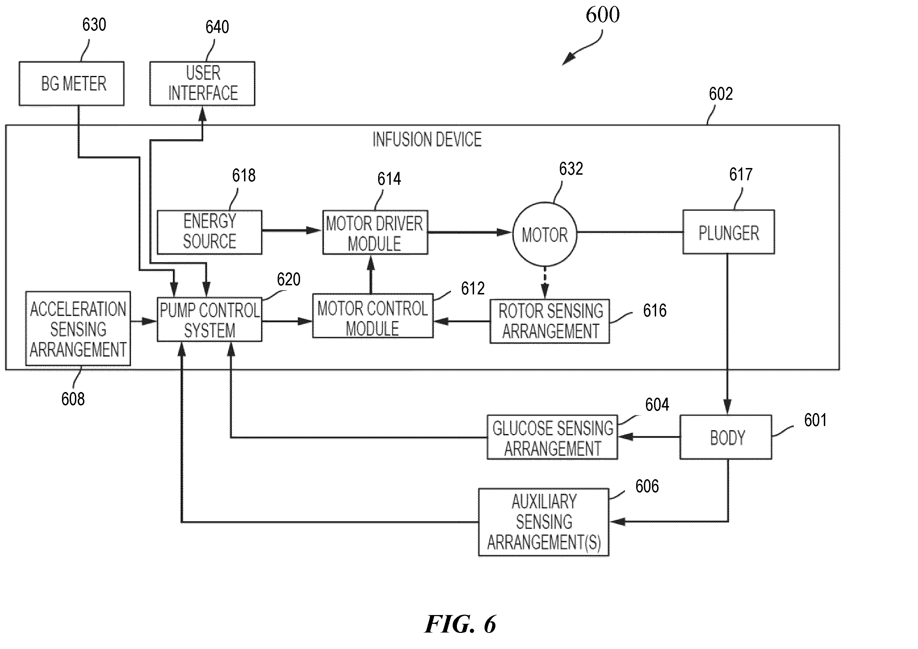

FIG. 6 is a block diagram of an example of an infusion system 600 suitable for use with an infusion device 602, such as delivery devices 102, insulin delivery subsystem 230, fluid delivery device 300, or fluid delivery device 400. The infusion system 600 is capable of controlling or otherwise regulating a physiological condition in the body 601 of a patient to a desired (or target) value or otherwise maintain the condition within a range of acceptable values in an automated or autonomous manner. In one or more exemplary embodiments, the condition being regulated is sensed, detected, measured or otherwise quantified by a sensing arrangement 604 (e.g., a BG or CGM sensing arrangement 604) communicatively coupled to the infusion device 602. However, it should be noted that in alternative embodiments, the condition being regulated by the infusion system 600 may be correlative to the measured values obtained by the sensing arrangement 604. That said, for clarity and purposes of explanation, the subject matter may be described herein in the context of the sensing arrangement 604 being realized as a glucose sensing arrangement that senses, detects, measures or otherwise quantifies the patient's glucose level, which is being regulated in the body 601 of the patient by the infusion system 600.

In exemplary embodiments, the sensing arrangement 604 includes one or more interstitial glucose sensing elements that generate or otherwise output electrical signals (alternatively referred to herein as measurement signals) having a signal characteristic that is correlative to, influenced by, or otherwise indicative of the relative interstitial fluid glucose level in the body 601 of the patient. The output electrical signals are filtered or otherwise processed to obtain a measurement value indicative of the patient's interstitial fluid glucose level. In exemplary embodiments, a BG meter 630, such as a finger stick device, is utilized to directly sense, detect, measure or otherwise quantify the BG in the body 601 of the patient. In this regard, the BG meter 630 outputs or otherwise provides a measured BG value that may be utilized as a reference measurement for calibrating the sensing arrangement 604 and converting a measurement value indicative of the patient's interstitial fluid glucose level into a corresponding calibrated BG value. For purposes of explanation, the calibrated BG value calculated based on the electrical signals output by the sensing element(s) of the sensing arrangement 604 may alternatively be referred to herein as the sensor glucose value, the sensed glucose value, or variants thereof.

In exemplary embodiments, the infusion system 600 also includes one or more additional sensing arrangements 606, 608 configured to sense, detect, measure or otherwise quantify a characteristic of the body 601 of the patient that is indicative of a condition in the body 601 of the patient. In this regard, in addition to the glucose sensing arrangement 604, one or more auxiliary sensing arrangements 606 may be worn, carried, or otherwise associated with the body 601 of the patient to measure characteristics or conditions of the patient (or the patient's activity) that may influence the patient's glucose levels or insulin sensitivity. For example, a heart rate sensing arrangement 606 could be worn on or otherwise associated with the patient's body 601 to sense, detect, measure or otherwise quantify the patient's heart rate, which, in turn, may be indicative of exercise (and the intensity thereof) that is likely to influence the patient's glucose levels or insulin response in the body 601. In yet another embodiment, another invasive, interstitial, or subcutaneous sensing arrangement 606 may be inserted into the body 601 of the patient to obtain measurements of another physiological condition that may be indicative of exercise (and the intensity thereof), such as, for example, a lactate sensor, a ketone sensor, or the like. Depending on the embodiment, the auxiliary sensing arrangement(s) 606 could be realized as a standalone component worn by the patient, or alternatively, the auxiliary sensing arrangement(s) 606 may be integrated with the infusion device 602 or the glucose sensing arrangement 604.

The illustrated infusion system 600 also includes an acceleration sensing arrangement 608 (or accelerometer) that may be worn on or otherwise associated with the patient's body 601 to sense, detect, measure or otherwise quantify an acceleration of the patient's body 601, which, in turn, may be indicative of exercise or some other condition in the body 601 that is likely to influence the patient's insulin response. While the acceleration sensing arrangement 608 is depicted as being integrated into the infusion device 602 in FIG. 6, in alternative embodiments, the acceleration sensing arrangement 608 may be integrated with another sensing arrangement 604, 606 on the body 601 of the patient, or the acceleration sensing arrangement 608 may be realized as a separate standalone component that is worn by the patient.

In the illustrated embodiment, the pump control system 620 generally represents the electronics and other components of the infusion device 602 that control operation of the infusion device 602 according to a desired infusion delivery program in a manner that is influenced by the sensed glucose value indicating the current glucose level in the body 601 of the patient. For example, to support a closed-loop operating mode, the pump control system 620 maintains, receives, or otherwise obtains a target or commanded glucose value, and automatically generates or otherwise determines dosage commands for operating an actuation arrangement, such as a motor 632, to displace the plunger 617 and deliver insulin to the body 601 of the patient based on the difference between the sensed glucose value and the target glucose value. In other operating modes, the pump control system 620 may generate or otherwise determine dosage commands configured to maintain the sensed glucose value below an upper glucose limit, above a lower glucose limit, or otherwise within a desired range of glucose values. In practice, the infusion device 602 may store or otherwise maintain the target value, upper and/or lower glucose limit(s), insulin delivery limit(s), and/or other glucose threshold value(s) in a data storage element accessible to the pump control system 620. As described in greater detail, in one or more exemplary embodiments, the pump control system 620 automatically adjusts or adapts one or more parameters or other control information used to generate commands for operating the motor 632 in a manner that accounts for a likely change in the patient's glucose level or insulin response resulting from a meal, exercise, or other activity.

Still referring to FIG. 6, the target glucose value and other threshold glucose values utilized by the pump control system 620 may be received from an external component or be input by a patient via a user interface element 640 associated with the infusion device 602. In practice, the one or more user interface element(s) 640 associated with the infusion device 602 typically include at least one input user interface element, such as, for example, a button, a keypad, a keyboard, a knob, a joystick, a mouse, a touch panel, a touchscreen, a microphone or another audio input device, and/or the like. Additionally, the one or more user interface element(s) 640 include at least one output user interface element, such as, for example, a display element (e.g., a light-emitting diode or the like), a display device (e.g., a liquid crystal display or the like), a speaker or another audio output device, a haptic feedback device, or the like, for providing notifications or other information to the patient. It should be noted that although FIG. 6 depicts the user interface element(s) 640 as being separate from the infusion device 602, in practice, one or more of the user interface element(s) 640 may be integrated with the infusion device 602. Furthermore, in some embodiments, one or more user interface element(s) 640 are integrated with the sensing arrangement 604 in addition to and/or in alternative to the user interface element(s) 640 integrated with the infusion device 602. The user interface element(s) 640 may be manipulated by the patient to operate the infusion device 602 to deliver correction boluses, adjust target and/or threshold values, modify the delivery control scheme or operating mode, and the like, as desired.

Still referring to FIG. 6, in the illustrated embodiment, the infusion device 602 includes a motor control module 612 coupled to a motor 632 (e.g., motor assembly 407) that is operable to displace a plunger 617 (e.g., plunger 417) in a reservoir (e.g., reservoir 405) and provide a desired amount of fluid to the body 601 of a patient. In this regard, displacement of the plunger 617 results in the delivery of a fluid (e.g., insulin) that is capable of influencing the patient's physiological condition to the body 601 of the patient via a fluid delivery path (e.g., via tubing 421 of an infusion set 425). A motor driver module 614 is coupled between an energy source 618 and the motor 632. The motor control module 612 is coupled to the motor driver module 614, and the motor control module 612 generates or otherwise provides command signals that operate the motor driver module 614 to provide current (or power) from the energy source 618 to the motor 632 to displace the plunger 617 in response to receiving, from a pump control system 620, a dosage command indicative of the desired amount of fluid to be delivered.

In exemplary embodiments, the energy source 618 is realized as a battery housed within the infusion device 602 (e.g., within housing 402) that provides direct current (DC) power. In this regard, the motor driver module 614 generally represents the combination of circuitry, hardware and/or other electrical components configured to convert or otherwise transfer DC power provided by the energy source 618 into alternating electrical signals applied to respective phases of the stator windings of the motor 632 that result in current flowing through the stator windings that generates a stator magnetic field and causes the rotor of the motor 632 to rotate. The motor control module 612 is configured to receive or otherwise obtain a commanded dosage from the pump control system 620, convert the commanded dosage to a commanded translational displacement of the plunger 617, and command, signal, or otherwise operate the motor driver module 614 to cause the rotor of the motor 632 to rotate by an amount that produces the commanded translational displacement of the plunger 617. For example, the motor control module 612 may determine an amount of rotation of the rotor required to produce translational displacement of the plunger 617 that achieves the commanded dosage received from the pump control system 620. Based on the current rotational position (or orientation) of the rotor with respect to the stator that is indicated by the output of the rotor sensing arrangement 616, the motor control module 612 determines the appropriate sequence of alternating electrical signals to be applied to the respective phases of the stator windings that should rotate the rotor by the determined amount of rotation from its current position (or orientation). In embodiments where the motor 632 is realized as a BLDC motor, the alternating electrical signals commutate the respective phases of the stator windings at the appropriate orientation of the rotor magnetic poles with respect to the stator and in the appropriate order to provide a rotating stator magnetic field that rotates the rotor in the desired direction. Thereafter, the motor control module 612 operates the motor driver module 614 to apply the determined alternating electrical signals (e.g., the command signals) to the stator windings of the motor 632 to achieve the desired delivery of fluid to the patient.

When the motor control module 612 is operating the motor driver module 614, current flows from the energy source 618 through the stator windings of the motor 632 to produce a stator magnetic field that interacts with the rotor magnetic field. In some embodiments, after the motor control module 612 operates the motor driver module 614 and/or motor 632 to achieve the commanded dosage, the motor control module 612 ceases operating the motor driver module 614 and/or motor 632 until a subsequent dosage command is received. In this regard, the motor driver module 614 and the motor 632 enter an idle state during which the motor driver module 614 effectively disconnects or isolates the stator windings of the motor 632 from the energy source 618. In other words, current does not flow from the energy source 618 through the stator windings of the motor 632 when the motor 632 is idle, and thus, the motor 632 does not consume power from the energy source 618 in the idle state, thereby improving efficiency.

Depending on the embodiment, the motor control module 612 may be implemented or realized with a general purpose processor, a microprocessor, a controller, a microcontroller, a state machine, a content addressable memory, an application specific integrated circuit, a field programmable gate array, any suitable programmable logic device, discrete gate or transistor logic, discrete hardware components, or any combination thereof, designed to perform the functions described herein. In exemplary embodiments, the motor control module 612 includes or otherwise accesses a data storage element or memory, including any sort of random access memory (RAM), read only memory (ROM), flash memory, registers, hard disks, removable disks, magnetic or optical mass storage, or any other short or long term storage media or other non-transitory computer-readable medium, which is capable of storing programming instructions for execution by the motor control module 612. The computer-executable programming instructions, when read and executed by the motor control module 612, cause the motor control module 612 to perform or otherwise support the tasks, operations, functions, and processes described herein.

It should be appreciated that FIG. 6 is a simplified representation of the infusion device 602 for purposes of explanation and is not intended to limit the subject matter described herein in any way. In this regard, depending on the embodiment, some features and/or functionality of the sensing arrangement 604 may implemented by or otherwise integrated into the pump control system 620, or vice versa. Similarly, in practice, the features and/or functionality of the motor control module 612 may implemented by or otherwise integrated into the pump control system 620, or vice versa. Furthermore, the features and/or functionality of the pump control system 620 may be implemented by control electronics 424 located in the infusion device 602, while in alternative embodiments, the pump control system 620 may be implemented by a remote computing device that is physically distinct and/or separate from the infusion device 602.

It is desirable that the insulin delivered, including the basal insulin and bolus insulin, can maintain a diabetes patient's glucose level within a desired range. Managing the patient's glucose level may be complicated by variations in, for example, the patient's individual physiological response to insulin and various types of food, and potentially other factors, such as the patient's daily activities (e.g., exercise). Thus, the delivery of insulin to the patient may need to be precisely controlled to occur at time instants and in amounts that are determined based on, for example, the amount of carbohydrates taken by a patient and/or real-time measurements of glucose levels (e.g., interstitial or blood glucose level) by a glucose sensor, such as a CGM sensor.

As described above, a fluid delivery device for automatically or semi-automatically delivering fluid to a patient may include a motor and other actuation arrangements that are operable to linearly displace a plunger (or stopper) of a fluid reservoir provided within the fluid delivery device to deliver a dosage of fluid, such as insulin, to the body of the patient. The fluid delivery device, such as an insulin pump, may be worn by a user to deliver fluid (e.g., insulin) in a substantially continuous and autonomous manner, for example, overnight while the patient is sleeping. The fluid reservoir may be disposable, replaceable, or refillable, whereas other portions of the fluid delivery device may be reuseable. Different fluid reservoirs may have different frictions between the plunger and the barrel, and may be filled with fluid of different volumes. The drive system, such as the motor and the drive train components, in different fluid delivery devices may also have different performance. Therefore, when a new or refilled reservoir is placed into the fluid delivery device, the volume of the fluid in the reservoir may need to be measured, and the fluid delivery device may need to be calibrated to compensate for variations in the drive system and reservoir, such as determining the appropriate drive voltage level and/or drive signal duty cycle and the corresponding deliver speed, in order to precisely control the amount of the fluid delivered to the user. In addition, since many fluid delivery devices, such as insulin pumps, may be portable and wearable and may generally be powered by batteries, it may be desirable that the fluid delivery device (e.g., an insulin pump) employs a safe, reliable, accurate, power efficient, and relatively simple and fast calibration and control schemes.

An insulin pump may utilize a motor for controlled delivery of insulin. To precisely control the dosing of insulin therapy and to make the insulin pump energy efficient for longer battery life, the motor may be controlled to perform pulsatile operations as opposed to continuous motion. For example, some insulin pumps may utilize a set of motor stroke sizes (e.g., 3 different stroke sizes), where each stroke may be used for delivering a set amount of insulin. The amount of insulin in each request for insulin delivery may be divided into one or more strokes. In each stroke, the motor may be driven at a constant voltage for a specified amount of time and then allowed to coast to a stop. In some examples, the drive time for each stroke may be adjusted based on the feedback from a motor position sensor to compensate for under or over delivery that may occur due to, for example, changes in fluidic back pressure and drive system friction. This driving technique may result in energy inefficiency due to, for example, a combination of short motor on times (which may result in high peak motor power) and long processor and sensor on times for each stroke (e.g., in order to capture the long coasting motion). In addition, this driving technique may result in variations of insulin delivery (and therefore errors) from stroke to stroke due to the indirect relationship between the motor drive time and the amount of insulin delivered. Furthermore, additional force, strain, or pressure sensors (which may be collectively referred to herein as strain sensors) may need to be used to detect the back-pressure generated from the fluid in the fluid reservoir, in order to detect the initial plunger position (and the amount of insulin in a reservoir) and possible occlusion during the fluid delivery. The inclusion of the additional strain sensor(s) in a fluid delivery device may significantly increase the design and control complexity, increase the cost, and reduce the reliability of the infusion pump due to, for example, the sensor fragility.

According to certain embodiments disclosed herein, the motor of a fluid delivery device may be controlled to perform pulsatile motor operations, where the set of stroke sizes may include many (e.g., more than 10, more than 20, or more than 30) different stroke sizes, depending on the maximum stroke size used. In one example, each stroke may have at least 4 steps, and may have a number of steps between 4 steps and 32 or more steps. The number of strokes and the number of steps in each stroke may be determined based on the total unit of fluid to be delivered in each delivery event (e.g., each insulin delivery request). Rather than controlling the delivery amount based on the motor drive time duration, step-by-step position control may be utilized to more precisely control the delivery amount. For example, the changes of position of the motor may be detected by a motor position sensor (e.g., a Hall effect sensor), the time interval for the motor to rotate from one position to the next position may be measured and used to determine a delivery rate (e.g., in units/minute), and the number of steps the motor has rotated may be used to determine the amount (e.g., units) of fluid delivered. Active motor braking (instead of coasting) may also be used to improve energy efficiency of the motor, position sensor, and controller. For example, after each stroke, the windings of the motor may be grounded to stop the rotation of the motor.

Each motor and drive system may be driven at a specific voltage level that is calibrated for a specific stall load during production. The friction between the barrel and the plunger (e.g., the O-rings on the plunger) of each reservoir may be measured and calibrated during the reservoir set up, such that the motor may be driven at the appropriate voltage level and duty cycle to achieve a desired delivery rate. These driving techniques allow plunger detection and occlusion detection to be performed using the motor speed (or rotation time and/or delivery rate) measured using motor position sensors that are also used for other purposes, rather than using the additional force, strain, or pressure sensors, such as sensor assembly 410.

As such, the fluid delivery device disclosed herein does not need a pressure sensor, and the driving techniques disclosed herein may significantly reduce energy consumption, improve delivery accuracy, and enable the detection of both the plunger during reservoir setup and possible occlusion during insulin delivery using a motor position sensor instead of an additional strain sensor. As such, the fluid delivery device may have a longer battery life, may use a smaller battery, or may use more energy for functions other than driving the motor. Improved stroke to stroke accuracy may result in more predictable therapy. The ability to detect both the plunger during reservoir setup and possible occlusion during insulin delivery using the motor position sensor instead of an additional pressure sensor may result in reduced mechanical design and control complexity, improved product reliability, and reduced product cost.

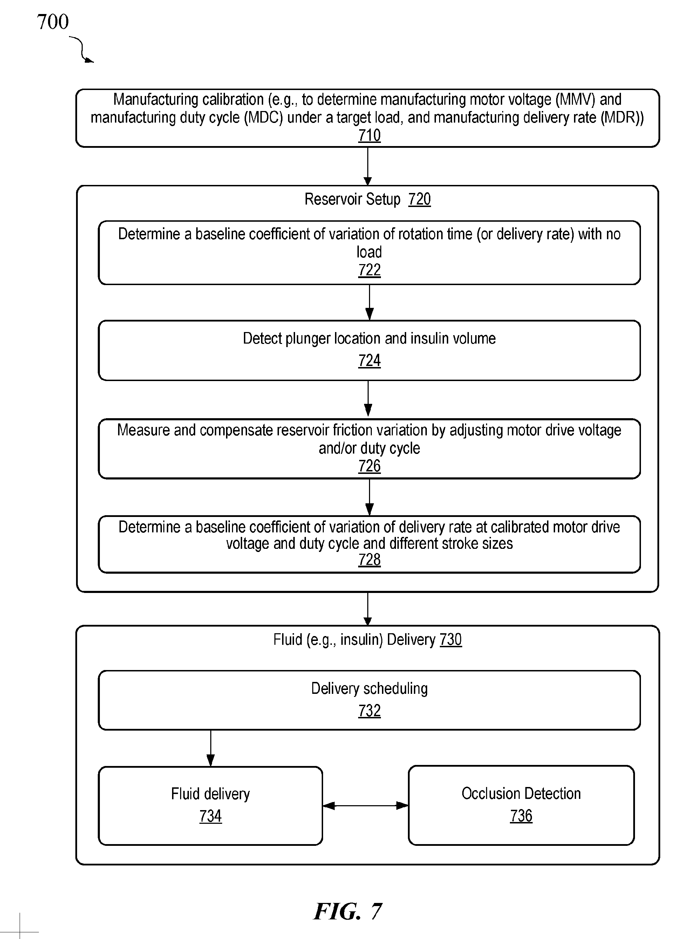

**FIG. 7** includes a flowchart 700 illustrating an example of a process of calibrating and operating a fluid delivery device according to certain embodiments. Operations in flowchart 700 may be performed by, for example, a controller or a processor of delivery device 102, insulin delivery subsystem 230, fluid delivery device 300, fluid delivery device 400 (without using sensor assembly 510), infusion device 602, and the like. Although flowchart 700 may describe the operations as a sequential process, some of the operations may be performed in parallel or concurrently. In addition, the order of the operations may be rearranged. The process may have additional steps not included in the figure. Some operations may be optional or may be omitted. Some operations may be merged with operations in another block, or may instead be performed in another block.

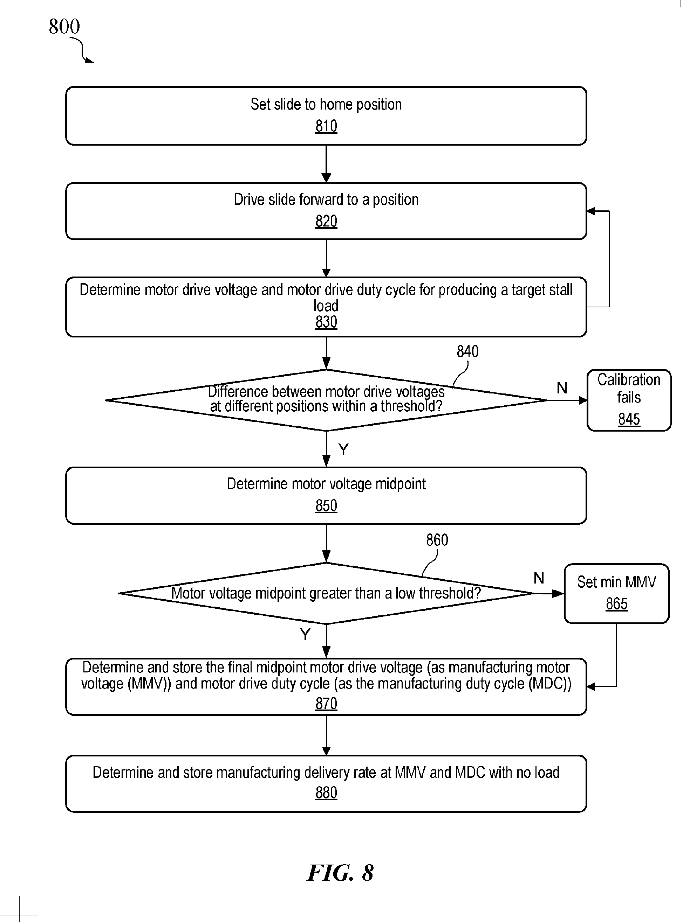

Flowchart 700 may include manufacturing calibration at block 710, reservoir setup at block 720, and fluid (e.g., insulin) delivery at block 730. During the manufacturing calibration at block 710, known loads may be applied by a testing system to the motor and other components of the drive system of an assembled fluid delivery device that may not have a reservoir installed. The motor drive voltage and motor drive duty cycle of a pulse width modulation (PWM) signal for driving the motor may be varied to maintain a certain delivery rate. The motor drive voltage and motor drive duty cycle that produce a target stall load may be stored (e.g., into a non-volatile memory of the fluid delivery device or a database on a server or another computing device) as a manufacturing motor voltage (MMV) and a manufacturing duty cycle (MDC), respectively. The target stall load may be greater than the back-pressure during an occlusion, for example, greater than about 2.5-3.5 pounds, such as about 4.25 pounds. To more accurately calibrate the fluid delivery device, the load and the motor drive voltage may be gradually increased until the load reaches the target stall load. A manufacturing delivery rate (MDR) may also be measured when the motor is driven at the MMV and MDC and no load is applied to the drive system. The manufacturing delivery rate (MDR) may also be stored in a non-volatile memory of the fluid delivery device for use in, for example, reservoir setup (e.g., reservoir friction compensation). More detail of the manufacturing calibration is described below with respect to, for example, FIG. 8.

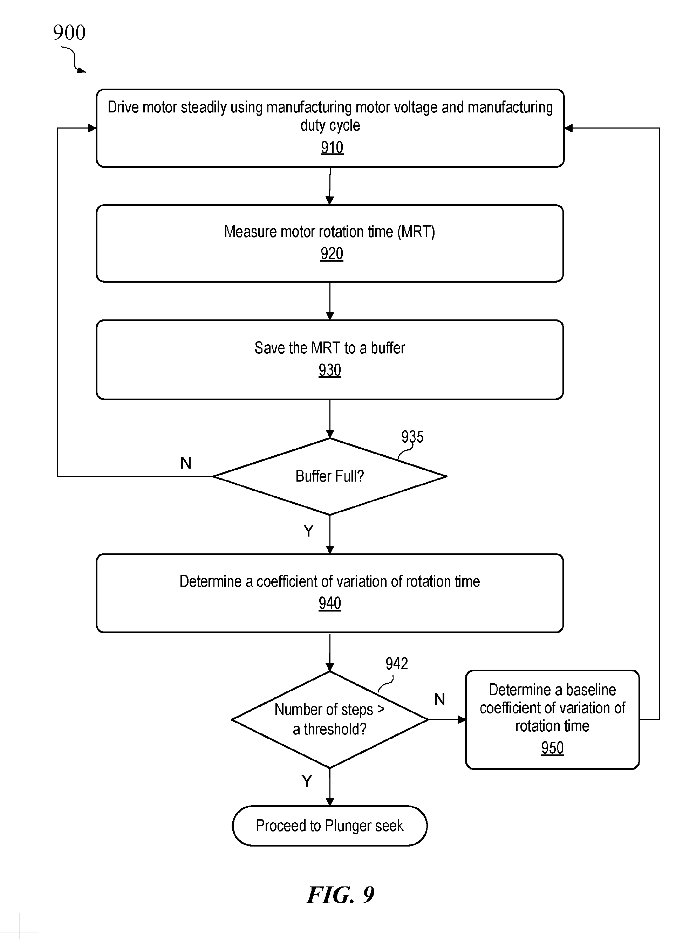

During reservoir setup at block 720, after a reservoir is placed into a chamber or cavity of the fluid delivery device, the motor may be driven at a steady-state speed (rather than in strokes) using the MMV and MDC measured during the manufacturing calibration, with no additional load applied to the drive system. The time of each rotation of the motor (referred to herein as motor rotation time) may be measured using a motor position sensor (e.g., a Hall effect sensor) while the motor is driven at the steady-state speed. A coefficient of variation of motor rotation time (or motor speed or delivery rate) may be determined during a first number of rotations at block 722, and may be used as a baseline coefficient of variation of motor rotation time for determining the plunger location (and insulin volume) in a reservoir at block 724. More detail of the operations in block 722 is described below with respect to, for example, FIG. 9.

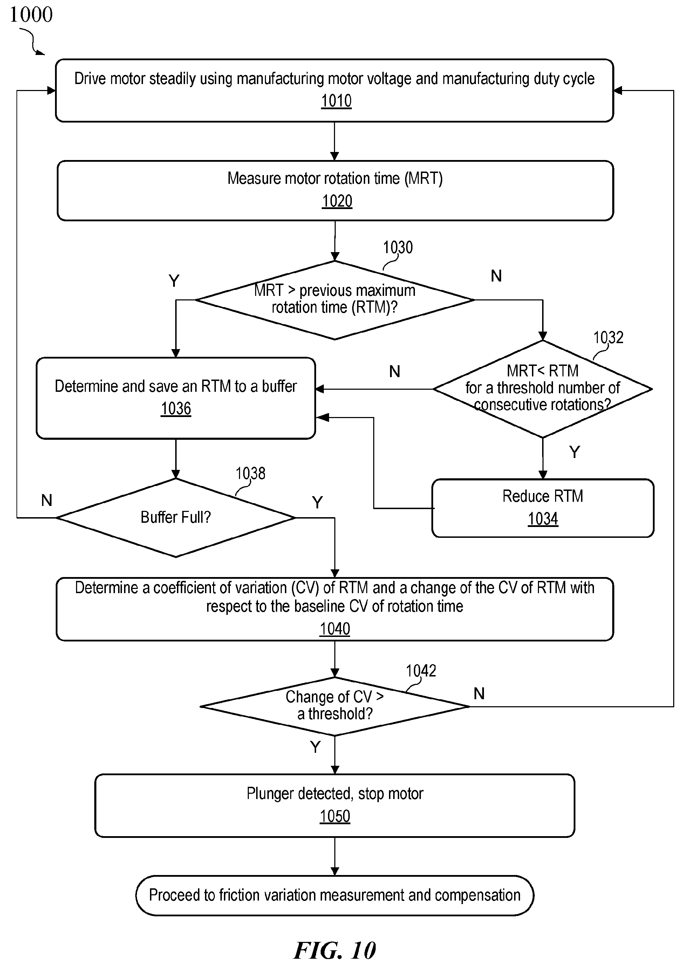

The location of the plunger may be determined at block 724 by continuing to drive the motor at the steady-state speed using the MMV and MDC with no additional load applied, and measuring the rotation time of the motor for a number of rotations to determine a moving coefficient of variation of motor rotation time. If the moving coefficient of variation of motor rotation time is above a threshold value relative to the baseline coefficient of variation of motor rotation time, the plunger may have been detected. If the moving coefficient of variation of motor rotation time is below the threshold value relative to the baseline coefficient of variation of motor rotation time, the plunger may not have been detected, the motor may continue to be driven at the steady-state speed, and the rotation time of the motor may continue to be measured for a number of rotations to determine the moving coefficient of variation of motor rotation time, until the measured moving coefficient of variation of motor rotation time is above the threshold value relative to the baseline coefficient of variation of motor rotation time. After the plunger location is determined, the volume of the fluid in the reservoir may be determined, for example, based on the total number of forward steps the motor has rotated from a home position. More detail of the operations in block 724 is described below with respect to, for example, FIG. 10.

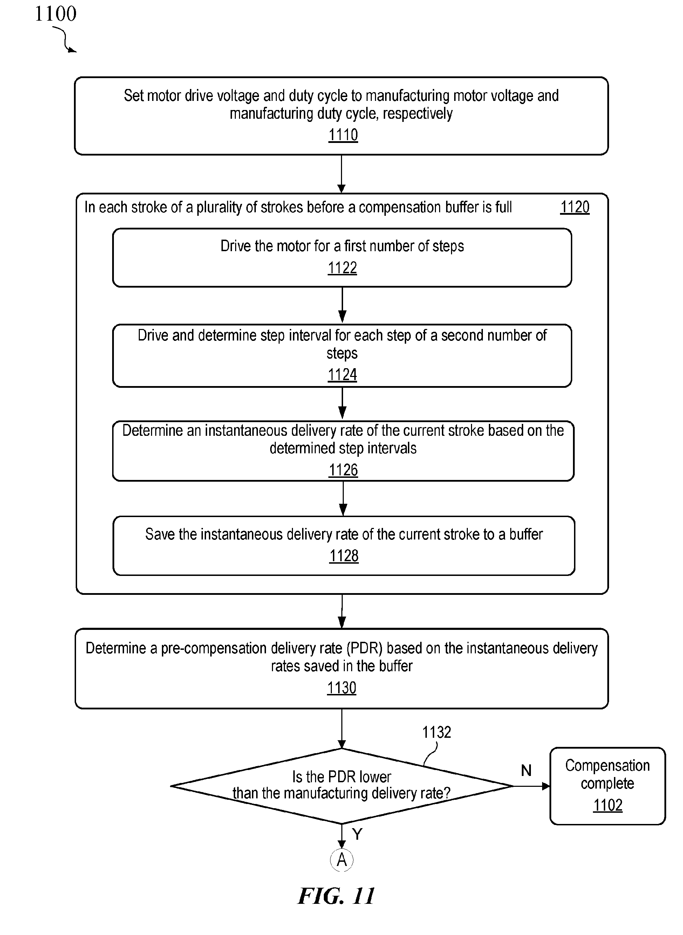

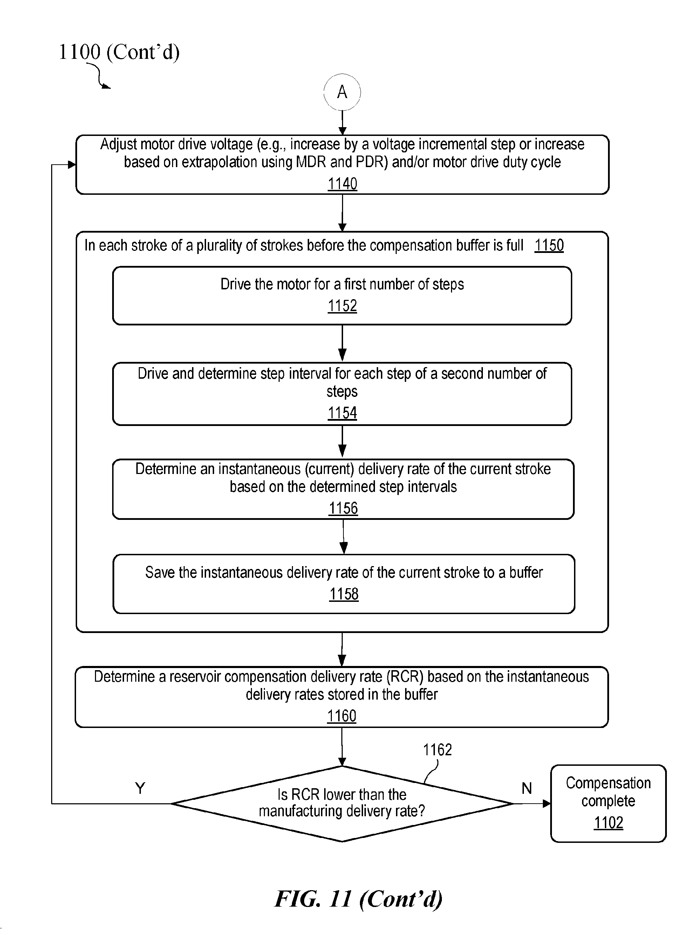

At block 726, after the plunger location is determined, the friction between the plunger (or R-rings) and the barrel of the reservoir may be determined, and the variation of the friction may be compensated by adjusting the motor drive voltage and/or motor drive duty cycle of a PWD signal for driving the motor. In one example, the motor may be driven in strokes of different sizes using the PWM signal with the MMV and MDC, the instantaneous delivery rate in each stroke may be measured, and the average delivery rate in the first number of strokes may be determined and used as the pre-compensation delivery rate. The motor may continue to be driven in strokes, the instantaneous delivery rate in each stroke may continue to be measured, and the average delivery rate in a certain number of strokes may continue to be determined and used as a current delivery rate. If the current delivery rate is at or greater than a threshold value (e.g., the manufacturing delivery rate measured and stored in the fluid delivery device during the manufacturing calibration), the current motor drive voltage and the motor drive duty cycle may be used as the calibrated motor drive voltage and motor drive duty cycle for the combination of the fluid delivery device and the specific reservoir. If the current delivery rate is lower than the threshold value, the current motor drive voltage and/or the motor drive duty cycle may be adjusted (e.g., increased), and the delivery rates in a certain number of strokes may be measured as described above, until an average delivery rate is at or greater than the threshold value. More detail of the operations in block 726 is described below with respect to, for example, FIG. 11.

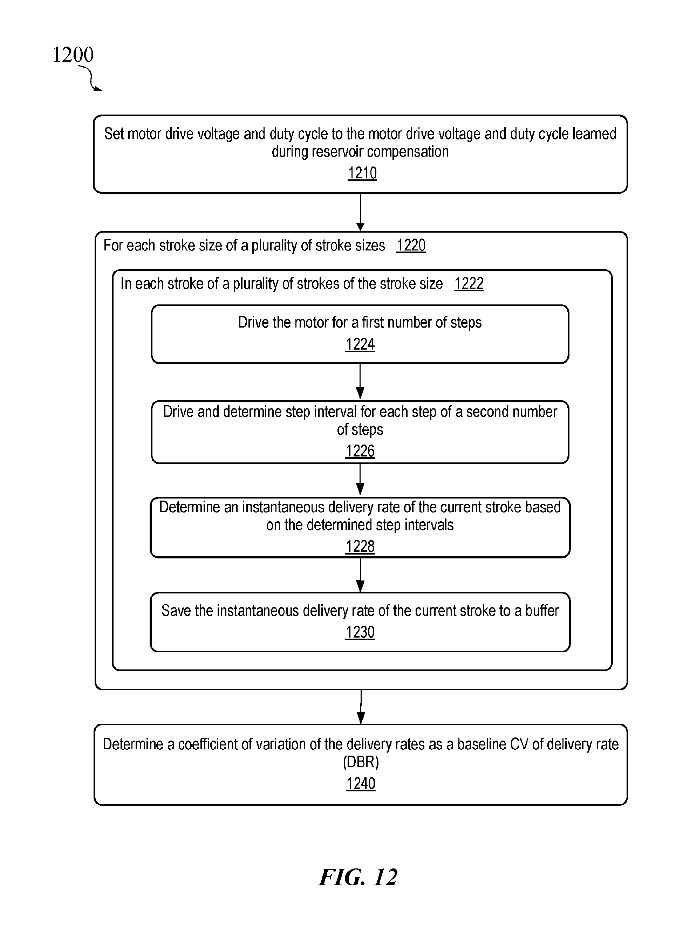

At block 728, a coefficient of variation of the delivery rate of the fluid delivery device may be determined during the next plurality of strokes. The coefficient of variation of the delivery rate may be saved and used as a baseline coefficient of variation of the delivery rate of the fluid delivery device for occlusion detection during fluid delivery. The baseline coefficient of variation of the delivery rate may be determined by driving the motor in strokes of various sizes using the calibrated motor drive voltage and/or motor drive duty cycle, measuring the delivery rate in each stroke, calculating a mean and a standard deviation of the delivery rates in the plurality of strokes, and determining the coefficient of variation based on the mean and standard deviation. As described above, the delivery rate may be determined by, for example, detecting the change of position of the motor using a position sensor (e.g., a Hall sensor) and measuring the time the motor takes to move from one position to another position in a step. More detail of the operations in block 728 is described below with respect to, for example, FIG. 12.

The operations in blocks 722-728 may be performed each time a reservoir is inserted into the fluid delivery device, and may be performed while the tubing of the infusion set is being flushed. After the calibration of the fluid delivery device and the reservoir, the fluid delivery device with the installed reservoir may be used to delivery fluid to a user at block 730 according to the calibrated motor drive voltage and/or motor drive duty cycle. For example, the motor may be driven in strokes of various step sizes to deliver fluid (e.g., insulin). During the fluid delivery, the instantaneous delivery rate may be continuously measured as described above to determine the moving coefficient of variation of delivery rate, an occlusion that may have occurred may be detected based on the changes in the moving coefficient of variation of delivery rate.

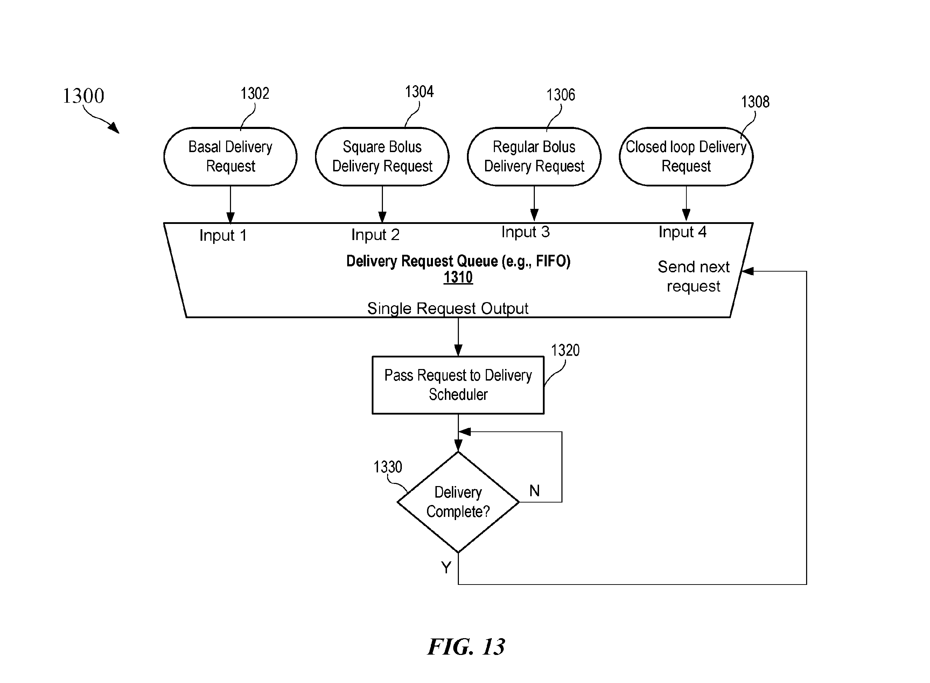

To deliver a certain amount of fluid in a certain period of time at block 730, a fluid delivery scheduler may determine at block 732, based on a fluid delivery request, parameters such as, for example, the number of delivery events, the delivery amount and number of strokes in a delivery event, the time interval between strokes, the size (e.g., the number of steps) of each stroke, and the like. The motor may then be controlled based on the fluid delivery scheduling determined at block 732 to deliver fluid in one or more events and one or more strokes in each event at block 734. For example, the motor may be driven using the calibrated motor drive voltage and/or motor drive duty cycle in one or more strokes of various stroke sizes to deliver a requested amount of fluid (e.g., insulin) at the scheduled rate. The fluid delivery may include, for example, basal insulin delivery, regular bolus delivery, square bolus delivery, or closed-loop insulin delivery. During the fluid delivery, the delivery rate may be continuously measured as described above. More detail of the operations in blocks 732 and 734 is described below with respect to, for example, FIGS. 13-18.

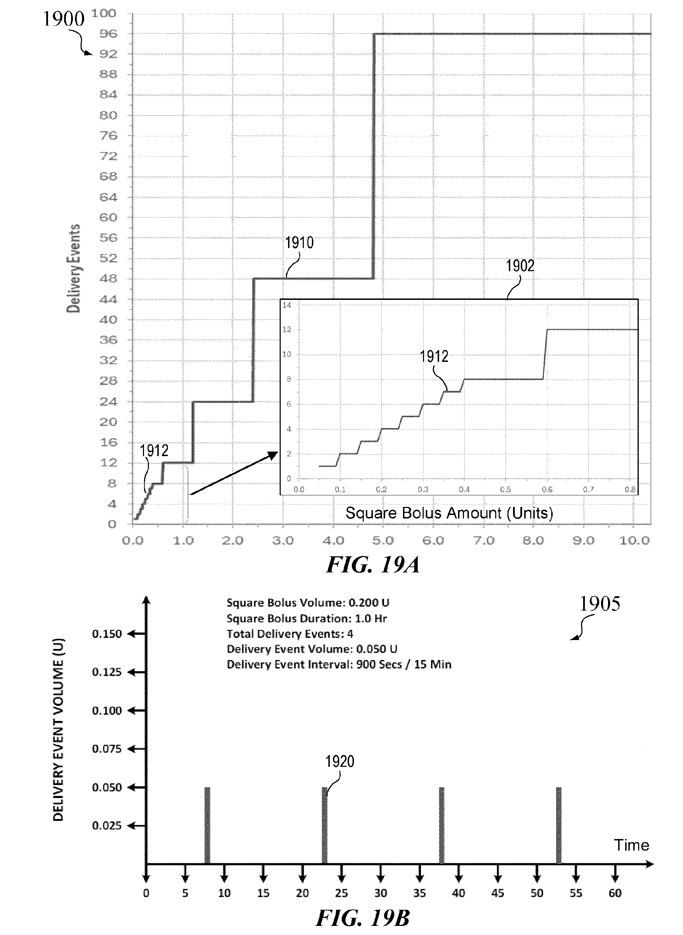

At block 736, the delivery rates measured during the fluid delivery may be used to detect possible occlusion of the fluid delivery. For example, the measured delivery rates may be filtered to keep the delivery rate reduction information, and the filtered delivery rates may be used to determine a moving coefficient of variation of delivery rate during each moving window of fluid delivery strokes. The change of the moving coefficient of variation of delivery rate with respect to the baseline coefficient of variation of the delivery rate determined at block 728 may be used to determine whether an occlusion has occurred. More detail of the operations in blocks 736 is described below with respect to, for example, FIG. 19.

## I. Manufacturing Calibration

In mass production, due to process variations, parameters of the manufactured components of fluid delivery devices, such as the motors, slides, sleeves, and other parts of the drive system of the fluid delivery device, may vary from part to part. While variations in some parameters of some components may have a negligible impact on pump functionality and performance, variations in some parameters of some components and/or the assembly of the components may significantly affect the performance of the fluid delivery device, such as the accuracy of the amount of fluid delivered, the sensitivity and the accuracy of plunger detection and occlusion detection, and the like. For example, since the motor rotation time or motor speed (and thus the delivery rate) is used for both reservoir setup (e.g., plunger detection) and occlusion detection, it may be desirable to know the relationship between the delivery system stall load and the motor drive voltage for each fluid delivery device, such that the motor in each fluid delivery device may be driven at an appropriate voltage for determining the motor speed/rotation time and the fluid delivery rate. The delivery system stall load may refer to the load applied axially to the slide that causes the motor to stall. For a same motor drive voltage, the stall load may vary from pump to pump due to variations in, for example, the motor torque constant, the gearbox friction, and the mating efficiency of the slide thread to the motor screw. These variations may cause the sensitivity and accuracy of occlusion detection to vary, such that some devices may not be able to detect occlusion in a timely manner (e.g., before 5 units of fluid are missed due to occlusion). Therefore, it may be desirable to calibrate the relationship between the delivery system stall load and the motor drive voltage for each fluid delivery device, which may be performed at the factory before the devices are provided to end users.

There may be several goals of the drive system calibration, including determining and storing the motor drive voltage (referred to as Manufacturing Motor Voltage (MMV)) and motor duty cycle (referred to as Manufacturing Duty Cycle (MDC)) for producing a target stall load (e.g., about 4.25 pounds) on each individual pump. The target stall load may be determined to balance between the desire to avoid a motor stall during an occlusion (e.g., where the back pressure may be between about 2.5 to 3.5 pounds) and the need to maintain a high level of sensitivity to changes in the motor load. If the target stall load is set to a value significantly higher than either the load at plunger detection or the load at occlusion, the motor speed reduction due to plunger detection or occlusion may not be detectable within the allowable amount of maximum missed delivery. Another goal of the drive system calibration is to measure the resultant no-load delivery rate (referred to as manufacturing delivery rate (MDR)) when the motor is driven at the determined motor drive voltage MMV and the determined motor duty cycle MDC, while no load is applied to the drive system. The manufacturing delivery rate (MDR) may be used as a reference to compensate for motor speed changes due to variations in the friction between the plunger and the barrel of the user-installed reservoir.

FIG. 8 includes a flowchart 800 illustrating an example of a process of manufacturing calibration of a fluid delivery device according to certain embodiments. Flowchart 800 may be an example of an implementation of the operation in block 710. Operations in flowchart 800 may be performed using, for example, a production tester. In flowchart 800, the motor drive voltage and motor drive duty cycle for producing a target stall load may be measured at multiple positions. The average or median values of the measurement results at the multiple positions may be used as the Manufacturing Motor Voltage (MMV) and Manufacturing Duty Cycle (MDC). The delivery rate when the motor is driven at the MMV and MDC with no load applied may then be measured and used as the manufacturing delivery rate (MDR). Although flowchart 800 may describe the operations as a sequential process, some of the operations may be performed in parallel or concurrently. In addition, the order of the operations may be rearranged. The process may have additional steps not included in the figure. Some operations may be optional or may be omitted. Some operations may be merged with operations in another block, or may instead be performed in another block.

In the illustrated example, operations in flowchart 800 may include setting the slide of the drive system of a fluid deliver device (without reservoir installed) at a home position (e.g., the total number of forward drive steps is zero) at block 810. At block 820, the motor may be driven at an initial (e.g., default) voltage level and duty cycle for a first number of steps to move the slide to a position. The motor may be driven in strokes, where each stroke may include multiple steps. At block 830, the motor drive voltage and motor drive duty cycle for producing a target stall load at the position may be measured by, for example, applying the target load to the slide, increasing the motor drive voltage to achieve a threshold delivery rate, and then gradually reducing the motor drive voltage and/or motor drive duty cycle until the motor stalls. The motor drive voltage and motor drive duty cycle at the time the motor starts to stall may be stored. The motor may then be driven for a second number of steps to move the slide to another position, and the motor drive voltage and motor drive duty cycle for producing the target stall load at the position may be measured as described above with respect to block 830. Operations at blocks 820 and 830 may be performed multiple times at multiple locations.

At block 840, the differences between the measured motor drive voltages at the multiple positions may be determined. If the maximum difference is greater than a threshold values, the calibration may fail. If the maximum difference is at or below the threshold value, the median or average of the measured motor drive voltages may be determined at block 850. At block 860, if the median or average of the measured motor drive voltages is above a threshold voltage level, the median or average of the measured motor drive voltages may be stored as the manufacturing motor voltage at block 870. The median or average of the measured motor drive duty cycles may also be saved as the manufacturing duty cycle. If the median or average of the measured motor drive voltages is below the threshold voltage level, a default manufacturing motor voltage (e.g., the threshold voltage level) may be used as the manufacturing motor voltage at block 865. The MMV and MDC may be stored in a non-volatile memory of the fluid delivery device, and/or may be saved to a database on a server.

After the MMV and MDC for producing the target stall load are determined for the fluid delivery device, the motor may be driven at the MMV and MDC, with no load applied to the slide, and the manufacturing delivery rate (MDR) may be determined at block 880 based on, for example, the time interval for the motor to move from one step position to the next step position, or the time for the motor to rotate a full circle. The MDR may also be stored in a non-volatile memory of the fluid delivery device, and/or may be saved to a database on a server. The MDR may be used, for example, for reservoir compensation as described in detail below.

## II. Reservoir Setup

An insulin pump may generally use an insulin reservoir or cartridge for storing insulin to be delivered. Some insulin pump may integrate the reservoir within the body of the insulin pump, and thus may be disposable after the reservoir is empty. But many insulin pumps are reusable pumps, where the insulin reservoir may be replaceable (or refillable) by a user, such that the reservoir may be replaced after the patient consumes all insulin in the reservoir (e.g., within 3-7 days) or after the reservoir has been in the insulin pump for a long time period, to provide continuous insulin delivery and to prevent insulin degradation or potential infection. Many other portions of the insulin pump, such as the housing, the controller, the motor, the drive train components, the user interface, and the communication interface, may be reused to reduce the overall cost in a long term.

Many different types of insulin reservoirs or cartridges may be used in insulin pumps. For example, as described above with respect to FIGS. 5-7, some insulin pumps may use syringe-type reservoirs that may include a hollow plastic barrel with one open end, a septum on the closed end, and a plunger (also referred to as a stopper) with rubber O-rings within the barrel. The plunger may be pushed linearly by the motor and the drive train of the insulin pump from the open end, and the linear motion of the plunger may displace the fluid from the reservoir to cause fluidic delivery through a needle piercing the septum (e.g., from an infusion set). The motor may be driven to rotate around an axis, and the drive train may convert the rotational motion of the motor to linear motion of the plunger, and may include, for example, a slide for pushing the plunger and a sleeve that causes the slide to move linearly.

Different users may use different total daily doses (TDDs) of insulin, and thus may need different amounts of insulin for a same time period. As such, some reservoirs and fluid delivery devices may allow the patients to fill the reservoirs to any volume that may be suitable for them. Therefore, the insulin pumps may need to be able to automatically determine the initial location of the plunger within the reservoir barrel and the volume of the insulin stored in each reservoir during reservoir setup. Some insulin pumps may determine the fill volume of the reservoir and the plunger position in the reservoir by driving the motor forward at a high speed and measuring the resultant back pressure using a strain sensor. For example, when the slide reaches the plunger position, the back pressure may increase drastically and may be detected by the strain sensor to indicate the detection of the plunger. As discussed above, incorporating a strain sensor into an insulin pump may increase the mechanical complexity as the entire drive system may need to "float" to allow the back pressure generated by the fluid due to the pressure applied to the fluid by the slide through the plunger or the back pressure caused by an occlusion to be detected by the strain sensor. The presence of the strain sensor may also reduce the reliability of the insulin pump due to, for example, the sensor fragility and the inability to fully secure the drive system, which may cause the insulin pump to be more susceptible to damage from shock and drop. In addition, strain sensors with sufficient accuracy and resolution for plunger and occlusion detection may be expensive, and thus may significantly increase the cost of an insulin pump.

According to certain embodiments disclosed herein, the plunger detection during reservoir setup may be performed using signals from motor position sensors (e.g., Hall effect sensors) that are also used for determining the delivery amount and/or speed of fluid delivery motor, instead of using a separate strain sensor or other sensors. For example, during the reservoir setup, the motor may be driven at a voltage level determined during the factory calibration procedure, and the motor rotational speed or motor rotation time may be determined using the motor position sensor. A baseline coefficient of variation (CV) of the motor rotation time (or motor rotational speed) may be determined during a first plurality of rotations of the motor when no load is applied to the motor slide. After the baseline CV of the motor rotation time is established, subsequent motor speed or rotation time measurements may be filtered by a series of mathematical and statistical filters (e.g., a maximum value filter) to generate a moving coefficient of variation of the motor rotation time. The moving coefficient of variation may be compared against the baseline coefficient of variation to detect the plunger. For example, when the moving coefficient of variation exceeds the baseline CV by a specified amount, the plunger may be detected. In this way, plunger detection may be performed during reservoir setup without the need for a separate strain sensor or another type of sensor, thereby reducing the design and control complexity, improving the reliability, and reducing the overall cost of the fluid delivery device.

FIG. 9 includes a flowchart 900 illustrating an example of a process of determining a baseline coefficient of variation of motor rotation time with no load applied in a fluid delivery system according to certain embodiments. Flowchart 900 may be an example of an implementation of the operation in block 722. Operations in flowchart 900 may be performed using, for example, a controller or a processor of delivery device 102, insulin delivery subsystem 230, fluid delivery device 300, fluid delivery device 400 (without using sensor assembly 510), infusion device 602, and the like. Although flowchart 900 may describe the operations as a sequential process, some of the operations may be performed in parallel or concurrently. In addition, the order of the operations may be rearranged. The process may have additional steps not included in the figure. Some operations may be optional or may be omitted. Some operations may be merged with operations in another block, or may instead be performed in another block.

Operations in flowchart 900 may include, after a reservoir is loaded into a fluid delivery device, continuously driving the motor steadily (rather than in strokes) at block 910 using the manufacturing motor voltage and manufacturing duty cycle determined and stored during the manufacturing calibration, with no additional loaded applied to the slide and motor. While the motor is driven continuously, a motor position sensor (e.g., a Hall sensor that may provide different output values when the motor is at different positions within a full rotation) may be checked to ensure that the motor position sensor functions properly, such as providing the correct output values and being able to detect the motor position changes. For example, if the output value of the motor position sensor does not change after the motor is driven to rotate a step, the motor position sensor may not function properly, or the motor may not function properly or may stall. The motor may also be checked to make sure that the motor drive voltage is at the programmed level and the motor drive current is below a threshold value. If any of these checks of the motor position sensor and motor checks fails (once or for a threshold number of times), the reservoir setup process may be terminated. The total number of forward driving steps may also be continuously checked against a threshold value that may correspond to an empty reservoir. If the current total number of forward driving steps is greater than the threshold value before the reservoir setup process completes, the reservoir may be empty, and the reservoir setup process may be terminated. In some embodiments, an alarm may be generated if any of the checks fails.

After the motor is controlled to rotate steadily for a certain number of steps (e.g., threshold number of steps to skip), the motor rotation time (MRT) may be continuously measured at block 920. For example, the time interval between any two consecutive steps may be measured, or the time for the motor to rotate a certain number of steps may be measure. In one example, the motor may be designed to rotate a full circle in 6 steps, and the total time for the motor to rotate 6 steps may be measured or determined and used to calculate a motor rotation time. In some examples, a timer for time measurement may be triggered, for example, when the motor position sensor outputs a new value indicating a new position of the motor, and the timer may be stopped when the motor position sensor outputs another value indicating that the motor reaches the next position, such that the value of the timer may correspond to the time that the motor takes to move from one position to the next position.

At blocks 930, the measured MRT for each rotation may be saved to a buffer. The motor rotation time may be measured for a certain number of rotations and saved to the buffer until it is determined at block 935 that buffer is full. If the buffer is not full, a new MRT may be measured while the motor is driven steadily, and the new MRT may be saved to the buffer. If the buffer is full, the motor rotation time values saved in the buffer may be used to determine a coefficient of variation of the motor rotation time values saved in the buffer at block 940. The coefficient of variation of the motor rotation time values may be determined by determining the mean and the standard deviation of the motor rotation time values, and determining the coefficient of variation as a value equal to the standard deviation divided by the mean.

At block 942, if the total number of forward drive steps is lower than a threshold number of steps, a baseline coefficient of variation of motor rotation time may be determined at block 950 based on the coefficient of variation of the motor rotation time determined at block 940. For example, the baseline CV of motor rotation time may be the product of a factor and the coefficient of variation of the motor rotation time determined at block 940. In some examples, if the baseline CV of motor rotation time is lower than a threshold value, the baseline CV of motor rotation time may be set to the threshold value. If the total number of forward drive steps is greater than the threshold number of steps, the coefficient of variation of the motor rotation time determined at block 940 may be used to determine if the plunger of the reservoir is detected.

FIG. 10 includes a flowchart 1000 illustrating an example of a process of detecting a plunger of a reservoir in a fluid delivery system according to certain embodiments. Flowchart 1000 may be an example of an implementation of the operation in block 724. Operations in flowchart 1000 may be performed using, for example, a controller or a processor of delivery device 102, insulin delivery subsystem 230, fluid delivery device 300, fluid delivery device 400 (without using sensor assembly 510), infusion device 602, and the like. Although flowchart 1000 may describe the operations as a sequential process, some of the operations may be performed in parallel or concurrently. In addition, the order of the operations may be rearranged. The process may have additional steps not included in the figure. Some operations may be optional or may be omitted.

Operations in flowchart 1000 may include, after a baseline coefficient of variation of motor rotation time is determined (e.g., as described above with respect to flowchart 900 of FIG. 9), continuously driving the motor steadily (rather than in strokes) at block 1010 using the manufacturing motor voltage and manufacturing duty cycle determined and stored during the manufacturing calibration, with no additional loaded applied to the motor and slide. While the motor is driven continuously, the motor position sensor (e.g., a Hall sensor) may be checked to ensure that the motor position sensor functions properly, such as providing the correct output values and being able to detect the motor position changes. For example, if the output value of the motor position sensor does not change after the motor is driven to rotate a step, the motor position sensor may not function properly, or the motor may not function properly or may stall. The motor may also be checked to make sure that the motor drive voltage is at the programmed level and the motor drive current is below a threshold value. If any of these motor position sensor and motor checks fails (once or a threshold number of times), the reservoir setup process may be terminated. The total number of forward driving steps may also be continuously checked against a threshold value that may correspond to an empty reservoir. If the current total number of forward driving steps is greater than the threshold value before the reservoir setup process completes, the reservoir may be empty, and the reservoir setup process may be terminated. In some embodiments, an alarm may be generated if any of the checks fails.

While the motor is controlled to rotate steadily, the motor rotation time MRT may be continuously measured at block 1020. For example, the time interval between any two consecutive steps may be measured, or the time for the motor to rotate a certain number of steps may be measure. In one example, the motor may be designed to rotate a full circle in 6 steps, and the total time for the motor to rotate 6 steps may be measured or determined and used to calculate a motor rotation time. In some examples, the time measurement may be triggered, for example, when the motor position sensor outputs a new value indicating a new position of the motor, or the output value of the motor position sensor has cycled through the values for different positions, which indicates that the motor has rotated a full circle. The motor rotation time may be measured for a certain number of rotations.

At blocks 1030-1036, the measured MRT for each rotation may be filtered using a maximum filter and results of the filtering may be saved to a buffer. For example, at block 1030, the current MRT may be compared with the previous maximum rotation time (RTM) of the motor rotation time measurements saved in the buffer. If the current MRT is lower than the previous RTM of the motor rotation time measurements saved in the buffer, the number of consecutive rotations during which no larger RTM has occurred may be compared with a threshold value at block 1032. If the number of consecutive rotations during which no larger RTM has occurred is lower than the threshold value, the previous RTM may be saved to the buffer at block 1036. If the number of consecutive rotations during which no larger RTM has occurred is greater than the threshold value, the previous RTM may be reduced by a certain value at block 1034, and the current MRT may be set as the new RTM and saved to the buffer at block 1036 if the reduced RTM is lower than the current MRT; otherwise, the reduced RTM may be saved to the buffer at block 1036.

If the current MRT is greater than the previous RTM of the motor rotation time measurements saved in the buffer, a new maximum rotation time RTM may be determined at block 1036 based on, for example, a weighted sum of the current MRT and the existing maximum rotation time RTM of the motor rotation time measurements in the buffer. The weights for the current MRT and existing RTM may be selected to increase or smooth the variation of the motor rotation time. For example, the weight for the current MRT may be between 0 and 1, where a weight value 1.0 may maximize the variation, and a weight value 0 may minimize the variation.

At block 1038, if the buffer is not full, a new MRT may be measured while the motor is driven steadily, and the new MRT may be processed as described above with respect to blocks 1030-1036. If the buffer is full, the rotation time values (e.g., RTMs) saved in the buffer may be used to determine a coefficient of variation of the RTM values saved in the buffer at block 1040. The coefficient of variation of the RTM values may be determined by determining the mean and the standard deviation of the RTM values, and determining the coefficient of variation as the ratio of the standard deviation relative to the mean. The buffer may have a size of a few hundred, such as about 100 or 200. The coefficient of variation of the RTM value may be a moving coefficient of variation using a moving window of the past a few hundred rotations of forward drive of the motor. A change of the coefficient of variation of the RTM values with respect to the baseline CV of motor rotation time determined in flowchart 900 may then be determined. For example, the change of the coefficient of variation with respect to the baseline CV of motor rotation time may be expressed as a percentage of the baseline CV of motor rotation time.

At block 1042, the change of the coefficient of variation may be compared with a threshold value, such as 100% or another value (e.g., 50% or larger). If the change of the coefficient of variation is greater than the threshold value, the plunger of the reservoir may be detected, and the motor may be stopped at block 1050. If the change of the coefficient of variation is not greater than the threshold value, the plunger of the reservoir may not have been detected, and the operations in blocks 1010-1042 may be performed again, until the change of the coefficient of variation is greater than the threshold value.

It is noted that even though motor rotation time is used for plunger detection in the example shown in FIG. 10, plunger location may be detected based on other parameters that may correlate with the motor rotation time, such as motor rotational speed or motor delivery rate. After the plunger is detected, friction measurement and compensation may be performed for the reservoir to determine the appropriate drive voltage for achieve a desired delivery rate from the reservoir as described in detail below.

In reusable insulin pumps that use replaceable insulin reservoirs, due to the high-volume manufacturing and lower cost of insulin reservoirs, the friction between the plastic barrel and the plunger (e.g., O-rings on the plunger) may vary from reservoir to reservoir. The variation in friction may make it difficult to accurately and timely determine whether the back pressure is due to the reservoir friction or a flow blockage, and thus may make it difficult to detect an occlusion quickly and accurately. The variation in friction may also cause variation in the fluid delivery rate under a same motor drive voltage, which may affect the accuracy of the amount of insulin delivered.

According to certain embodiments disclosed herein, the variation in the friction between the plunger (and the O-rings) and the reservoir barrel may be measured and compensated during the reservoir setup, such that appropriate respective motor drive voltages and duty cycles may be used to drive the specific reservoirs to achieve a more constant or consistent delivery rate, thereby enabling more accurate and timely detection of a real occlusion based on the changes in the fluid delivery rate measured by the motor position sensors. In one example, a reference motor speed (or a reference fluid delivery rate) at no load and under a motor drive voltage level and a motor drive duty cycle that have been calibrated against a known load during manufacturing calibration of each insulin pump may be measured during the manufacturing calibration, and may be saved to the insulin pump. After an insulin reservoir is positioned in the insulin pump, the motor may be driven using a series of strokes (e.g., each including a plurality of steps). A controller may obtain data from a motor position sensor to determine a motor speed (or rotation time) or fluid delivery rate during a plurality of strokes, and compare the measured motor speed or fluid delivery rate against the reference speed or fluid delivery rate. If the measured fluid delivery rate is lower than the reference fluid delivery rate, the reservoir friction may cause the delivery rate reduction and the motor voltage may be increased to compensate for the delivery rate reduction. This process may be repeated until the measured delivery rate is equal to or greater than the reference delivery rate. The reservoir friction measurement and compensation may be performed in a small number of strokes while the tube connecting the reservoir to a cannular is being flushed. The reservoir friction measurement and compensation technique disclosed herein may allow the insulin pump to compensate for the unique friction presented by each insulin reservoir and allow more accurate and timely occlusion detection, thereby providing a better user experience and improves clinical outcomes.

**FIG. 11** includes a flowchart 1100 illustrating an example of a process of measuring and compensating friction between a plunger and a barrel of a fluid reservoir in a fluid delivery system according to certain embodiments. Flowchart 1100 may be an example of an implementation of the operation in block 726. Operations in flowchart 1100 may be performed using, for example, a controller or a processor of delivery device 102, insulin delivery subsystem 230, fluid delivery device 300, fluid delivery device 400 (without using sensor assembly 510), infusion device 602, and the like.

At block 1110, the motor drive voltage and the motor drive duty cycle may be set to the MMV and MDC measured during the factory calibration as described above with respect to, for example, FIGS. 7 and 8. As described above, the MMV and MDC may be stored in a non-volatile memory in the fluid delivery device during the manufacturing calibration. The motor may be driven in strokes, where each stroke may include multiple steps. In some embodiments, the number of steps (e.g., 5 or 7 steps) in each stroke may be different from the number of steps (e.g., 6 steps) for the motor to rotate a full circle. The number of steps in each stroke may be a constant number or may be variable from stroke to stroke. The strokes may be similar to the strokes performed during normal fluid delivery.

Even though not shown in FIG. 11, in some embodiments, before and/or during the operations in flowchart 1100, the motor may be checked to make sure that the motor drive voltage is at the programmed level and the motor drive current is below a threshold value. The motor position sensor (e.g., a Hall sensor that may provide different output values when the motor is at different positions within a full rotation) may be checked to ensure that the motor position sensor functions properly, such as providing the correct output values and being able to detect the motor position changes. For example, if the output value of the motor position sensor does not change after the motor is driven to rotate a step, the motor position sensor may not function properly, or the motor may not function properly or may stall. If any of these motor position sensor and motor checks fails (once or for a threshold number of times), the process of flowchart 1100 may be terminated. In some embodiments, an alarm may be generated if any of the checks fails.

At block 1120, in each strokes of a plurality of strokes (e.g., 3-5 strokes), the motor may be driven to rotate multiple steps, and the time intervals between some steps may be measured to determine a current delivery rate of the fluid delivery device. In the illustrated example, at block 1122, the motor may be driven for a first number of steps, such as 3 or 4 steps, so that the motor may have a relatively steady speed. At block 1124, during each step of a second number of steps (e.g., steps 3-5) after the first number of steps, the time interval for the motor to move from one position to the next position may be determined. For example, a timer may be used to count the time for the motor position sensor to change its output from one value to another value.

An instantaneous delivery rate for the current stroke may then be determined at lock 1126 based on the measured time intervals of the second number of steps. For example, the instantaneous delivery rate may be determined based on the amount of delivery in each motor step, and the time for the motor to rotate from one position to the next position in the motor step. In another example, the instantaneous delivery rate may be determined based on the amount of delivery in each step, and an average time for the motor to rotate one step as determined based on the time it takes the motor to rotate in each step of step 3-5 of each stroke. At block 1128, the instantaneous delivery rate for the current stroke may be saved to a buffer for friction compensation. After each stroke, the motor may be stopped, for example, by setting all windings of the motor to ground, and disabling the motor power supply. In some examples, the motor position sensor may continue to monitoring any possible forward or backward rotation steps of the motor, so that the total number of forward rotation steps may be tracked for a reservoir. Operations of blocks 1122-1128 may be performed for a plurality of motor strokes, until the buffer is full. As described above, the sizes of the strokes may be different to replicate the strokes of normal fluid delivery.

After the buffer is full, a pre-compensation delivery rate (PDR) of the fluid delivery device may be determined at block 1130, based on the instantaneous delivery rates saved in the buffer. For example, the pre-compensation delivery rate may be the average of the instantaneous delivery rates saved in the buffer. The pre-compensation delivery rate may indicate the impact of the reservoir friction on the delivery rate and may vary from reservoir to reservoir. The PDR may be saved and used to determine the amount of adjustment to the motor drive voltage for friction compensation.

At block 1132, the PDR may be compared with the MDR determined during the manufacturing calibration. As described above, the MDR may be stored in a non-volatile memory in the fluid delivery device during the manufacturing calibration. If the PDR is equal to or greater than MDR, the friction calibration process may be completed as block 1102, and the MMV and MDC determined during the manufacturing calibration may be saved and used for delivering the fluid during normal operation. If the PDR is lower than the MDR, the motor drive voltage and/or duty cycle may need to be adjusted as described in more detail below.

At block 1140, the motor drive voltage and/or the motor drive duty cycle may be adjusted, for example, by a voltage incremental step or based on extrapolation using MDR and PDR. In one example, if the motor drive voltage is being adjusted for the first few times, a predetermined voltage incremental step (e.g., about 100 mV) may be added to the current motor drive voltage; if the motor drive voltage has been increased for multiple times but the measured delivery rate is still lower than the MDR, the motor drive voltage may be determined by extrapolation based on the amount of delivery rate increase caused by the drive voltage increase during the previous few adjustment steps, and the total difference between the PDR and MDR, to speed up the compensation process.

After block 1150, in each strokes of a plurality of strokes (e.g., 3-5 strokes), the motor may be driven by the adjusted drive voltage and/or duty cycle to rotate multiple steps, and the time intervals between some steps (e.g., steps 3-5) of the stroke may be measured to determine a current delivery rate of the fluid delivery device. For example, at block 1152, the motor may be driven for a first number of steps, such as 3 or 4 steps, so that the motor may have a relatively steady speed. At block 1154, during each step of a second number of steps (e.g., steps 3-5) after the first number of steps, the time interval for the motor to move from one position to the next position in the step may be determined. For example, a timer may be used to count the time for the motor position sensor to change its output from one value to another value. An instantaneous delivery rate for the current stroke may then be determined based on the measured time intervals of the second number of steps. For example, the instantaneous delivery rate may be determined based on the amount of delivery in each step, and an average time for the motor to rotate one step as determined based on the time it takes the motor to rotate in each step of step 3-5 of each stroke. At block 1158, the instantaneous (or current) delivery rate for the current stroke may be saved to the buffer for friction compensation. After each stroke, the motor may be stopped, for example, by setting all windings of the motor to ground. In some examples, the motor position sensor may continue to monitoring any possible forward or backward rotation steps of the motor, so that the total number of forward steps may be tracked for a reservoir. Operations of blocks 1152-1158 may be performed for a plurality of motor strokes, until the buffer is full. In some examples, the sizes (numbers of steps) of the strokes may be different.

After the buffer is full, a reservoir compensation delivery rate (RCR) of the fluid delivery device may be determined at block 1160, based on the instantaneous (or current) delivery rates saved in the buffer. For example, the RCR may be the average of the instantaneous delivery rates saved in the buffer. At block 1162, the RCR may be compared with the MDR determined during the manufacturing calibration. If the RCR is equal to or greater than the MDR, the friction calibration process may be completed as block 1102, and the current motor drive voltage and duty cycle may be saved and used for delivering the fluid during normal operation. If the RCR is still lower than the MDR, operations at blocks 1140-1162 may be performed again, until the RCR is equal to or greater than the MDR. As described above, in some examples, an extrapolation may be used for determining the adjustment to the motor drive voltage and/or motor drive duty cycle, in order to speed up the calibration process.

The final motor drive voltage and duty cycle may be stored and used to drive the motor for fluid delivery during normal operations of the fluid delivery device. If the RCR is still lower than the MDR after a threshold number of motor drive voltage and/or cycle time adjustments (e.g., iterations of operations in blocks 1140-1162), the reservoir calibration may have failed, and an alarm or a notification may be generated. After the reservoir calibration is complete, a baseline coefficient of variation of the fluid delivery rate of the fluid delivery device may be determined and used as a reference for occlusion detection during normal operations of the fluid delivery device.

FIG. 12 includes a flowchart 1200 illustrating an example of a process of determining a baseline coefficient of variation of delivery rate of a fluid delivery device according to certain embodiments. Flowchart 1200 may be an example of an implementation of the operation in block 728. The baseline coefficient of variation of delivery rate may be determined based on the delivery rates measured during multiple strokes for each stroke size of several different stroke sizes that may be used during normal fluid delivery operations. Operations in flowchart 1200 may be performed using, for example, a controller or a processor of delivery device 102, insulin delivery subsystem 230, fluid delivery device 300, fluid delivery device 400 (without using sensor assembly 510), infusion device 602, and the like. Operations in flowchart 1200 may be performed during reservoir setup, for example, after performing the operations in flowchart 1100.

At block 1210, the motor drive voltage and duty cycle of a PWM signal for driving the motor of a fluid delivery device may be set to the motor drive voltage and duty cycle learned during reservoir compensation, for driving the motor in strokes of different sizes. Even though not shown in FIG. 12, in some embodiments, before and/or during the operations (e.g., each stroke) in flowchart 1200, the motor may be checked to make sure that the motor drive voltage is at the programmed level and the motor drive current is below a threshold value. The motor position sensor (e.g., a Hall sensor) may also be checked to ensure that the motor position sensor functions properly, such as providing the correct output values and being able to detect the motor position changes. For example, if the output value of the motor position sensor does not change after the motor is driven to rotate a step, the motor position sensor may not function properly, or the motor may not function properly or may stall. If any of these motor position sensor and motor checks fails (once or a threshold number of times), the process of flowchart 1200 may be terminated. In some embodiments, an alarm may be generated if any of the checks fails.

At block 1220, the motor may be controlled to rotate a few strokes for each stroke size of multiple different stroke sizes, and the fluid delivery rate may be measured in each stroke. As shown in FIG. 12, at block 1220, for each stroke size of a plurality of stroke sizes, operations in block 1222 may be performed. The operations in block 1222 may include, for each stroke of a plurality of strokes of a stroke size, driving the motor for a first number of steps (e.g., 3-4 steps or another number of steps) at block 1224, driving the motor for a second number of steps (e.g., 2 or more steps) and determining the time interval for the motor to move from one position to the next position during each step of the second number of steps using a motor position sensor and a timer at block 1226 as described above, determining an instantaneous (or current) delivery rate for the current stroke at block 1228 based on the measured time intervals of the second number of steps, and saving the instantaneous delivery rate for the current stroke to a buffer at block 1230. After each stroke, the motor may be stopped, for example, by setting all windings of the motor to ground and disabling the motor power supply. In some examples, the motor position sensor may continue to monitor any possible forward or backward rotation steps of the motor, so that the total number of forward rotation steps may be tracked for a reservoir.

At each stroke size, operations at blocks 1224-1230 may be performed a plurality of times in a plurality of motor strokes. The motor may then be driven according to another stroke size for a plurality of strokes, during which an instantaneous delivery rate may be determined for each stroke. In one example, the motor may be controlled to rotate at 4 different stroke sizes, where each stroke size may correspond to a different respective number of steps (e.g., a number between 4 and 32), and, for each stroke size, the motor may be driven to delivery fluid for 6 strokes. Therefore, in this example, the fluid delivery rate may be measured in 24 strokes in block 1220, and the buffer may include 24 instantaneous delivery rates.

At block 1240, a coefficient of variation of the delivery rates saved in the buffer may be calculated and used as a baseline coefficient of variation BDR. The coefficient of variation may be calculated by computing the mean and standard deviation of the delivery rates saved in the buffer, and dividing the standard deviation by the mean. In some examples, if the calculated coefficient of variation is lower than a minimum threshold value, the minimum threshold value may be used as the baseline coefficient of variation.

### III. Insulin Scheduling and Delivery

Insulin therapy using an insulin pump may include several modes of delivery, such as bolus insulin delivery and basal insulin delivery. Bolus insulin may be delivered at a larger dose around meal time or any other time when a patient's glucose level is significantly higher than the desired target. While a typical bolus may be delivered with a series of successive strokes in rapid succession, a basal insulin delivery and a square bolus insulin delivery may be scheduled as a series of strokes over a longer time period, such as 30 minutes or longer. Insulin pumps that use a limited set of stroke sizes may need to use a complex scheduling scheme for basal delivery, and may have a higher power consumption for the delivery because most basal rates may need to be achieved using small strokes every minute or every few minutes. While the scheduling scheme may ultimately result in the correct amount of insulin being delivered, each specific basal rate may result in a different series of strokes and time intervals between strokes, which may be difficult to predict.

According to certain embodiments disclosed herein, a series of multiple quick checks may be performed to determine how many delivery events need to be performed for each basal rate (including temporary basal rate) or square bolus rate, and the insulin delivery may be evenly divided across the calculated number of delivery events. This scheduling scheme may result in a uniform and predictable series of basal deliveries, and may also greatly reduce the number of deliveries performed for a given basal rate, which allows the delivery motor to operate more efficiently. Square bolus may also be scheduled and delivered in a similar manner. In each delivery event, the insulin may be delivered in one or more strokes, where the stroke size may be more flexible to accommodate different requested amounts. The size of each stroke may be selected to match the requested amount closely and also reduce the number of strokes, such that the power consumption for the delivery may be reduced.

**FIG. 13** includes a flowchart 1300 illustrating an example of a process of scheduling and delivering fluid using a fluid delivery system according to certain embodiments. Flowchart 1300 may be an example of an implementation of the operation in block 730. Operations in flowchart 1300 may be performed using, for example, a controller or a processor of delivery device 102, insulin delivery subsystem 230, fluid delivery device 300, fluid delivery device 400 (without using sensor assembly 510), infusion device 602, and the like. In the illustrated example, the fluid to be delivered by the fluid delivery device may be insulin, which may be delivered as basal insulin, regular bolus insulin, square bolus insulin, or insulin dynamically determined in a closed loop based on, for example, measured glucose level data and other data associated with a user of the fluid delivery device.

As shown in FIG. 13, flowchart 1300 may include receiving one or more insulin delivery requests at block 1302 (e.g., a basal delivery request), block 1304 (e.g., a square bolus delivery request), block 1306 (e.g., a regular bolus delivery request), or block 1308 (e.g., a closed loop insulin delivery request). The one or more requests may be stored in a delivery request queue at block 1310. The delivery request queue may include, for example, a first-in-first-out (FIFO) buffer. The requests may be saved in the delivery request queue based on, for example, the order in which the one or more requests are received, and/or the priorities of the one or more requests. The requests saved in the delivery request queue may be passed to a delivery scheduler (e.g., implemented using a controller or a processor) one at a time at block 1320. The delivery request queue may ensure that the insulin scheduler may receive a single delivery request at a time, and subsequent requests are held until the previous request has been completed. This technique can minimize the overall complexity of the delivery algorithm, and ensure that the specified delivery rate or amount can be achieved regardless of the number of delivery requests received.

The delivery scheduler may determine, for example, the number of delivery events per unit time (or the time interval between the delivery events), the amount of insulin to deliver for each event, the number of strokes or steps in each delivery event, the time interval between strokes, the number of steps in each stroke, and the like. Based on the determined delivery schedule, the motor may be controlled to deliver the insulin. The controller may check if the delivery for the current request is complete at block 1330. If the delivery for the current request is not complete yet, the controller may wait for the delivery to complete. After the delivery for the current request is complete, the next request in the delivery request queue may be sent to the delivery scheduler for scheduling and delivery.

The delivery scheduler may use some input parameters to schedule the delivery in response to a delivery request. The input parameters may include, for example, the total volume, average delivery rate, delivery amount per rotation, steps per rotation, maximum step interval, largest stroke size, smallest stroke size, and the like. As described above, changes in the motor load may cause changes in the motor velocity when the motor is driven at the same voltage level and duty cycle, but the amount of fluid delivered in each motor step may not vary with the motor load. The delivery scheduler may provide step-by-step motor commutation control without the need for closed-loop speed control. For example, the total amount of delivery may be determined based on the number of forward motor drive steps, and a target delivery rate may be achieved using a proper stroke interval and stroke size (e.g., the number of steps in each stroke), without the need for measuring the actual motor rotation velocity and compensating for changes that may affect the motor velocity, such as the variation in motor load.

As described above, the delivery techniques disclosed herein utilize the manufacturing calibration process to determine an appropriate motor drive voltage and duty cycle for the drive system in each fluid delivery device, to ensure that the drive system in every fluid delivery device is sensitive to motor load changes and the changes in delivery rate can be correlated to relevant system events, such as occlusion. If the motor is driven with a drive voltage and/or duty cycle that is too high, the motor sensitivity may be reduced and the resultant delivery rate reduction due to an occlusion may not be detectable during fluid delivery. A specified average delivery rate can be achieved by modifying the time interval between delivery strokes, rather than by changing the motor speed. The actual speed or velocity of the motor in each stroke is not controlled and may vary throughout the duration of a stroke, as the load changes. The speed or velocity of the motor during the normal delivery may also vary from system to system.

One goal of any fluid delivery technique is an accurate amount of fluid delivery. The motor control of a fluid delivery device may inevitably introduce errors. In general, for insulin delivery, under-delivery is safer (to avoid hypoglycemic) for the patient and easier to correct than over-delivery. As such, techniques disclosed herein may be designed to deliver as close to the requested delivery volume as possible without exceeding it. Such techniques may result in a small under-delivery in each stroke due to the finite resolution of the motor rotor encoders (or motor position sensors, such as Hall effect sensors) and the motor step size. The under-deliveries may be accumulated until they are large enough (e.g., more than the volume for a minimum stroke size) to be added onto a subsequent delivery event, thereby compensating for the under-deliveries.

In some examples, the delivery techniques disclosed herein may divide each delivery stroke into an active drive phase and a braking phase. During the active drive phase, the motor may be driven, for example, using a PWM drive signal having a frequency about 20 kHz and the learned motor drive voltage and motor drive duty cycle, to move step by step in a single instance of continuous motor motion of each stroke. After a specified number of motor steps have been performed, braking may be applied to the motor, during which the motor windings may be shorted together. During the braking, excess motor energy may be dissipated as heat in the motor windings, and thus the motor may stop rapidly. Braking can help to minimize the amount of excess delivery (over-shoot) during each stroke, but may not eliminate it. However, the amount of excess delivery that may occur during the braking may be taken into consideration during, for example, the delivery scheduling, such that the amount of fluid delivered during the active drive phase may be adjusted (e.g., reduced) by the corresponding amount to compensate for any excess delivery during the braking.

In addition, in the delivery techniques disclosed herein, a plurality of variable stroke sizes, rather than only a few discrete stroke sizes, may be used for the fluid delivery. For example, the delivery techniques allow strokes of any stroke size between the specified minimum and maximum stroke sizes to be performed. The maximum stroke size may be selected such that any occlusion can be quickly detected based on the change of the current delivery rate measured in each stroke. Using variable stroke sizes may reduce the complexity of smaller delivery requests by enabling them to be completed in a single stroke. Using variable stroke sizes may also reduce the energy consumption of the delivery system by enabling larger deliveries to be completed in fewer, larger strokes. Reducing the number of strokes for a delivery request may reduce the number of motor starts, which may be the most inefficient phase of a motor sequence. In addition, by allowing the motor to run longer during a given stroke, the motor can reach a steady-state velocity, which may be the most efficient phase of a motor sequence.

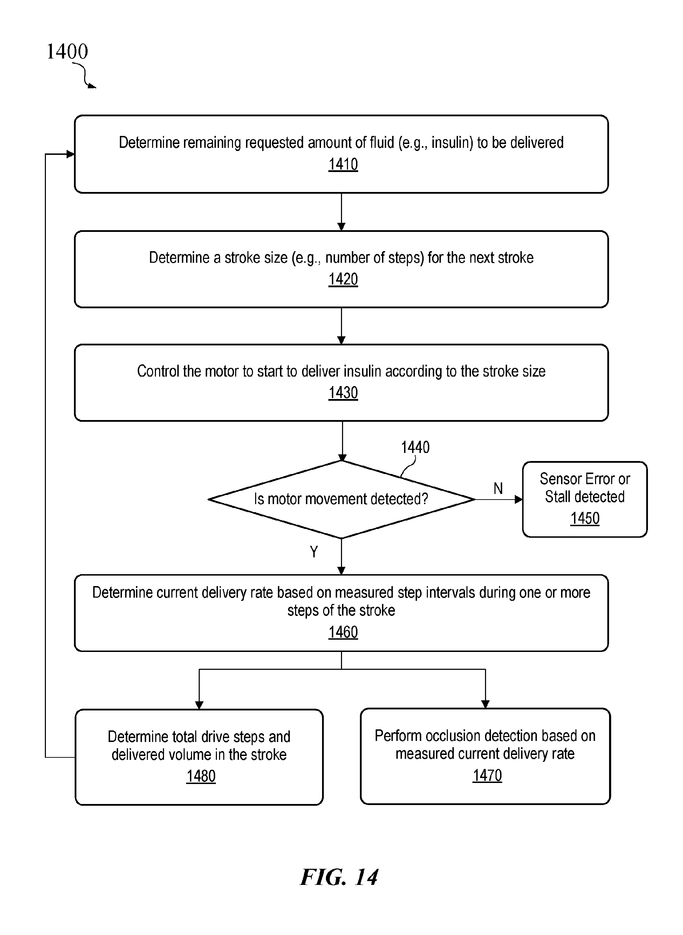

FIG. 14 includes a flowchart 1400 illustrating an example of a process of controlling a fluid delivery system for fluid delivery according to certain embodiments. Operations in flowchart 1400 may be performed using, for example, a controller or a processor of delivery device 102, insulin delivery subsystem 230, fluid delivery device 300, fluid delivery device 400 (without using sensor assembly 510), infusion device 602, and the like. In the illustrated example, the fluid to be delivered by the fluid delivery device may be insulin, which may be delivered as basal insulin, regular bolus insulin, square bolus insulin, or insulin dynamically determined in a closed loop based on, for example, measured glucose level data and other data associated with a user of the fluid delivery device.

In the illustrated example, flowchart 1400 may include, at block 1410, determining a remaining amount of fluid (e.g., insulin) to be delivered to a user in a fluid delivery request (or a delivery event). As described above, the fluid delivery request may include, for example, a basal delivery request, a regular bolus delivery request, a square bolus delivery request, or a closed-loop delivery request. If a portion of the requested delivery amount has been delivered already, the delivered amount may be subtracted from the requested delivery amount.

Operations in block 1420 may include, determining the stroke size (e.g., the number of steps in a stroke) for the next stroke based on the remain amount of fluid to be delivered. The stroke size for the next strobe may be determined based additionally on the delivery amount of a minimum stroke size and the delivery amount of the maximum stroke size. The delivery techniques may utilize the variable stroke sizes described above and a comparison scheme to determine and schedule the next stroke. In one example, the remaining amount of fluid to be delivered may be compared against the delivery amount of the minimum stroke size. If the remaining amount is smaller than the delivery amount of the minimum stroke size, the remaining amount may be added to the accumulated under-delivery amount for future delivery, since a delivery less than the delivery amount of the minimum stroke size may not be performed as a standalone delivery. The minimum stroke size is used in order to perform more accurate occlusion detection, because a minimum number of motor steps may be needed in each stroke to make an accurate delivery rate measurement for each stroke. If the remaining amount is larger than the delivery amount of the minimum stroke size but smaller than the delivery amount of the maximum stroke size, the remaining amount may be delivered in one stroke with a delivery amount equal to or smaller than but close to the remaining amount, and the stroke may be immediately delivered. If the remaining amount is equal to or larger than the delivery amount of the maximum stroke size, a maximum stroke may be immediately delivered, and the delivered volume may be subtracted from the remaining amount. The maximum stroke size limitation is imposed in order to more quickly detect the occlusion before the undelivered amount due to the occlusion is large, and also to minimize the risk of patient discomfort due to a large amount of insulin collecting beneath the skin. The remaining portion of the requested delivery amount may then be scheduled and delivered using the same comparison process.

Based on the stroke size determined at block 1420, the motor may be controlled to deliver the fluid at block 1430. As described above, the motor may be controlled using the motor drive voltage and duty cycle learned through the manufacturing calibration and reservoir friction compensation to rotate in a number of consecutive steps in each stroke. The amount of insulin delivered in each step may be approximately a constant value, but the rotation speed and thus the rotation time of the motor in each step may vary due to, for example, variation of the load of motor (e.g., caused by the back pressure from the fluid in the reservoir).

In some examples, during the motor rotation, a motor position sensor (e.g., a Hall effect sensor) may be checked at block 1440 to determine whether the output of the motor position sensor is expected and whether the output of the motor position sensor changes to indicate a rotation of the motor. If the output value of the motor position sensor is not an expect value and/or the output value does not change after the motor is driven, a sensor error or a motor stall may be detected at block 1450. Upon detection of the sensor error or motor stall, an alarm or notification may be provided to a user, and the flowchart 1400 may be terminated.

If motor movement (rotation) is correctly detected (e.g., the output values of the motor position sensor are expected values), a current motor speed or a current delivery rate of the current stroke may be measured at block 1460 based on, for example, a step interval between two steps of a stroke (e.g., the time interval for the motor to change from one position to the next position in a motor step) and the delivery amount in each step as describe above. The step interval may not be measured at the start of each stroke as the motor may not be at a relatively steady speed at the start of each stroke. The step interval may be measured in one or more steps when the motor may be at a relatively steady speed after a few steps from the start of the motor in the current stroke. The step interval may be measured, for example, using a timer to count the time for the output of the motor position sensor to change from one value to the next value. The current delivery rate may be determined by dividing the amount of fluid delivery during the one or more steps by the time intervals of the one or more steps, and may indicate the amount of fluid delivery per unit time (e.g., insulin unit per minute, or milliliter per minute). In one example, the current delivery rate may be measured in two or more steps (e.g., the fourth step and the fifth step) based on the sum of the time for the motor to rotate in each step of the two or more steps (e.g., the time for the motor to move from the position of the third step to the position of the fourth step, and the time for the motor to move from the position of the fourth step to the position of the fifth step), and may be the average of the delivery rate in the two or more steps of the same stroke. In some examples, if the step interval is greater than a threshold time or the motor does not change position after a threshold time, the motor may be stopped or powered off and an error message or alert may be generated.

At block 1470, the current delivery rate measured for the current stroke may be used, in combination with the delivery rates measured in previous strokes, to determine if an occlusion has occurred. More details of the occlusion detection are described below with respect to, for example, FIG. 19.

At block 1480, the total drive steps and the delivery amount in the stroke may be determined and used to determine the remaining amount to be delivered for the current delivery request at block 1410. Operations in flowchart 1400 may be performed iteratively until the remaining portion of the requested delivery amount is less than the delivery amount of the minimum stroke size.

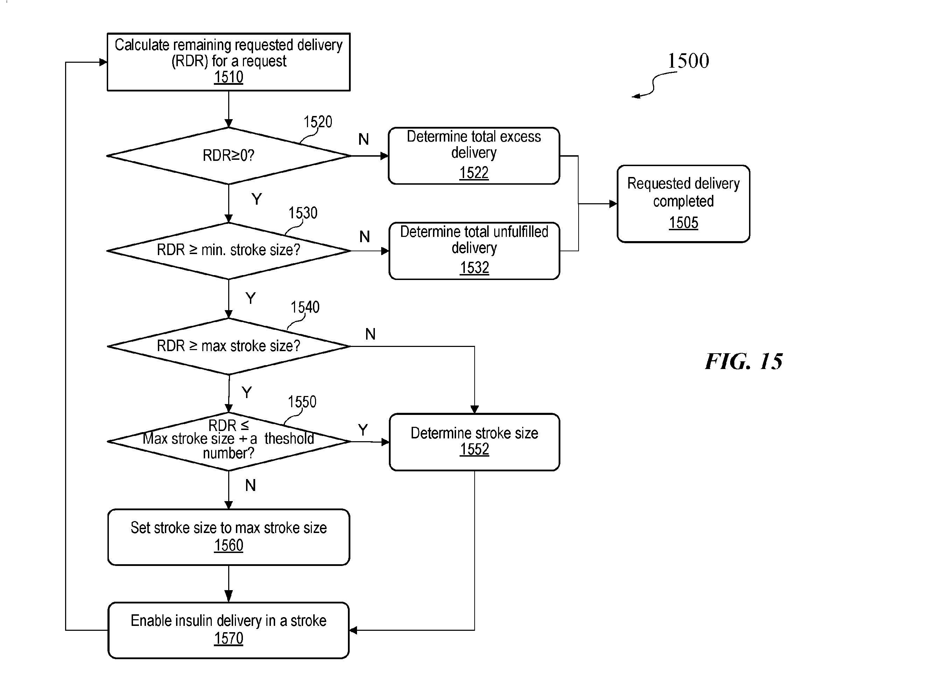

FIG. 15 includes a flowchart 1500 illustrating an example of a process of determining the stroke size for the next stroke based on the remaining amount of fluid to be delivered according to certain embodiments. Flowchart 1500 may be an example of an implementation of block 1420 of flowchart 1400. Operations in flowchart 1500 may be performed using, for example, a controller or a processor of delivery device 102, insulin delivery subsystem 230, fluid delivery device 300, fluid delivery device 400 (without using sensor assembly 510), infusion device 602, and the like.

Operation in flowchart 1500 may include, at block 1510, determining the remaining requested delivery (RDR) amount for a delivery request. The delivery request may include, for example, a basal delivery request, a regular bolus delivery request, or a square bolus delivery request. The RDR amount may be determined by subtracting the amount (if any) that has already been delivered for the current request from the requested delivery amount of the current request.

Operations at block 1520 may include determining whether the RDR amount is a negative value, which may indicate an excess delivery. If the RDR amount is a negative value, the total excess delivery amount may be determined at block 1522, and the current delivery request may be indicated as completed at block 1505. If the RDR amount is not a negative value, the RDR amount may be compared with the delivery amount of the minimum stroke size at block 1530. If the RDR amount is lower than the delivery amount of the minimum stroke size, the total unfulfilled delivery amount for the current request may be determined at block 1532, and the current delivery request may be indicated as completed at block 1505.

When the RDR amount is equal to or greater than the delivery amount of the minimum stroke size, the RDR amount may be compared with the delivery amount of the maximum stroke size at block 1540. If the RDR amount is lower than the delivery amount of the maximum stroke size, a stroke size in the range of the minimum stroke size and the maximum stroke size may be determined at block 1522. If the RDR amount is more than the delivery amount of the maximum stroke size, the RDR amount may be compared with a value that is slightly higher (e.g., by a threshold value less than 5 steps, such as 1 or 2 steps) than the delivery amount of the maximum stroke size at block 1550, to determine if the delivery request should be completed in a single stroke or should be split into two or more strokes. If the RDR amount is slightly higher than the delivery amount of the maximum stroke size, it may be more efficient to complete the request in a single stroke and use a stroke that has a larger size than the maximum stroke size. If the RDR amount is much higher than the delivery amount of the maximum stroke size, the remaining undelivered amount may need to be delivered in two or more strokes, and the stroke size may be set to the maximum stroke size at block 1560.

The stroke size determined at block 1552 or 1560 may be used to control the motor to deliver the fluid in one stroke at block 1570. After the delivery of the stroke, operations in flowchart 1500 may be performed again, until it is determined that the current request has been completed as block 1505.

As described above, the deliver request may include, for example, a basal delivery request, which may be fulfilled using a basal delivery technique disclosed herein. For example, according to the basal delivery technique disclosed herein, a user-entered basal rate (standard or temporary basal rate) may be converted into an integer number of equally-spaced, equal-volume delivery events to be performed in each hour. A temporary basal may be used to temporarily increase or decrease the basal rate for a specified period of time, and may be used when a user is under a certain condition, such as an exercise or illness condition. In one example, a series of three mathematical checks may be used to calculate the optimum number of hourly delivery events based on the requested basal rate. The basal delivery technique disclosed herein may attempt to maximize the number of hourly delivery events while ensuring that the minimum volume of each delivery event is at or greater than a threshold value (e.g., 0.05 units or more). To increase the delivery accuracy and reduce the overall energy consumption, the basal delivery technique disclosed herein may limit the maximum number of hourly delivery events to, for example, 12 (e.g., one delivery event in every 5 minutes). Temporary basal may be automatically delayed by one-half of the time interval between delivery events to minimize delivery stacking when transitioning between standard and temporary basal patterns.

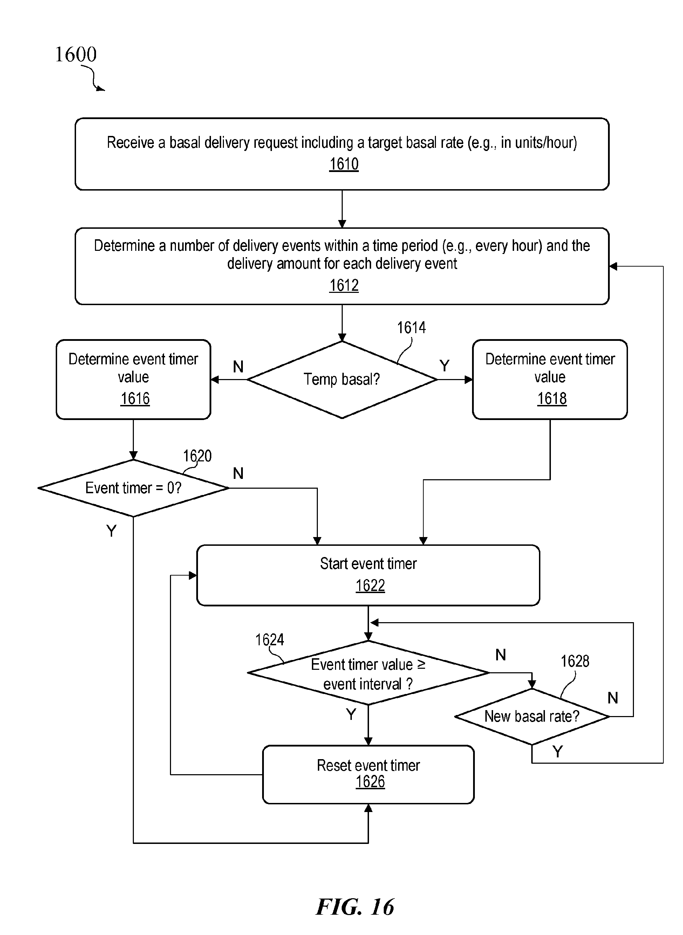

FIG. 16 includes a flowchart 1600 illustrating an example of a process of scheduling and delivering basal insulin using a fluid delivery system according to certain embodiments. Operations in flowchart 1600 may be performed using, for example, a controller or a processor of delivery device 102, insulin delivery subsystem 230, fluid delivery device 300, fluid delivery device 400 (without using sensor assembly 510), infusion device 602, and the like.

Operations in flowchart 1600 may include, at block 1610, receiving a basal delivery request that includes a target basal rate, which may be specified in, for example, units per hour. The target basal rate may be a standard basal rate or a temporary basal rate.

At block 1612, the number of delivery events within a certain time period (e.g., every hour) and the delivery amount for each delivery event may be determined based on the target basal rate. For example, the target basal rate may be evenly distributed among a number of delivery events within the time period, such that the delivery amount in each delivery event may be approximately equal and the time interval between the delivery events may also be approximately equal. The number of delivery events within the time period and the delivery amount in each delivery event may be determined based on the minimum delivery amount for each delivery event and the minimum increment for the delivery amount in each delivery event. In one example, the minimum delivery amount for each delivery event may be set to 0.05 units, whereas the minimum increment for the delivery amount in each delivery event may be set to 0.025 units. If the target basal rate is low, fewer events may be scheduled every hour, so that the delivery amount for each delivery event may be equal to or greater than 0.05 units. If the target basal rate is high, more events may be scheduled every hour. The number of delivery events and the delivery amount for each delivery event may be selected such that the target basal rate can be achieved with minimum or no under delivery and no over delivery, given the limitation of the minimum delivery amount for each delivery event and the minimum increment for the delivery amount in each delivery event. Each delivery event may be fulfilled with one or more strokes using techniques described with respect to, for example, FIGS. 14 and 15.

In one example, when the target basal rate is less than 0.1 units/hour, the number of delivery events per hour may be set to one. When the target basal rate is between 0.1 units/hour and 0.2 units/hour, the number of delivery events per hour may be set to two. When the target basal rate is between 0.2 units/hour and 0.6 units/hour, the number of delivery events per hour may be set to four. When the target basal rate is greater than 0.6 units/hour, the number of delivery events per hour may be set to 12. The delivery amount for each delivery event may then be determined based on the target basal rate and the number of delivery events per hour.

At block 1614, the basal delivery request may be identified as either standard basal or temporary basal. If the basal delivery request is a standard basal delivery request, an event timer value may be determined for the standard basal delivery at block 1616 based on, for example, the time interval between delivery events (referred to as event interval) determined at block 1612 and the current time. If it is determined at block 1620 that the event timer value determined at block 1616 is zero, the event timer may be stopped and reset (to zero) and the delivery amount requested may be set to the delivery amount calculated for each delivery event at block 1626, and then a delivery event and the event timer may be started at block 1622. If the event timer value determined at block 1616 is not zero, the event timer may not be reset and may continue to count until the value of the event counter reaches the calculated time interval between delivery events.

If the basal delivery request is a temporary basal delivery request, an event timer value may be determined for the temporary basal delivery at block 1618 based on the time interval between delivery events determined at block 1612. For example, the event timer value may be set to one half of the time interval between delivery events. The event timer may continue to count at block 1622.

Operations at block 1624 may include determining if the value of the event timer has reached the calculated event interval. If the value of the event timer is equal to or greater than the calculated event interval, the current delivery event is completed, the event timer can be reset at block 1626, and a new delivery event and the event timer can be started at block 1622. If the value of the event timer is smaller than the calculated event interval, the current delivery event may not have been completed yet, and the next delivery event should not be started yet. The controller may determine at block 1628 whether a new basal rate is requested. The new basal rate may be requested by, for example, starting/stopping a temporary basal, or changing the active basal rate. If no new basal rate has been requested, the controller may continue to wait for the value of the event timer to reach the calculated event interval. If a new basal rate has been requested, the number of delivery events per unit time and the delivery amount for each delivery event may be determined for the new basal rate at block 1612, and the new basal rate may be scheduled accordingly based on the process of flowchart 1600.

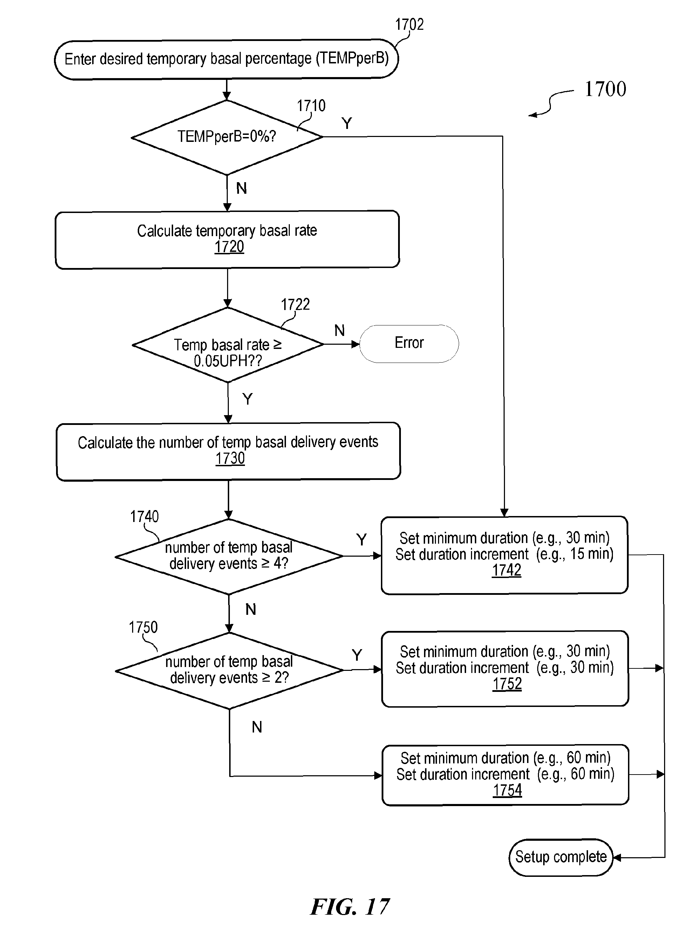

FIG. 17 includes a flowchart 1700 illustrating an example of a process of scheduling and delivering temporary basal insulin using a fluid delivery system according to certain embodiments. Operations in flowchart 1700 may be performed using, for example, a controller or a processor of delivery device 102, insulin delivery subsystem 230, fluid delivery device 300, fluid delivery device 400 (without using sensor assembly 510), infusion device 602, and the like.

At block 1702, the user desired temporary basal percentage may be received. If it is determined at block 1710 that the desired temporary basal percentage is zero, the minimum allowable duration of the requested basal rate and the duration increment above the minimum allowable duration may be set to a first set of values (e.g., 30 minutes and 15 minutes, respectively). If it is determined at block 1710 that the desired temporary basal percentage is greater than zero, the temporary basal rate (e.g., in units per hour) may be determined at block 1720 based on the current basal rate and the user desired temporary basal percentage, such as a product of the current basal rate and the user desired temporary basal percentage. At block 1722, if the temporary basal rate determined at block 1720 is below a minimum temporary basal rate (e.g., about 0.05 units per hour), the user desired temporary basal percentage may be invalid. If the temporary basal rate determined at block 1720 is at or above the minimum temporary basal rate, the number of temporary basal delivery events per unit time (e.g., per hour) may be determined at block 1730 based on the calculated temporary basal rate.

Based on the number of temporary basal delivery events per unit time, the minimum allowable duration of the requested basal rate and the duration increment above the minimum allowable duration may be set for scheduling the temporary basal delivery events. For example, if it is determined at block 1740 that the number of temporary basal delivery events per unit time (e.g., per hour) determined at block 1730 is equal to or greater than a first number (e.g., 4), the minimum allowable duration of the requested basal rate and the duration increment above the minimum allowable duration may be set to the first set of value (e.g., 30 minutes and 15 minutes, respectively). If the number of temporary basal delivery events per unit time (e.g., per hour) is lower than the first number (e.g., 4) but greater than a second number (e.g., 2) as determined at block 1750, the minimum allowable duration of the requested basal rate and the duration increment above the minimum allowable duration may be set to a second set of value (e.g., 30 minutes and 30 minutes, respectively). If the number of temporary basal delivery events per unit time is lower than the second number (e.g., 2), the minimum allowable duration of the requested basal rate and the duration increment above the minimum allowable duration may be set to a third set of value (e.g., 60 minutes and 60 minutes, respectively). Based on these minimum allowable duration of the requested basal rate and the duration increment, the temporary basal may be scheduled and delivered as described above with respect to, for example, FIGS. 13-16.

A square bolus request may be scheduled and delivered based on, for example, a user-entered square bolus amount (e.g., in units of insulin) and the time duration (e.g., in hours). For example, the square bolus amount and the time duration may be converted into an equivalent hourly delivery rate, and then the equivalent hourly delivery rate may be divided into an integer number of equally-spaced, equal-volume delivery events to be performed over the requested time duration. Techniques disclosed herein may attempt to maximize the number of hourly delivery events while ensuring that the minimum delivery amount in each delivery event is at or above a minimum delivery amount (e.g., 0.05 units or more). To increase the delivery accuracy and reduce overall energy consumption, the techniques disclosed herein may limit the maximum number of hourly delivery events to, for example, 12 (or one delivery event every 5 minutes). The minimum delivery amount per delivery event may depend on the capability of the fluid delivery device. For example, for one type of fluid delivery device, the minimum allowable square bolus amount in each delivery event may be about 0.05 units, a minimum increment of the square bolus amount in each delivery event may be about 0.025 units, the minimum square bolus duration may be about 30 minutes, and the minimum duration increment may be about 15 minutes. The maximum square bolus duration may depend on the desired bolus amount and the minimum allowable square bolus amount in each delivery event (e.g., 0.05 units).

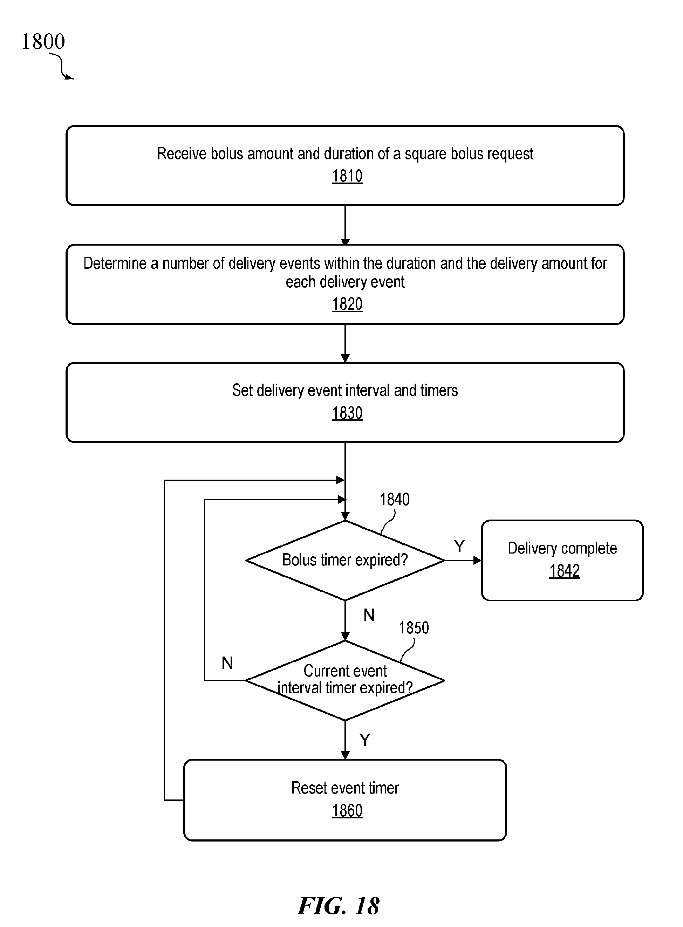

**FIG. 18** includes a flowchart 1800 illustrating an example of a process of scheduling and delivering square bolus insulin using a fluid delivery system according to certain embodiments. Operations in flowchart 1800 may be performed using, for example, a controller or a processor of delivery device 102, insulin delivery subsystem 230, fluid delivery device 300, fluid delivery device 400 (without using sensor assembly 510), infusion device 602, and the like.

Flowchart 1800 may include receiving a square bolus request that includes a bolus amount and duration at block 1810, and determining the number of delivery events within the duration and the delivery amount for each delivery event based on the bolus amount and duration at block 1820. For example, a square bolus delivery rate may be calculated based on the bolus amount and duration (e.g., bolus amount divided by duration). The square bolus delivery rate may be evenly distributed among a number of delivery events within the duration, such that the delivery amount in each delivery event may be approximately equal and the time interval between the delivery events may also be approximately equal. The number of delivery events with the time period and the delivery amount in each delivery event may be determined based on the minimum delivery amount for each delivery event and the minimum increment for the delivery amount in each delivery event. In one example, the minimum delivery amount for each delivery event may be set to 0.05 units, whereas the minimum increment for the delivery amount in each delivery event may be set to 0.025 units. The number of delivery events and the delivery amount for each delivery event may be selected such that the square bolus delivery rate can be achieved with minimum or no under delivery and no over delivery, given the limitation of the minimum delivery amount for each delivery event and the minimum increment for the delivery amount in each delivery event. Each delivery event may be fulfilled with one or more strokes using techniques described with respect to, for example, FIGS. 14 and 15.

In one example, when the square bolus delivery rate is less than 0.1 units/hour, the number of delivery events per hour may be set to one. When the square bolus delivery rate is between 0.1 units/hour and 0.2 units/hour, the number of delivery events per hour may be set to two. When the square bolus delivery rate is between 0.2 units/hour and 0.6 units/hour, the number of delivery events per hour may be set to four. When the square bolus delivery rate is greater than 0.6 units/hour, the number of delivery events per hour may be set to 12.

Operations in block 1830 may include setting the delivery event interval and timers. The delivery event interval may be determined by evenly distributing the number of delivery events in an hour, and the delivery amount for each delivery events may then be determined by evenly distributing the square bolus delivery rate among the number of delivery events per hour. Operations at block 1830 may also include resetting a bolus timer to zero, and setting the initial value of an event interval timer to a value corresponding to the determined delivery event interval. The timers may then be started and the motor may be driven to deliver the bolus. As described above, each event may include one or more strokes determined and delivered using techniques described with respect to, for example, FIGS. 14 and 15.

When a delivery event and the timers are started, the controller may check if the bolus timer has expired at block 1840. If the bolus timer has expired, the bolus time may have lasted for the requested duration and the square bolus delivery may be complete at block 1842, where the motor may be stopped and powered off and the total delivery amount may be determined. If the bolus timer has not expired, the controller may continuously check if the current event interval timer has expired at block 1850. If the current interval event timer has not expired, the event interval time has not been reached yet, and the controller may continue to monitor the bolus timer and the event interval timer. If the current event interval timer has expired, the current delivery event is completed and the next delivery event can be started, and thus the event interval timer may be reset and the total delivery amount may be determined at block 1860. The square bolus delivery process may continue until the bolus timer expires.

FIG. 19A is a diagram 1900 illustrating an example of selecting the number of delivery events for a square bolus based on the total amount of the square bolus according to certain embodiments. In diagram 1900, the horizontal axis corresponds to the total amount of a square bolus, whereas the vertical axis corresponds to the number of delivery events for the square bolus. A curve 1910 in diagram 1900 shows the number of delivery events of a square bolus as a function of the total amount of the square bolus. An inset 1902 in FIG. 19A shows a portion 1912 of curve 1910. Curve 1910 may be used to select the number of delivery events for a square bolus at block 1820 of FIG. 18. It is noted that the example shown in FIG. 19A is for illustration purposes only. In other examples, the minimum volume of each delivery event may be different (e.g., about 0.1 units or larger), and/or the number of delivery events may be determined using another function or curve.

In the example illustrated in FIG. 19A, the number of delivery events for a square bolus may be 1, 2, 3, 4, 5, 6, 7, 8, 12, 24, 48, or 96, depending on the total amount of bolus insulin to be delivered in the square bolus. For example, when the total amount of the square bolus is no more than 0.4 units, the number of delivery events may be equal to the floor function of the total amount divided by the minimum volume of each delivery event (e.g., 0.05 units). In one example, if the total amount of a square bolus is 0.24 units, the number of delivery events may be four. When the total amount of the square bolus is between about 0.4 and about 0.6 units, the number of delivery events may be eight. When the total amount of the square bolus is between about 0.6 and about 1.2 units, the number of delivery events may be 12. When the total amount of the square bolus is between about 1.2 and about 2.4 units, the number of delivery events may be 24. When the total amount of the square bolus is between about 2.4 and about 4.8 units, the number of delivery events may be 48. When the total amount of a square bolus is between about 4.8 and about 25 units, the number of delivery events may be 96.

FIG. 19B includes a diagram 1905 illustrating an example of a delivery pattern for delivering an amount of square bolus within a time window according to some examples. In diagram 1905, the horizontal axis corresponds to the time in a delivery window of a square bolus, whereas the vertical axis corresponds to the delivery volume. Bars 1920 shows both the delivery time and the delivery volume of each delivery event as determined in, for example, blocks 1820 and 1830 of FIG. 18.

In the illustrated example, the total amount of the square bolus may be about 0.2 units, the duration of the square bolus may be about 1 hour, and the number of delivery events for the square bolus may be 4 (e.g., determined based on curve 910). The delivery event interval may be about 15 minutes (1 hour divided by 4), and the delivery volume of each delivery event may be about 0.05 units (0.2 units divided by 4). In the illustrated example, the first delivery event of the square bolus may be delayed by a half of the delivery event interval to ensure that the deliveries are centered as close as possible within the bolus time window. It is noted that some combinations of the bolus amount and duration may result in a delivery pattern that may be slightly off-center within the bolus time window due to integer rounding of the time interval. It is also noted that the example shown in FIG. 19B is for illustration purposes only. In other examples, the delivery time and the delivery volume of each delivery event may be different from the example shown in FIG. 19B. For example, the first delivery event of the square bolus may be delayed by a time that is different from a half of the delivery event interval.

## IV. Occlusion Detection

During the delivery of the fluid using a fluid delivery device, occlusions may occur. An occlusion may be a blockage of insulin flow between the pump and the patient's subcutaneous tissue, thereby preventing the fluid from reaching the patient's subcutaneous tissue. Occlusion may cause a total loss of insulin therapy and thus may present a substantial risk to diabetic patients as they are likely to become hyperglycemic if insulin therapy is not restored in a timely manner. If undetected, an occlusion may lead to potentially harmful under-delivery. To reduce the risk of harms from an occlusion, it is desirable to detect the blockage as early as possible. Therefore, fluid delivery devices such as insulin pumps may need to be able to automatically detect flow blockage caused by occlusion in a timely manner during insulin delivery, such as within, for example, 5 units of missed delivery of insulin, so that a notification can be provided to the patient as early as possible.

As described above, some insulin pumps may use force, strain, or pressure sensors to detect the back-pressure generated from pumping insulin into an occluded infusion set, thereby inferring an occlusion based on the change of the measured back pressure. As also described above, incorporating a strain or pressure sensor into an insulin pump may increase the mechanical complexity as the entire drive system may need to "float" to allow the back pressure generated by the fluid due to the pressure applied to the fluid by the slide through the plunger or the back pressure caused by an occlusion to be detected by the strain sensor. The presence of the strain sensor may reduce the reliability of the insulin pump due to, for example, the sensor fragility and the inability to fully secure the drive system, which may cause the insulin pump to be more susceptible to damage from shock and drop. In addition, strain sensors with sufficient accuracy and resolution for plunger and occlusion detection may be significantly more expensive, and thus may significantly increase the cost of an insulin pump.

Since changes in the motor load may cause changes in the motor velocity when the motor is driven at the same voltage level and duty cycle, the motor load may be indirectly monitored through changes in the motor velocity, rotation time, or instantaneous delivery rate (because the amount of fluid delivered in each motor step may not vary with the motor load). As such, the changes in motor velocity, rotation time, or instantaneous delivery rate can be used for occlusion detection.

According to certain embodiments disclosed herein, occlusion of the fluid delivery device may be detected based on the instantaneous fluid delivery rate (or alternatively the instantaneous motor speed or rotation time) measured using the motor position sensor (e.g., a Hall effect sensor) of the fluid delivery device, in combination with the calibration techniques described above. The calibration techniques may be used to compensate the variations in the motor torque constant and the drive system friction that may otherwise cause the occlusion detection sensitivity to vary widely and result in failure to meet the specified performance metrics. In one example, before the beginning of the therapy, for example, right after the friction between the plunger and a barrel of a reservoir is compensated to adjust the fluid delivery rate from the reservoir (e.g., by adjusting the motor drive voltage and/or duty cycle), motor speeds or fluid delivery rates during a plurality of strokes may be determined based on the positions of the motor measured by the motor position sensor during insulin delivery using the adjusted motor drive voltage and/or duty cycle. The measured motor speeds or fluid delivery rates may be used to generate a coefficient of variation of the delivery rates, which may be used as a baseline coefficient of variation of (unfiltered) delivery rate DBR for occlusion detection.

During fluid delivery, motor speeds or instantaneous current delivery rates (CDRs) may be continuously determined based on the time intervals between the changes of position of the motor (e.g., in the fourth step and/or fifth step of each stroke) measured by the motor position sensor using the adjusted motor drive voltage and/or duty cycle. The measured motor speeds or instantaneous current delivery rates (CDRs) may be filtered by a series of mathematical and statistical filters and saved to a buffer of a certain size to determine a moving coefficient of variation of the filtered delivery rates saved in the buffer. For example, a CDR measured during the fluid delivery may first be passed through a minimum filter, which reduces the stroke-to-stroke variation while allowing a consistent decrease in speed that would occur during an occlusion to become evident. The coefficient of variation of the filtered CDRs stored in the buffer, referred to herein as the buffered delivery rate (BDR), may be calculated in real-time, using a moving standard deviation and moving average of the filtered delivery rates stored in the buffer.

The moving coefficient of variation of the filtered delivery rates may be compared against the baseline coefficient of variation of unfiltered delivery rate DBR determined at, for example, block 1240, for occlusion detection. The results of the comparison may be scored against multiple thresholds, where the different thresholds are used to identify the probability that an actual occlusion may have occurred. When the probability of an occlusion increases, the response time of the occlusion detection may decrease, and fewer delivery strokes may be needed before a notification of occlusion is generated.

In one example, when the moving coefficient of variation exceeds the baseline coefficient of variation and the moving coefficient of variation continues to increase (e.g., the rate of change of the moving CV is greater than a first threshold) for a specified amount of delivery (e.g., a first threshold number of steps), an occlusion may be declared. In some examples, a pre-occlusion threshold may be used to detect a pre-occlusion condition when the moving coefficient of variation exceeds the baseline coefficient of variation and continues to increase at a lower rate (e.g., the rate of change of the moving CV is greater than a second threshold) for a specified amount of delivery (e.g., a second threshold number of steps). In some examples, after a pre-occlusion is triggered, the current rate of change of the moving CV may be compared with a previous rate of change of the moving CV, and an occlusion may be detected if the current rate of change of the moving CV is greater than the previous rate of change of the moving CV by a threshold value and the number of delivery steps during which the fluid delivery device is in the pre-occlusion state is greater than a threshold number. If the number of delivery steps during which the fluid delivery device is in the pre-occlusion state is not greater than the threshold number, an occlusion may not have occurred. In some examples, the pre-occlusion threshold may be automatically adapted to changing motor load conditions during insulin therapy.

For example, if a pre-occlusion state is declared but later undeclared because the number of subsequent steps during which the pre-occlusion condition is not met is greater than a threshold number, the pre-occlusion threshold may be increased.

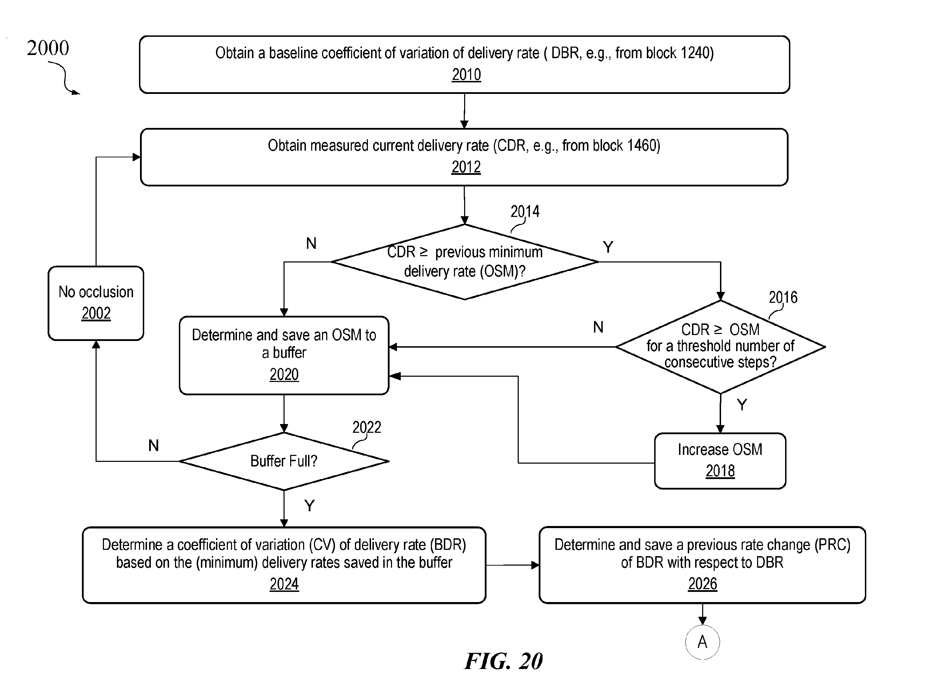

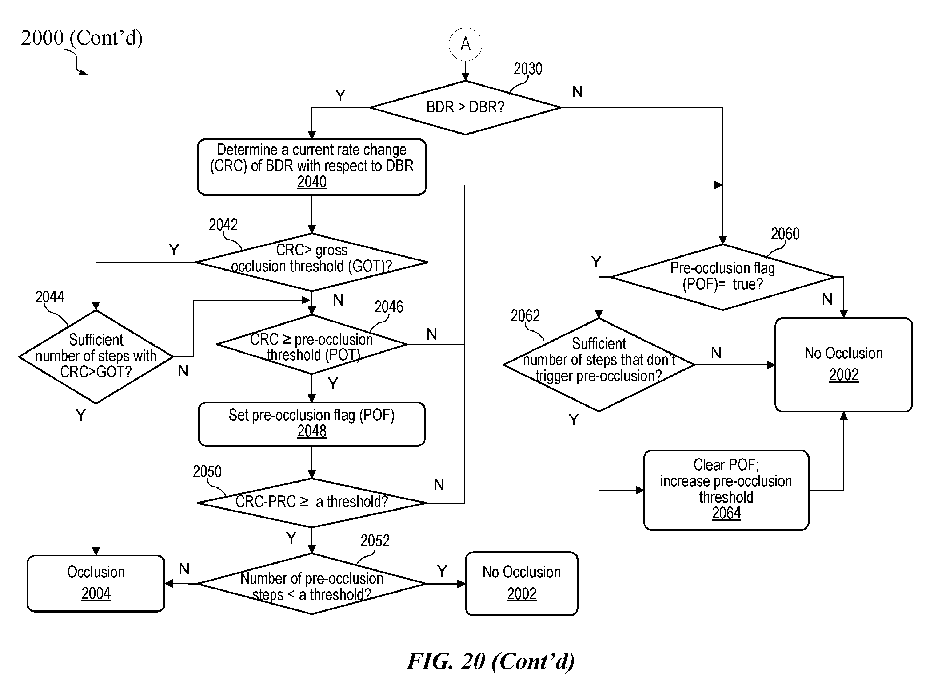

FIG. 20 includes a flowchart 2000 illustrating an example of a process of detecting occlusion during fluid delivery using a fluid delivery system according to certain embodiments. Operations in flowchart 2000 may be performed using, for example, a controller or a processor of delivery device 102, insulin delivery subsystem 230, fluid delivery device 300, fluid delivery device 400 (without using sensor assembly 510), infusion device 602, and the like.

In the illustrated example, at block 2010 of flowchart 2000, a controller may receive or otherwise obtain a baseline coefficient of variation of (unfiltered) delivery rate, such as the baseline coefficient of variation of delivery rate DBR determined at blocks 1230 and 1240 of flowchart 1200. The baseline coefficient of variation of delivery rate DBR may be used as a reference value for occlusion detection. At block 2020, the controller may receive or otherwise obtain a current delivery rate (CDR) measured for a current stroke as described above with respect to, for example, block 1460. The current delivery rate CDR may be an instantaneous delivery rate measured in one or more steps (e.g., two or more steps, such as step 4 and step 5) of a stroke during normal operation of the fluid delivery system.

At blocks 2014-2022, the received CDR may be filtered using a minimum filter and the filtered CDR (e.g., the determined minimum CDR) may be saved in a buffer of a certain size, such as a first-in-first-out (FIFO) buffer. For example, at block 2014, the received CDR may be compared with the minimum value of previously measured CDRs (referred to as occlusion speed minimum or OSM) during a certain number of previous strokes. If there are no previously measured CDRs or OSM, the received CDR may be set as the OSM and saved into the buffer. If it is determined at block 2014 that the received CDR is lower than the current OSM, a new OSM may be determined based on the CDR and saved into the buffer at block 2020. For example, the new OSM may be determined as a weighted sum of the CDR and the existing OSM of the delivery rates saved in the buffer. The weights for the CDR and existing OSM may be selected to increase or smooth the variation of the delivery rate. For example, the weight for the CDR may be between 0 and 1, where a weight value 1.0 may maximize the variation, and a weight value 0 may minimize the variation. If the received CDR is not lower than the current OSM, the controller may determine if the received CDRs have been equal to or greater than the current OSM for a threshold number of delivery steps at block 2016. If the received CDRs have not been equal to or greater than the current OSM for over the threshold number of delivery steps, the current OSM (rather than the received CDR) may be saved to the buffer at block 2020. If the received CDRs have been equal to or greater than the current OSM for over the threshold number of delivery steps, the current OSM may be increased by an increment step at block 2018, and the increased OSM may be compared with the received CDR to determine a new OSM and a delivery rate to save to the buffer at block 2020. For example, if the increased OSM is greater than the received CDR, the received CDR may be set as the new OSM and saved to the buffer at block 2020. If the increased OSM is equal to or lower than the received CDR, the increased OSM may be saved to the buffer and set as the new OSM at block 2020.

In some examples, the OSM determined for the current stroke as described above may be saved to the buffer multiple times if the buffer is not full yet. The number of times that the OSM determined for the current stroke is saved to the buffer may be determined based on the size of the current stroke. For example, the number of times may be equal to the largest integer at or below the ratio between the number of steps in the current stroke and a minimum stroke size of the fluid delivery system. In one specific example, if the current stroke includes 12 steps and the minimum stroke size is 5 steps, the OSM determined for the current stroke may be saved to the buffer twice. After the OSM determined for the current stroke have been saved to the buffer one or more times, the controller may determine whether the buffer is full at block 2022. If the buffer is not full yet, there may not be occlusion detected in the current stroke, and the controller may indicate that no occlusion has occurred at block 2002 and wait for the next CDR measured for the next delivery stroke at block 2012. If the buffer is full, the controller may proceed to block 2024.

At block 2024, a moving coefficient of variation BDR of the filtered delivery rates (e.g., OSMs) currently saved in the buffer may be determined by calculating the mean and the standard deviation of the delivery rates saved in the buffer and calculating the coefficient of variation by dividing the standard deviation by the mean. In some examples, the controller may determine a previous rate of change (PRC) of the moving BDR with respect to the baseline coefficient of variation of delivery rate DBR at block 2026. The PRC may be a percentage of change of the previous moving BDR with respect to the baseline coefficient of variation of delivery rate DBR, and may be saved for occlusion detection.

At block 2030, the controller may determine whether the coefficient of variation BDR is greater than the baseline coefficient of variation of delivery rate DBR. If the coefficient of variation BDR is greater than the baseline coefficient of variation of delivery rate DBR, a current rate (e.g., percentage) of change (CRC) of the coefficient of variation BDR with respect to the baseline coefficient of variation of delivery rate DBR may be determined at block 2040. The CRC may be compared with a gross occlusion threshold GOT at block 2042. If the current rate of change CRC is greater than the gross occlusion threshold GOT, the total number of motor steps with the CRC greater than the GOT may be determined and compared with a threshold value at block 2044. If the total number of motor steps with the CRC greater than the GOT is greater than the threshold value, an occlusion may be detected and declared at block 2004. If the CRC is greater than the GOT, but the total number of motor steps with the CRC greater than the GOT is not greater than the threshold value yet, the CRC may be compared with a pre-occlusion threshold POT at block 2046. The pre-occlusion threshold POT may be lower than the gross occlusion threshold GOT. If the current rate of change CRC is not greater than the gross occlusion threshold GOT, the CRC may also be compared with the pre-occlusion threshold (POT) at block 2046.

If it is determined at block 2046 that the CRC is equal to or greater than the POT, a pre-occlusion flag (POF) may be set at block 2048. At block 2050, the controller may determine if the difference between the CRC and the PRC is greater than a threshold value, where the threshold value may be determined based on, for example, the stroke size of the current stroke. In one example, the threshold value may be equal to the product of (1) the delivery volume per motor step, (2) the number of steps in the stroke, and (3) the minimum change between the CRC and the PRC to maintain the pre-occlusion state. If the difference between the CRC and PRC is greater than the threshold value, the controller may determine, at block 2052, whether the total number of delivery steps since the pre-occlusion flag is set is greater than a threshold. If the total number of delivery steps since the pre-occlusion flag is set is greater than the threshold, an occlusion may be detected and declared at block 2004. If the total number of delivery steps since the pre-occlusion flag is set is not greater than the threshold yet, an occlusion may have not been detected in the current stroke.

The process may proceed to block 2060 if it is determined at block 2030 that the coefficient of variation BDR is not greater than the baseline coefficient of variation DBR, if the CRC is lower than the POT at block 2046, or if the difference between the CRC and PRC is not greater than the threshold value at block 2050. At block 2060, the controller may determine if the pre-occlusion flag (POF) is set. If it is determined at block 2060 that the POF is not set, no occlusion may be detected in the current stroke. If the POF has been set and it is determined at block 2062 that there may have been sufficient number of delivery steps during which no pre-occlusion has been triggered, no occlusion may be detected in the current stroke, the POF may be cleared, and the pre-occlusion threshold (POT) may be increased at block 2064, since the current POT may be low and may cause false triggering of the pre-occlusion condition. If it is determined at block 2062 that there may not have been sufficient number of delivery steps during which no pre-occlusion has been triggered, the pre-occlusion flag may not be unclear and no occlusion may have been detected in the current stroke.

Operations at blocks 2012-2064 may be performed at each delivery stroke based on the current delivery rate measured in one or more steps of each delivery stroke to determine if an occlusion has occurred to the fluid delivery system in real time. The occlusion detection techniques disclosed herein do not use an additional strain sensor or another type of sensor, and thus may significantly reduce the design and control complexity and cost of the fluid delivery system (e.g., insulin pump) and increase the reliability of the fluid delivery system. The occlusion detection techniques disclosed herein may increase the occlusion detection sensitivity to enable faster and more accurate occlusion detection, and may also provide increased robustness against false occlusions by automatically adapting to changing motor load conditions during insulin therapy. The improved occlusion detection performance may improve the user experience and clinical outcomes.

In some circumstances, during operations of a fluid delivery system, the ambient temperature and thus the operating temperature of the fluid delivery system may change, which may change the viscosity of the lubricant of the fluid delivery system and thus the drive speed and delivery rate of the fluid delivery system. The changes in the delivery rate caused by temperature changes (e.g., temperature drop) may increase the coefficient of variation BDR of the filtered delivery rates, and thus may cause false occlusion detections if the same baseline coefficient of variation of delivery rate DBR is used across the operating temperature range of the fluid delivery system.

According to certain embodiments, the occlusion detection techniques disclosed herein may use the operating temperature of the fluid delivery system as an input to control the detection sensitivity (e.g., the threshold for comparing with the coefficient of variation BDR of the filtered delivery rates), thereby avoiding false occlusion detections caused by the impact of temperature drop on the delivery rate. In one example, a buffer (e.g., a FIFO) may be used to store a number of temperature values of the fluid delivery system measured for a number of delivery strokes, where a temperature measurement may be saved to the buffer for each delivery stroke. The maximum temperature value of the temperature values in the buffer may be compared with the current temperature value obtained in the most recent temperature measurement. A temperature flag may be set if the current temperature value is lower than the maximum temperature value by a value greater than a threshold (e.g., 5 °C). The temperature flag may remain set for a certain duration. During the duration, if the fluid delivery system is not in a pre-occlusion state already (e.g., the pre-occlusion flag is not set) and the coefficient of variation BDR of the filtered delivery rates reaches the baseline coefficient of variation of delivery rate DBR, the detection sensitivity may be automatically decreased (e.g., by multiplying the baseline coefficient of variation of delivery rate DBR by a desensitization factor greater than one and using the product as a new occlusion detection threshold), such that false occlusion detections may be reduced or avoid. The detection sensitivity may be reset to a normal value (e.g., the desensitization factor may be reset to one) after the coefficient of variation BDR of the filtered delivery rates falls below the baseline coefficient of variation of delivery rate DBR.

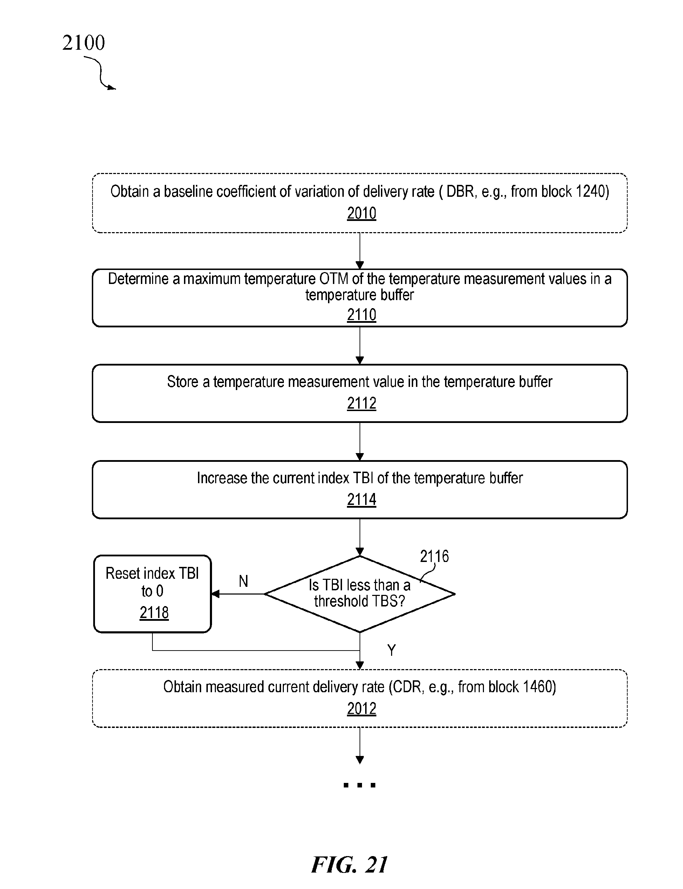

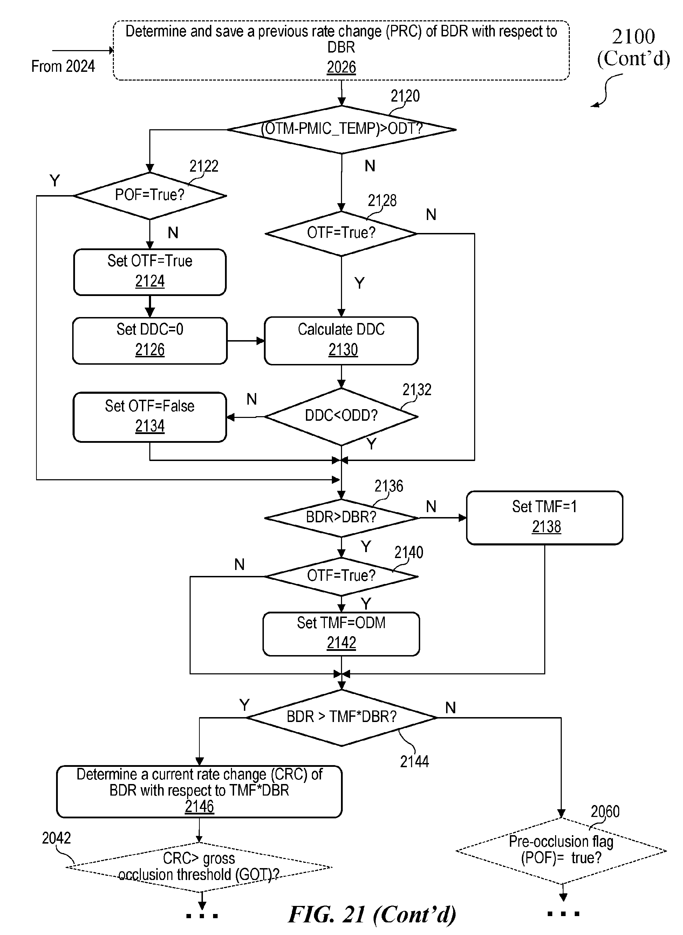

FIG. 21 includes a flowchart 2100 illustrating an example of a process of detecting occlusion of a fluid delivery system while the operating temperature changes according to certain embodiments. Flowchart 2100 may be similar to flowchart 2000, and may include some modifications to flowchart 2000. For example, flowchart 2100 may include additional blocks 2110-2118 between block 2010 and block 2012 of flowchart 2000, and may replace blocks 2030 and 2040 of flowchart 2000 with blocks 2120-2146. Other blocks of flowchart 2100 may be the same as other blocks of flowchart 2000, and thus may not be shown again in FIG. 21. Operations in flowchart 2100 may be performed using, for example, a controller or a processor of delivery device 102, insulin delivery subsystem 230, fluid delivery device 300, fluid delivery device 400 (without using sensor assembly 510), infusion device 602, and the like.

In the illustrated example, flowchart 2100 may include, after block 2010, a block 2110 in which the controller may determine a maximum temperature OTM of temperature measurement values stored in a temperature buffer. The temperature buffer may be a FIFO (e.g., a circular buffer) of a certain size to store a certain number of temperature values of the fluid delivery system measured for a certain number of preceding delivery strokes. As described above, a measured temperature value of the fluid delivery system may be saved to the temperature buffer for each delivery stroke. In some examples, the temperature of the fluid delivery system may be measured at a certain interval (e.g., every 5 minutes). If multiple delivery strokes occur within a temperature measurement interval (e.g., a 5-minute period), for example, for delivering a bolus, the same temperature measurement may be saved to the buffer multiple times for the multiple delivery strokes.

At block 2112, the controller may store a current temperature measurement value PMIC_TEMP (e.g., measured within the temperature measurement interval) for the current stroke in the temperature buffer. The current index TBI of the temperature buffer may be increased by one at block 2114. At block 2116, the controller may compare the current index TBI of the temperature buffer with a threshold buffer size TBS (e.g., 5, 10, or larger). If the current index TBI is not less than the threshold buffer size TBS, the current index TBI may be reset to 0 at block 2118. If the current index TBI is less than the threshold buffer size TBS, the controller may proceed to perform operations in block 2012 and the subsequent blocks (blocks 2012-2026) of flowchart 2000 as described above.

The operations in flowchart 2100 may also include, after block 2026, a block 2120 at which the controller may compare the maximum temperature OTM determined at block 2110 with the current temperature measurement value PMIC_TEMP. If the current temperature measurement value PMIC_TEMP is lower than the maximum temperature OTM by a value greater than a threshold value ODT (e.g., 5°C or higher), the controller may proceed with operations at block 2122; otherwise the controller may proceed with operations at block 2128. At block 2122, the controller may check the pre-occlusion flag POF to determine if the fluid delivery system is in a pre-occlusion state before the temperature decrease. If the pre-occlusion flag POF is True (the fluid delivery system is already in a pre-occlusion state), an occlusion temperature flag OTF may not be set and the controller may proceed with operations at block 2136, such that the occlusion detection desensitization for mitigating the impact of temperature decrease may not be performed and a potential true occlusion may not be undetected due to the desensitization. If the pre-occlusion flag POF is False (the fluid delivery system is not in a pre-occlusion state), the occlusion temperature flag OTF may be set to True at block 2124 and a desensitization delay counter DDC may be started with an initial value set to zero at block 2126. The controller may then proceed with operations at block 2130. At block 2128, if the occlusion temperature flag OTF is not set to True, the controller may proceed with operations at block 2136; otherwise the controller may proceed with operations at block 2130, where the value of the desensitization delay counter DDC may be calculated (e.g., by increasing the desensitization delay counter DDC by a calculated stroke size of the current stroke). The calculated value of the desensitization delay counter DDC may be compared with an occlusion desensitization delay ODD at block 2132. If the value of the desensitization delay counter DDC is lower than the occlusion desensitization delay ODD, the controller may proceed with operations at block 2136; otherwise, the occlusion desensitization delay ODD may have been reached, and the controller may proceed with operations at block 2134, where the occlusion temperature flag OTF may be reset to False to disable occlusion detection desensitization.

At block 2136, the controller may compare the coefficient of variation BDR of the filtered delivery rates with the baseline coefficient of variation of delivery rate DBR. If the coefficient of variation BDR is not greater than the baseline coefficient of variation of delivery rate DBR, a temperature multiplication factor TMF may be set to one at block 2138 so that the occlusion detection sensitivity may not be decreased. If the coefficient of variation BDR is greater than the baseline coefficient of variation of delivery rate DBR, the controller may proceed with operations at block 2140, where the controller may check if the occlusion temperature flag OTF is True. If the occlusion temperature flag OTF is not True, the temperature multiplication factor TMF may not be changed (e.g., having a value of one). If the occlusion temperature flag OTF is True, the temperature multiplication factor TMF may be set to an occlusion desensitization multiplier ODM (e.g., greater than one) at block 2142 to desensitize the occlusion detection, thereby avoiding a false occlusion detection caused by a decrease in the operating temperature.

At block 2144, the controller may determine whether the coefficient of variation BDR is greater than the product of the baseline coefficient of variation of delivery rate DBR and the temperature multiplication factor TMF. If the coefficient of variation BDR is greater than the product of the baseline coefficient of variation of delivery rate DBR and the temperature multiplication factor TMF, a current rate (e.g., percentage) of change (CRC) of the coefficient of variation BDR with respect to the product of the baseline coefficient of variation of delivery rate DBR and the temperature multiplication factor TMF may be determined at block 2046 (e.g.,

$$CRC = \frac{BDR - TMF \times DBR}{TMF \times DBR} \times 100$$

). The controller may then compare the CRC with a gross occlusion threshold GOT at block 2042 and perform operations at other blocks as described above with respect to flowchart 2000. If the coefficient of variation BDR is not greater than the product of the baseline coefficient of variation of delivery rate DBR and the temperature multiplication factor TMF, the controller may proceed with operations at block 2060 and other blocks as described above with respect to flowchart 2000.

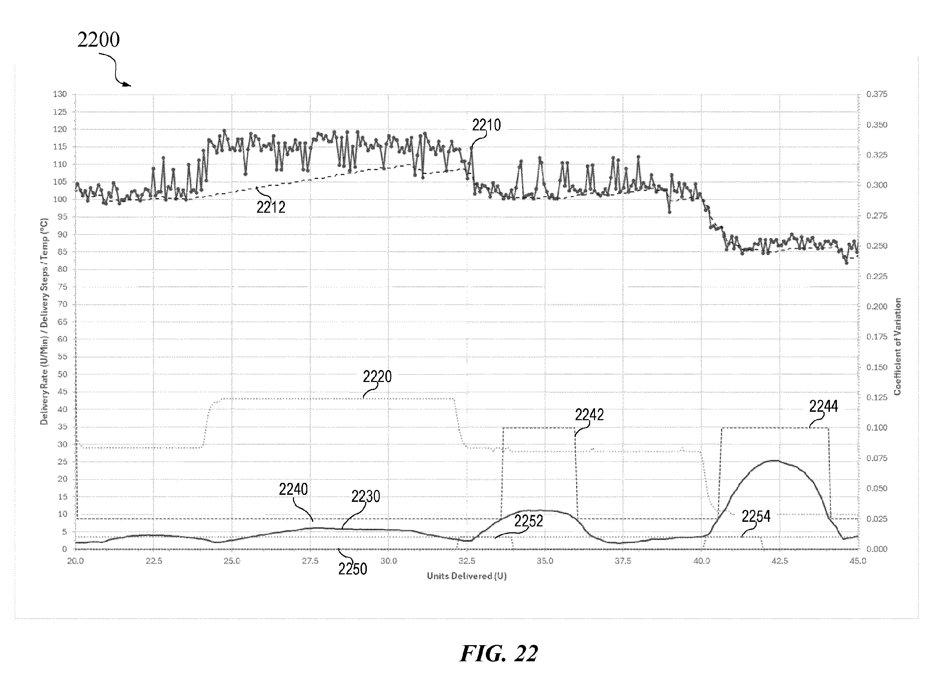

FIG. 22 includes a graph 2200 illustrating examples of parameters used in an example of a process of detecting occlusion of a fluid delivery system while the operating temperature changes according to certain embodiments. In FIG. 22, the horizontal axis corresponds to the cumulative amount of fluid (e.g., insulin) delivered by the fluid delivery system, the primary vertical axis (on the left side) indicates the delivery rate (in units/min), the number of delivery steps, or the operating temperature of the fluid delivery system, while the secondary vertical axis (on the right side) indicates the coefficient of variation of the delivery rate.

A curve 2210 in graph 2200 indicates the current delivery rate CDR of the fluid delivery system over time. A curve 2212 indicates the minimum value OSM of the current delivery rate CDR within a certain number of preceding strokes (e.g., the filtered minimum delivery rate) over time. A curve 2220 indicates the operating temperature of the fluid delivery system over time. A curve 2230 indicates the coefficient of variation BDR determined by filtering the current delivery rate CDR shown by curve 2210 using techniques disclosed herein. A curve 2240 indicates the product of the baseline coefficient of variation of delivery rate DBR and the temperature multiplication factor TMF described above, and a curve 2250 indicates the occlusion temperature flag OTF, as described above with respect to FIG. 21.

In the illustrated example, the operating temperature of the fluid delivery system may increase from about 29°C to about 43°C, which may cause the current delivery rate CDR to increase, but the coefficient of variation BDR determined by filtering the current delivery rate CDR may not change significantly due to the filtering. After the fluid delivery system delivered about 32 units of fluid (e.g., insulin), the operating temperature of the fluid delivery system may decrease from about 43°C to about 28°C, which may cause the delivery rate of the fluid delivery system to decrease, cause the coefficient of variation BDR of the filtered delivery rates to increase, and cause the controller of the fluid delivery system to set the occlusion temperature flag OTF to True as shown by a section 2252 of curve 2250. When the coefficient of variation BDR reaches the baseline coefficient of variation of delivery rate DBR while the occlusion temperature flag OTF is still True, the temperature multiplication factor TMF may be set to a value that is greater than one (e.g., occlusion desensitization multiplier ODM), such that the product of the baseline coefficient of variation of delivery rate DBR and the temperature multiplication factor TMF may be increased as shown by a section 2242 of curve 2240 to desensitize the occlusion detection. As such, the coefficient of variation BDR may be lower than a desensitized threshold that is equal to the product of the baseline coefficient of variation of delivery rate DBR and the temperature multiplication factor TMF (e.g., the occlusion desensitization multiplier ODM), and thus a pre-occlusion flag may not be set and an occlusion may not be detected, even though the coefficient of variation BDR may be greater than the baseline coefficient of variation of delivery rate DBR. If the coefficient of variation BDR is higher than the desensitized threshold (e.g., the product of the baseline coefficient of variation of delivery rate DBR and the occlusion desensitization multiplier ODM), a pre-occlusion flag may be set and/or an occlusion may be detected. When the coefficient of variation BDR drops to the value of the baseline coefficient of variation of delivery rate DBR, the temperature multiplication factor TMF may be reset to one to resensitize the occlusion detection.

After the fluid delivery system delivered about 40 units of fluid (e.g., insulin), the operating temperature of the fluid delivery system may decrease from about 28°C to about 10°C, which may cause the delivery rate of the fluid delivery system to further decrease, cause the coefficient of variation BDR of the filtered delivery rates to increase, and cause the controller of the fluid delivery system to set the occlusion temperature flag OTF to True again as shown by a section 2254 of curve 2250. When the coefficient of variation BDR reaches the baseline coefficient of variation of delivery rate DBR while the occlusion temperature flag OTF is still True, the temperature multiplication factor TMF may be set to a value that is greater than one (e.g., occlusion desensitization multiplier ODM), such that the product of the baseline coefficient of variation of delivery rate DBR and the temperature multiplication factor TMF may be increased as shown by a section 2244 of curve 2240, to desensitize the occlusion detection. As such, the coefficient of variation BDR may be lower than a desensitized threshold that is equal to the product of the baseline coefficient of variation of delivery rate DBR and the temperature multiplication factor TMF (e.g., the occlusion desensitization multiplier ODM), and thus a pre-occlusion flag may not be set and an occlusion may not be detected, even though the coefficient of variation BDR may be greater than the baseline coefficient of variation of delivery rate DBR. If the coefficient of variation BDR is higher than the desensitized threshold (e.g., the product of the baseline coefficient of variation of delivery rate DBR and the occlusion desensitization multiplier ODM), a pre-occlusion flag may be set and/or an occlusion may be detected. When the coefficient of variation BDR drops to the value of the baseline coefficient of variation of delivery rate DBR, the temperature multiplication factor TMF may be reset to one to resensitize the occlusion detection.

The threshold value ODT described above may be set to, for example, 5°C or another suitable value, which may be determined based on, for example, experimental results. In some examples, the threshold value ODT may be a function of, for example, the temperature before the temperature decrease. The temperature multiplication factor TMF or the occlusion desensitization multiplier ODM described above may be determined based on, for example, experimental results, and can be a constant value or a variable that may be a function of the temperature before the temperature decrease, the magnitude of the temperature decrease, and the like. Similarly, the occlusion desensitization delay ODD may be determined based on, for example, experimental results, and can be a constant value or a variable that may be a function of the temperature before the temperature decrease, the magnitude of the temperature decrease, and the like.

Operations in flowcharts 900-1800, 2000, and 2100 may be performed using, for example, a controller or a processor of delivery device 102, insulin delivery subsystem 230, fluid delivery system 300, fluid delivery system 400 (without using sensor assembly 510), infusion device 602, and the like. Although each of flowcharts 900-1800, 2000, and 2100 may describe the operations as a sequential process, some of the operations may be performed in parallel or concurrently. In addition, the order of the operations may be rearranged. The process may have additional steps not included in the figure. Some operations may be optional or may be omitted. Some operations may be merged with operations in another block, or may instead be performed in another block.

Embodiments of the methods disclosed herein may be implemented by hardware, software, firmware, middleware, microcode, hardware description languages, or any combination thereof. For example, the operations in the flowcharts may be performed by a server, a personal computer, a mobile device (e.g., a smartphone or a tablet), a medical device (e.g., a glucose sensor or an insulin delivery device), or a combination thereof. When implemented in software, firmware, middleware, or microcode, the program code or code segments to perform the associated tasks may be stored in one or more computer-readable media such as a storage medium, and may be executed by one or more processors to perform the associated tasks. The computer-readable media may include transitory or non-transitory computer-readable media, such as RAM, ROM, EEPROM, flash memory, solid-state drive, hard drive, CD, DVD, or any other medium that can be used to store desired program code in the form of instructions or data structures and that can be accessed by a computer. The one or more processors may include general purpose microprocessors, application specific integrated circuits (ASICs), graphic processing units (GPUs), network processing units (NPUs), digital signal processors (DSPs), field programmable logic arrays (FPGAs), and the like.

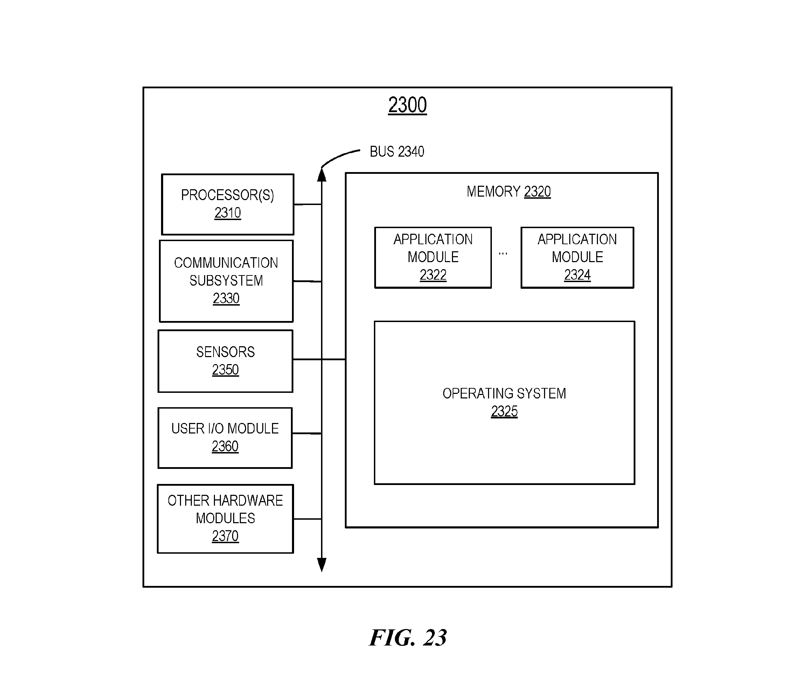

FIG. 23 is a block diagram of an example of an electronic device 2300 that may implement some of the examples disclosed herein. For example, electronic device 2300 may be used to implement delivery device 102, monitoring device 104, computing device 106, and remote/cloud computing system 108, glucose sensor subsystem 210, controller 220, insulin delivery subsystem 230, glucose delivery subsystem 240, glucagon delivery subsystem 250, fluid delivery device 300, fluid delivery device 400 (without using sensor assembly 510), infusion device 602, or another device that may include a controller or a processor to perform processor-implemented methods, functions, or operations. In the illustrated example, electronic device 2300 may include one or more processor(s) 2310 and memory 2320. Processor(s) 2310 may be configured to execute instructions stored in memory 2320 for performing one or more of the methods described herein and other applications. Processor(s) 2310 may include, for example, one or more central processing units, microprocessors, microcontrollers, special-purpose processors (e.g., digital signal processors), ASICs, DSPs, FPGAs, or other processors suitable for implementation within a portable electronic device. Processor(s) 2310 may be communicatively coupled with a plurality of components within electronic device 2300. To realize this communicative coupling, processor(s) 2310 may communicate with the other illustrated components across a bus 2340. Bus 2340 may be any subsystem adapted to transfer data within electronic device 2300. Bus 2340 may include a plurality of computer buses and additional circuitry to transfer data.

Memory 2320 may include one or more transitory and/or non-transitory storage devices, such as, for example, a static random access memory (SRAM), a dynamic random access memory (DRAM), a read-access memory (RAM), a read-only memory (ROM), a programmable read-only memory (PROM), an erasable programmable read-only memory (EPROM), a FLASH-EPROM, a secure digital (SD) card, and any other memory chip or cartridge. Such storage devices may be configured to implement any appropriate data stores, including without limitation, various file systems, data structures, computer-readable instructions, program modules, and the like. In some embodiments, memory 2320 may be distributed into different hardware modules.

Memory 2320 may include an operating system 2325 loaded therein. Operating system 2325 may be operable to initiate the execution of the instructions provided by application modules 2322-2324 and/or manage other hardware modules 2370, as well as interface with a communication subsystem 2330 which may include one or more wired and/or wireless transceivers. Operating system 2325 may be adapted to perform other operations across the components of electronic device 2300 including threading, virtualization, resource management, data storage control, and other similar functionality. In some embodiments, memory 2320 may store a plurality of application modules 2322 through 2324, which may include any number of applications. Examples of the applications may include an insulin calculator, a blood glucose level monitor or predictor, a glucose level management application, and the like. Application modules 2322-2324 may include particular instructions to be executed by processor(s) 2310. In some embodiments, certain applications or parts of application modules 2322-2324 may be executable by other hardware modules 2370.

Communication subsystem 2330 may include, for example, an infrared communication device, a wireless communication device and/or chipset (such as a Bluetooth® device, an IEEE 802.11 device, a Wi-Fi device, a WiMax device, cellular communication devices, etc.), and/or similar communication interfaces. One or more antennas (not shown) may be used for wireless communication as part of communication subsystem 2330 or as a separate component coupled to any portion of electronic device 2300, such as a wireless charging receiver or a near-field communication receiver. In some embodiments, communication subsystem 2330 may include circuits for wired communication technologies, such as Ethernet, coaxial communications, universal serial bus (USB), and the like. Communications subsystem 2330 may permit data to be exchanged with a network, other computer systems, and/or any other devices. For example, communications subsystem 2330 may be used to receive therapy determinations for therapeutic fluid (e.g., insulin) delivery, such as from a cloud computing system via an intermediary computing device (e.g., a controller) communicatively coupled to electronic device 2300, where processor(s) 2310 may, based on the therapy determinations, send commands to an actuator controller to cause the delivery of appropriate amounts of therapeutic fluid (e.g., insulin) to a user. In another example, communications subsystem 2330 may be used to communicate measurement results (e.g., sensor glucose levels) to a computing device (e.g., a smartphone or a personal health monitoring device) and/or to a remote server via the computing device, or receive data (e.g., calibration data, configuration data, etc.) from the computing device or the remote server via the computing device.

Sensor(s) 2350 may include, for example, a camera, an infrared sensor, an accelerometer, a pressure sensor, a temperature sensor, a proximity sensor, a magnetometer, a gyroscope, an inertial sensor (e.g., an inertial measurement unit (IMU)), an ambient light sensor, a position sensor, a depth sensor, a gesture detector, or any other similar module operable to provide sensory output and/or receive sensory input. In one example, sensor(s) 2350 may be used to implement a meal detector.

Input/output user interface 2360 may allow a user to send action requests to electronic device 2300 to perform particular actions, and may provide information (e.g., status of electronic device 2300, measurement results, alerts, etc.) to the user. Input/output user interface 2360 may include one or more input devices, such as, for example, a touchscreen, a touch pad, microphone(s), button(s), dial(s), switch(es), a keyboard, a mouse, a game controller, or any other suitable device for receiving action requests and communicating the received action requests to processor(s) 2310. In some embodiments, input/output user interface 2360 may include one or more output devices, such as a display, a speaker, a light emitting device, a haptic device, and the like, to provide feedback or alarm to the user.

In some embodiments, electronic device 2300 may include a plurality of other hardware modules 2370. Each of other hardware modules 2370 may be a physical module within electronic device 2300. While each of other hardware modules 2370 may be permanently configured as a structure, some of other hardware modules 2370 may be temporarily configured to perform specific functions or temporarily activated. Examples of other hardware modules 2370 may include, for example, an audio output and/or input module (e.g., a microphone or speaker), a near field communication (NFC) module, a rechargeable battery, a battery management system, a wired/wireless battery charging system, an actuator controller, and the like. In some embodiments, one or more functions of other hardware modules 2370 may be implemented in software.

In one example, electronic device 2300 may be part of an insulin delivery device (e.g., a pump) that can deliver fast-acting insulin through a small tube configured for fluidic connection with a cannula inserted subcutaneously. Electronic device 2300 may cause the delivery of two types of dosages-a basal dosage, which can be delivered periodically (e.g., every five minutes) in tiny amounts throughout the day and night, and a bolus dosage to cover an increase in blood glucose from meals and/or to correct high blood glucose levels. The insulin delivery device may include a user interface having button elements that can be manipulated to administer a bolus of insulin, to change therapy settings, to change user preferences, to select display features, and the like.

The insulin delivery device may also include a display device that can be used to present various types of information or data to the user. In accordance with aspects of the present disclosure, a user of the insulin delivery device may use the button elements to input certain event data (e.g., event type, event start time, event details, etc.), and the user inputs can be confirmed using the display device.

In various implementations, the above-described hardware and modules may be implemented on a single device or on multiple devices that can communicate with one another using wired or wireless connections. In alternative configurations, different and/or additional components may be included in electronic device 2300. Similarly, functionality of one or more of the components can be distributed among the components in a manner different from the manner described above.

The methods, systems, and devices discussed above are examples. Various embodiments may omit, substitute, or add various procedures or components as appropriate. For instance, in alternative configurations, the methods described may be performed in an order different from that described, and/or various stages may be added, omitted, and/or combined. Also, features described with respect to certain embodiments may be combined in various other embodiments. Different aspects and elements of the embodiments may be combined in a similar manner. Also, technology evolves and, thus, many of the elements are examples that do not limit the scope of the disclosure to those specific examples.

Specific details are given in the description to provide a thorough understanding of the embodiments. However, embodiments may be practiced without these specific details. For example, well-known circuits, processes, systems, structures, and techniques have been shown without unnecessary detail in order to avoid obscuring the embodiments. This description provides example embodiments only, and is not intended to limit the scope, applicability, or configuration of the invention. Rather, the preceding description of the embodiments will provide those skilled in the art with an enabling description for implementing various embodiments. Various changes may be made in the function and arrangement of elements without departing from the spirit and scope of the present disclosure.

Also, some embodiments were described as processes depicted as flow diagrams or block diagrams. Although each may describe the operations as a sequential process, many of the operations may be performed in parallel or concurrently. In addition, the order of the operations may be rearranged. A process may have additional steps not included in the figure. Furthermore, embodiments of the methods may be implemented by hardware, software, firmware, middleware, microcode, hardware description languages, or any combination thereof. When implemented in software, firmware, middleware, or microcode, the program code or code segments to perform the associated tasks may be stored in a computer-readable medium such as a storage medium. Processors may perform the associated tasks.

It will be apparent to those skilled in the art that substantial variations may be made in accordance with specific requirements. For example, customized or special-purpose hardware might also be used, and/or particular elements might be implemented in hardware, software (including portable software, such as applets, etc.), or both. Further, connection to other computing devices such as network input/output devices may be employed.

The embodiments disclosed herein are examples of the disclosure and may be embodied in various forms. For instance, although certain embodiments herein are described as separate embodiments, each of the embodiments herein may be combined with one or more of the other embodiments herein. Specific structural and functional details disclosed herein are not to be interpreted as limiting, but as a basis for the claims and as a representative basis for teaching one skilled in the art to variously employ the present disclosure in virtually any appropriately detailed structure. Like reference numerals may refer to like elements throughout the description of the figures.

Any of the herein described techniques, operations, methods, programs, algorithms, or codes may be converted to, or expressed in, a programming language or computer program embodied on a computer, processor, or machine-readable medium. The terms "programming language" and "computer program," as used herein, each include any language used to specify instructions to a computer or processor, and include (but is not limited to) the following languages and their derivatives: Assembler, Basic, Batch files, BCPL, C, C+, C++, Delphi, Fortran, Java, JavaScript, machine code, operating system command languages, Pascal, Perl, PL1, Python, scripting languages, Visual Basic, metalanguages which themselves specify programs, and all first, second, third, fourth, fifth, or further generation computer languages. Also included are database and other data schemas, and any other meta-languages. No distinction is made between languages which are interpreted, compiled, or use both compiled and interpreted approaches. No distinction is made between compiled and source versions of a program. Thus, reference to a program, where the programming language could exist in more than one state (such as source, compiled, object, or linked) is a reference to any and all such states. Reference to a program may encompass the actual instructions and/or the intent of those instructions.

It should be understood that the foregoing description is only illustrative of the present disclosure. To the extent consistent, any or all of the aspects detailed herein may be used in conjunction with any or all of the other aspects detailed herein. Various alternatives and modifications can be devised by those skilled in the art without departing from the disclosure. Accordingly, the present disclosure is intended to embrace all such alternatives, modifications, and variances. The embodiments described with reference to the attached drawing figures are presented only to demonstrate certain examples of the disclosure. Other elements, steps, methods, and techniques that are insubstantially different from those described above and/or in the appended claims are also intended to be within the scope of the disclosure. While several embodiments of the disclosure have been depicted in the drawings, it is not intended that the disclosure be limited thereto, as it is intended that the disclosure be as broad in scope as the art will allow and that the specification be read likewise. Therefore, the above description should not be construed as limiting, but merely as exemplifications of particular embodiments. Those skilled in the art will envision other modifications within the scope and spirit of the claims appended hereto. The aspects and features of the present disclosure and may be embodied in various forms. Thus, specific structural and functional details disclosed herein are not to be interpreted as limiting, but as a basis for the claims and as a representative basis for teaching one skilled in the art to variously employ the present disclosure in virtually any appropriately detailed structure.

With reference to the appended figures, components that can include memory can include non-transitory machine-readable media. The term "machine-readable medium," "processor-readable medium," or "computer-readable medium" may refer to any storage medium that participates in providing data that causes a machine to operate in a specific fashion. In embodiments provided hereinabove, various machine-readable media might be involved in providing instructions/code to processing units and/or other device(s) for execution. Additionally or alternatively, the machine-readable media might be used to store and/or carry such instructions/code. In many implementations, a computer-readable medium is a physical and/or tangible storage medium. Such a medium may take many forms, including, but not limited to, non-volatile media, volatile media, and transmission media. Common forms of computer-readable media include, for example, magnetic and/or optical media such as compact disk (CD) or digital versatile disk (DVD), punch cards, paper tape, any other physical medium with patterns of holes, a RAM, a programmable read-only memory (PROM), an erasable programmable read-only memory (EPROM), a FLASH-EPROM, any other memory chip or cartridge, a carrier wave as described hereinafter, or any other medium from which a computer can read instructions and/or code. A computer program product may include code and/or machine-executable instructions that may represent a procedure, a function, a subprogram, a program, a routine, an application (App), a subroutine, a module, a software package, a class, or any combination of instructions, data structures, or program statements.

Those of skill in the art will appreciate that information and signals used to communicate the messages described herein may be represented using any of a variety of different technologies and techniques. For example, data, instructions, commands, information, signals, bits, symbols, and chips that may be referenced throughout the above description may be represented by voltages, currents, electromagnetic waves, magnetic fields or particles, optical fields or particles, or any combination thereof.

Terms, "and" and "or" as used herein, may include a variety of meanings that are also expected to depend at least in part upon the context in which such terms are used. Typically, "or" if used to associate a list, such as A, B, or C, is intended to mean A, B, and C, here used in the inclusive sense, as well as A, B, or C, here used in the exclusive sense. In addition, the term "one or more" as used herein may be used to describe any feature, structure, or characteristic in the singular or may be used to describe some combination of features, structures, or characteristics. However, it should be noted that this is merely an illustrative example and claimed subject matter is not limited to this example. Furthermore, the term "at least one of" if used to associate a list, such as A, B, or C, can be interpreted to mean A, B, C, or any combination of A, B, and/or C, such as AB, AC, BC, AA, ABC, AAB, AABBCCC, etc.

Further, while certain embodiments have been described using a particular combination of hardware and software, it should be recognized that other combinations of hardware and software are also possible. Certain embodiments may be implemented only in hardware, or only in software, or using combinations thereof. In one example, software may be implemented with a computer program product containing computer program code or instructions executable by one or more processors for performing any or all of the steps, operations, or processes described in this disclosure, where the computer program may be stored on a non-transitory computer readable medium. The various processes described herein can be implemented on the same processor or different processors in any combination.

Where devices, systems, components or modules are described as being configured to perform certain operations or functions, such configuration can be accomplished, for example, by designing electronic circuits to perform the operation, by programming programmable electronic circuits (such as microprocessors) to perform the operation such as by executing computer instructions or code, or processors or cores programmed to execute code or instructions stored on a non-transitory memory medium, or any combination thereof. Processes can communicate using a variety of techniques, including, but not limited to, conventional techniques for inter-process communications, and different pairs of processes may use different techniques, or the same pair of processes may use different techniques at different times.

In view of this description, embodiments may include different combinations of features. Implementation examples are described in the following numbered clauses:

Clause 1. A processor-implemented method comprising:

determining a requested delivery rate of a request for fluid delivery using a fluid delivery device; determining, based on a range that the requested delivery rate falls in, a corresponding number of delivery events per hour for the request; determining, based on the requested delivery rate and the number of delivery events, a delivery volume of each delivery event of the number of delivery events and an event interval between adjacent deliver events of the number of delivery events; and causing performance of the number of delivery events according to the delivery volume and the event interval.

Clause 2. The processor-implemented method of clause 1, wherein the request includes a basal insulin delivery request, a temporary basal insulin delivery request, or a square bolus delivery request.

Clause 3. The processor-implemented method of clause 1 or 2, wherein the number of delivery events includes 1, 2, 3, 4, 5, 6, 7, 8, 12, 24, 48, or 96 delivery events.

Clause 4. The processor-implemented method of any of clauses 1-3, wherein each delivery event of the number of delivery events includes one or more strokes.

Clause 5. The processor-implemented method of any of clauses 1-4, wherein causing performance of each delivery event of the number of delivery events includes, in response to determining that the delivery volume of the delivery event is equal to or greater than a delivery volume of a minimum stroke size of the fluid delivery device and is equal to or lower than a delivery volume of a maximum stroke size, causing performance of the delivery event in one stroke.

Clause 6. The processor-implemented method of any of clauses 1-4, wherein causing performance of each delivery event of the number of delivery events includes, in response to determining that the delivery volume of the delivery event is greater than a delivery volume of a maximum stroke size, causing performance of the delivery event in a stroke having the maximum stroke size and one or more additional strokes.

Clause 7. The processor-implemented method of clause 6, wherein causing performance of each delivery event of the number of delivery events further includes: determining a remaining requested delivery amount based on a difference between the delivery volume of the delivery event and the delivery volume of the maximum stroke size; and in response to determining that the remaining requested delivery amount is equal to or greater than a delivery volume of a minimum stroke size of the fluid delivery device and is equal to or lower than the delivery volume of the maximum stroke size, causing delivery of the remaining requested delivery amount in one stroke.

Clause 8. The processor-implemented method of clause 6, wherein causing performance of each delivery event of the number of delivery events further includes: determining a remaining requested delivery amount based on a difference between the delivery volume of the delivery event and the delivery volume of the maximum stroke size; and in response to determining that the remaining requested delivery amount is greater than the delivery volume of the maximum stroke size, causing delivery of the delivery volume of the maximum stroke size in one stroke.

**Clause 9.** The processor-implemented method of any of clauses 1-4, wherein causing performance of each delivery event of the number of delivery events includes, in response to determining that (1) the delivery volume of the delivery event is greater than a delivery volume of a maximum stroke size and (2) a difference between the delivery volume of the delivery event and the delivery volume of the maximum stroke size is less than a threshold value, causing performance of the delivery event in one stroke with a stroke size greater than maximum stroke size.

**Clause 10.** The processor-implemented method of any of clauses 1-4, wherein causing performance of each delivery event of the number of delivery events includes, in response to determining that the delivery volume of the delivery event is lower than a delivery volume of a minimum stroke size of the fluid delivery device, determining a total unfulfilled delivery amount for future delivery.

**Clause 11.** A system comprising: one or more processors; and one or more processor-readable storage media storing instructions which, when executed by the one or more processors, cause performance of operations including: determining a requested delivery rate of a request for fluid delivery; determining, based on a range that the requested delivery rate falls in, a corresponding number of delivery events per hour for the request; determining, based on the requested delivery rate and the number of delivery events, a delivery volume of each delivery event of the number of delivery events and an event interval between adjacent deliver events of the number of delivery events; and causing performance of the number of delivery events according to the delivery volume and the event interval.

**Clause 12.** The system of clause 11, wherein the request includes a basal insulin delivery request, a temporary basal insulin delivery request, or a square bolus delivery request.

**Clause 13.** The system of clause 11 or 12, wherein the number of delivery events includes one delivery event, two delivery events, four delivery events, or 12 delivery events.

**Clause 14.** The system of any of clauses 11-13, wherein each delivery event of the number of delivery events includes one or more strokes.

**Clause 15.** The system of claim any of clauses 11-14, wherein causing performance of each delivery event of the number of delivery events includes, in response to determining that the delivery volume of the delivery event is equal to or greater than a delivery volume of a minimum stroke size and is equal to or lower than a delivery volume of a maximum stroke size, causing performance of the delivery event in one stroke.

**Clause 16.** The system of any of clauses 11-14, wherein causing performance of each delivery event of the number of delivery events includes, in response to determining that the delivery volume of the delivery event is greater than a delivery volume of a maximum stroke size, causing performance of the delivery event in a stroke having the maximum stroke size and one or more additional strokes.

**Clause 17.** The system of clause 16, wherein causing performance of each delivery event of the number of delivery events further includes: determining a remaining requested delivery amount based on a difference between the delivery volume of the delivery event and the delivery volume of the maximum stroke size; and in response to determining that the remaining requested delivery amount is equal to or greater than a delivery volume of a minimum stroke size and is equal to or lower than the delivery volume of the maximum stroke size, causing delivery of the remaining requested delivery amount in one stroke.

**Clause 18.** The system of clause 16, wherein causing performance of each delivery event of the number of delivery events further includes: determining a remaining requested delivery amount based on a difference between the delivery volume of the delivery event and the delivery volume of the maximum stroke size; and

in response to determining that the remaining requested delivery amount is greater than the delivery volume of the maximum stroke size, causing delivery of the delivery volume of the maximum stroke size in one stroke.

**Clause 19.** The system of any of clauses 11-14, wherein causing performance of each delivery event of the number of delivery events includes, in response to determining that (1) the delivery volume of the delivery event is greater than a delivery volume of a maximum stroke size and (2) a difference between the delivery volume of the delivery event and the delivery volume of the maximum stroke size is less than a threshold value, causing performance of the delivery event in one stroke with a stroke size greater than maximum stroke size.

**Clause 20**. The system of any of clauses 11-14, wherein causing performance of each delivery event of the number of delivery events includes, in response to determining that the delivery volume of the delivery event is lower than a delivery volume of a minimum stroke size, determining a total unfulfilled delivery amount for future delivery.

**Clause 21**. A fluid delivery system comprising: a reservoir including a plunger and barrel for storing fluid; a drive system configured to linearly translate the plunger, the drive system including a motor; a motor position sensor configured to measure positions of the motor; one or more processors electrically coupled to the motor and the motor position sensor; and one or more processor-readable storage media storing instructions which, when executed by the one or more processors, cause the one or more processors to perform operations including: determining a requested delivery rate of a request for fluid delivery; determining, based on a range that the requested delivery rate falls in, a corresponding number of delivery events per hour for the request; determining, based on the requested delivery rate and the number of delivery events, a delivery volume of each delivery event of the number of delivery events and an event interval between adjacent deliver events of the number of delivery events; and causing performance of the number of delivery events according to the delivery volume and the event interval.

**Clause 22**. The fluid delivery system of clause 21, wherein the request includes a basal insulin delivery request, a temporary basal insulin delivery request, or a square bolus delivery request.

**Clause 23**. The fluid delivery system of clause 21 or 22, wherein the number of delivery events includes one delivery event, two delivery events, four delivery events, or 12 delivery events.

**Clause 24**. The fluid delivery system of any of clauses 21-23, wherein each delivery event of the number of delivery events includes one or more strokes.

**Clause 25**. The fluid delivery system of any of clauses 21-24, wherein causing performance of each delivery event of the number of delivery events includes, in response to determining that the delivery volume of the delivery event is equal to or greater than a delivery volume of a minimum stroke size of the fluid delivery system and is equal to or lower than a delivery volume of a maximum stroke size, causing performance of the delivery event in one stroke.

**Clause 26**. The fluid delivery system of any of clauses 21-24, wherein causing performance of each delivery event of the number of delivery events includes, in response to determining that the delivery volume of the delivery event is greater than a delivery volume of a maximum stroke size, causing performance of the delivery event in a stroke having the maximum stroke size and one or more additional strokes.

**Clause 27.** The fluid delivery system of clause 26, wherein causing performance of each delivery event of the number of delivery events further includes: determining a remaining requested delivery amount based on a difference between the delivery volume of the delivery event and the delivery volume of the maximum stroke size; and in response to determining that the remaining requested delivery amount is equal to or greater than a delivery volume of a minimum stroke size of the fluid delivery system and is equal to or lower than the delivery volume of the maximum stroke size, causing delivery of the remaining requested delivery amount in one stroke.

**Clause 28.** The fluid delivery system of clause 26, wherein causing performance of each delivery event of the number of delivery events further includes: determining a remaining requested delivery amount based on a difference between the delivery volume of the delivery event and the delivery volume of the maximum stroke size; and in response to determining that the remaining requested delivery amount is greater than the delivery volume of the maximum stroke size, causing delivery of the delivery volume of the maximum stroke size in one stroke.

**Clause 29.** The fluid delivery system of any of clauses 21-24, wherein causing performance of each delivery event of the number of delivery events includes, in response to determining that (1) the delivery volume of the delivery event is greater than a delivery volume of a maximum stroke size and (2) a difference between the delivery volume of the delivery event and the delivery volume of the maximum stroke size is less than a threshold value, causing performance of the delivery event in one stroke with a stroke size greater than maximum stroke size.

**Clause 30.** The fluid delivery system of any of clauses 21-24, wherein causing performance of each delivery event of the number of delivery events includes, in response to determining that the delivery volume of the delivery event is lower than a delivery volume of a minimum stroke size, determining a total unfulfilled delivery amount for future delivery.

Further disclosed herein is the subject-matter of the following items:

1. A processor-implemented method comprising:

determining a requested delivery rate of a request for fluid delivery using a fluid delivery device;
determining, based on a range that the requested delivery rate falls in, a corresponding number of delivery events per hour for the request;
determining, based on the requested delivery rate and the number of delivery events, a delivery volume of each delivery event of the number of delivery events and an event interval between adjacent deliver events of the number of delivery events; and
causing performance of the number of delivery events according to the delivery volume and the event interval.

2. The processor-implemented method of item 1, wherein the request includes a basal insulin delivery request, a temporary basal insulin delivery request, or a square bolus delivery request.

3. The processor-implemented method of item 1 or 2, wherein the number of delivery events includes 1, 2, 3, 4, 5, 6, 7, 8, 12, 24, 48, or 96 delivery events.

4. The processor-implemented method of item 1 or of one of items 1 to 3, wherein each delivery event of the number of delivery events includes one or more strokes.

5. The processor-implemented method of item 1 or of one of items 1 to 4, wherein causing performance of each delivery event of the number of delivery events includes, in response to determining that the delivery volume of the delivery event is equal to or greater than a delivery volume of a minimum stroke size of the fluid delivery device and is equal to or lower than a delivery volume of a maximum stroke size, causing performance of the delivery event in one stroke.

6. The processor-implemented method of item 1 or of one of items 1 to 5, wherein causing performance of each delivery event of the number of delivery events includes, in response to determining that the delivery volume of the delivery event is greater than a delivery volume of a maximum stroke size, causing performance of the delivery event in a stroke having the maximum stroke size and one or more additional strokes.

7. The processor-implemented method of item 6 or of one of items 1 to 6, wherein causing performance of each delivery event of the number of delivery events further includes:

    determining a remaining requested delivery amount based on a difference between the delivery volume of the delivery event and the delivery volume of the maximum stroke size; and in response to determining that the remaining requested delivery amount is equal to or greater than a delivery volume of a minimum stroke size of the fluid delivery device and is equal to or lower than the delivery volume of the maximum stroke size, causing delivery of the remaining requested delivery amount in one stroke.

8. The processor-implemented method of item 6 or of one of items 1 to 7, wherein causing performance of each delivery event of the number of delivery events further includes:

    determining a remaining requested delivery amount based on a difference between the delivery volume of the delivery event and the delivery volume of the maximum stroke size; and in response to determining that the remaining requested delivery amount is greater than the delivery volume of the maximum stroke size, causing delivery of the delivery volume of the maximum stroke size in one stroke.

9. The processor-implemented method of item 1 or of one of items 1 to 8, wherein causing performance of each delivery event of the number of delivery events includes, in response to determining that (1) the delivery volume of the delivery event is greater than a delivery volume of a maximum stroke size and (2) a difference between the delivery volume of the delivery event and the delivery volume of the maximum stroke size is less than a threshold value, causing performance of the delivery event in one stroke with a stroke size greater than maximum stroke size.

10. The processor-implemented method of item 1 or of one of items 1 to 9, wherein causing performance of each delivery event of the number of delivery events includes, in response to determining that the delivery volume of the delivery event is lower than a delivery volume of a minimum stroke size of the fluid delivery device, determining a total unfulfilled delivery amount for future delivery.

11. The processor-implemented method of item 1 or of one of items 1 to 10, wherein:

    the request includes a square bolus delivery request; and causing performance of the number of delivery events according to the delivery volume and the event interval includes delaying the first delivery event of the number of delivery events by a half of the event interval.

12. A system comprising:

    one or more processors; and one or more processor-readable storage media storing instructions which, when executed by the one or more processors, cause performance of operations including:

        determining a requested delivery rate of a request for fluid delivery; determining, based on a range that the requested delivery rate falls in, a corresponding number of delivery events per hour for the request; determining, based on the requested delivery rate and the number of delivery events, a delivery volume of each delivery event of the number of delivery events and an event interval between adjacent deliver events of the number of delivery events; and causing performance of the number of delivery events according to the delivery volume and the event interval.

13. The system of item 12, wherein the request includes a basal insulin delivery request, a temporary basal insulin delivery request, or a square bolus delivery request.

14. The system of item 12 or 13, wherein each delivery event of the number of delivery events includes one or more strokes.

15. The system of item 12 or of one of items 12 to 14, wherein causing performance of each delivery event of the number of delivery events includes, in response to determining that the delivery volume of the delivery event is equal to or greater than a delivery volume of a minimum stroke size and is equal to or lower than a delivery volume of a maximum stroke size, causing performance of the delivery event in one stroke.

16. The system of item 12 or of one of items 12 to 15, wherein causing performance of each delivery event of the number of delivery events includes, in response to determining that the delivery volume of the delivery event is greater than a delivery volume of a maximum stroke size, causing performance of the delivery event in a stroke having the maximum stroke size and one or more additional strokes.

17. The system of item 16 or of one of items 12 to 16, wherein causing performance of each delivery event of the number of delivery events further includes:

determining a remaining requested delivery amount based on a difference between the delivery volume of the delivery event and the delivery volume of the maximum stroke size; and in response to determining that the remaining requested delivery amount is equal to or greater than a delivery volume of a minimum stroke size and is equal to or lower than the delivery volume of the maximum stroke size, causing delivery of the remaining requested delivery amount in one stroke.

18. The system of item 16 or of one of items 12 to 17, wherein causing performance of each delivery event of the number of delivery events further includes:

determining a remaining requested delivery amount based on a difference between the delivery volume of the delivery event and the delivery volume of the maximum stroke size; and

in response to determining that the remaining requested delivery amount is greater than the delivery volume of the maximum stroke size, causing delivery of the delivery volume of the maximum stroke size in one stroke.

19. The system of item 11 or of one of items 12 to 18, wherein causing performance of each delivery event of the number of delivery events includes, in response to determining that (1) the delivery volume of the delivery event is greater than a delivery volume of a maximum stroke size and (2) a difference between the delivery volume of the delivery event and the delivery volume of the maximum stroke size is less than a threshold value, causing performance of the delivery event in one stroke with a stroke size greater than maximum stroke size.

20. A fluid delivery system comprising:

a reservoir including a plunger and barrel for storing fluid;
a drive system configured to linearly translate the plunger, the drive system including a motor;
a motor position sensor configured to measure positions of the motor;
one or more processors electrically coupled to the motor and the motor position sensor; and
one or more processor-readable storage media storing instructions which, when executed by the one or more processors, cause the one or more processors to perform operations including:

determining a requested delivery rate of a request for fluid delivery;
determining, based on a range that the requested delivery rate falls in, a corresponding number of delivery events per hour for the request;
determining, based on the requested delivery rate and the number of delivery events, a delivery volume of each delivery event of the number of delivery events and an event interval between adjacent deliver events of the number of delivery events; and
causing performance of the number of delivery events according to the delivery volume and the event interval.

Claims

1. A processor-implemented method comprising:

determining a requested delivery rate of a request for fluid delivery using a fluid delivery device;
determining, based on a range that the requested delivery rate falls in, a corresponding number of delivery events per hour for the request;

determining, based on the requested delivery rate and the number of delivery events, a delivery volume of each delivery event of the number of delivery events and an event interval between adjacent deliver events of the number of delivery events; and

causing performance of the number of delivery events according to the delivery volume and the event interval.

2. The processor-implemented method of claim 1, wherein the request includes a basal insulin delivery request, a temporary basal insulin delivery request, or a square bolus delivery request.

3. The processor-implemented method of claim 1 or 2, wherein the number of delivery events includes 1, 2, 3, 4, 5, 6, 7, 8, 12, 24, 48, or 96 delivery events, and/or wherein each delivery event of the number of delivery events includes one or more strokes.

4. The processor-implemented method of one of the claims 1 to 3, wherein causing performance of each delivery event of the number of delivery events includes, in response to determining that the delivery volume of the delivery event is equal to or greater than a delivery volume of a minimum stroke size of the fluid delivery device and is equal to or lower than a delivery volume of a maximum stroke size, causing performance of the delivery event in one stroke.

5. The processor-implemented method of one of the claims 1 to 4, wherein causing performance of each delivery event of the number of delivery events includes, in response to determining that the delivery volume of the delivery event is greater than a delivery volume of a maximum stroke size, causing performance of the delivery event in a stroke having the maximum stroke size and one or more additional strokes.

6. The processor-implemented method of claim 5, wherein causing performance of each delivery event of the number of delivery events further includes:

determining a remaining requested delivery amount based on a difference between the delivery volume of the delivery event and the delivery volume of the maximum stroke size; and in response to determining that the remaining requested delivery amount is equal to or greater than a delivery volume of a minimum stroke size of the fluid delivery device and is equal to or lower than the delivery volume of the maximum stroke size, causing delivery of the remaining requested delivery amount in one stroke, and/or wherein causing performance of each delivery event of the number of delivery events further includes:

determining a remaining requested delivery amount based on a difference between the delivery volume of the delivery event and the delivery volume of the maximum stroke size; and in response to determining that the remaining requested delivery amount is greater than the delivery volume of the maximum stroke size, causing delivery of the delivery volume of the maximum stroke size in one stroke.

7. The processor-implemented method of one of the claims 1 to 6, wherein causing performance of each delivery event of the number of delivery events includes, in response to determining that (1) the delivery volume of the delivery event is greater than a delivery volume of a maximum stroke size and (2) a difference between the delivery volume of the delivery event and the delivery volume of the maximum stroke size is less than a threshold value, causing performance of the delivery event in one stroke with a stroke size greater than maximum stroke size, and/or wherein causing performance of each delivery event of the number of delivery events includes, in response to determining that the delivery volume of the delivery event is lower than a delivery volume of a minimum stroke size of the fluid delivery device, determining a total unfulfilled delivery amount for future delivery.

8. The processor-implemented method of one of the claims 1 to 7, wherein:

the request includes a square bolus delivery request; and causing performance of the number of delivery events according to the delivery volume and the event interval includes delaying the first delivery event of the number of delivery events by a half of the event interval.

9. A system comprising:

one or more processors; and one or more processor-readable storage media storing instructions which, when executed by the one or more processors, cause performance of operations including:

determining a requested delivery rate of a request for fluid delivery;

determining, based on a range that the requested delivery rate falls in, a corresponding number of delivery events per hour for the request;

determining, based on the requested delivery rate and the number of delivery events, a delivery volume of each delivery event of the number of delivery events and an event interval between adjacent deliver events of the number of delivery events; and

causing performance of the number of delivery events according to the delivery volume and the event interval.

10. The system of claim 9, wherein the request includes a basal insulin delivery request, a temporary basal insulin delivery request, or a square bolus delivery request,

and/or

wherein each delivery event of the number of delivery events includes one or more strokes.

11. The system of claim 9 or 10, wherein causing performance of each delivery event of the number of delivery events includes, in response to determining that the delivery volume of the delivery event is equal to or greater than a delivery volume of a minimum stroke size and is equal to or lower than a delivery volume of a maximum stroke size, causing performance of the delivery event in one stroke.

12. The system of one of the claims 9 to 11, wherein causing performance of each delivery event of the number of delivery events includes, in response to determining that the delivery volume of the delivery event is greater than a delivery volume of a maximum stroke size, causing performance of the delivery event in a stroke having the maximum stroke size and one or more additional strokes.

13. The system of claim 12, wherein causing performance of each delivery event of the number of delivery events further includes:

determining a remaining requested delivery amount based on a difference between the delivery volume of the delivery event and the delivery volume of the maximum stroke size; and

in response to determining that the remaining requested delivery amount is equal to or greater than a delivery volume of a minimum stroke size and is equal to or lower than the delivery volume of the maximum stroke size, causing delivery of the remaining requested delivery amount in one stroke,

and/or

wherein causing performance of each delivery event of the number of delivery events further includes:

determining a remaining requested delivery amount based on a difference between the delivery volume of the delivery event and the delivery volume of the maximum stroke size; and

in response to determining that the remaining requested delivery amount is greater than the delivery volume of the maximum stroke size, causing delivery of the delivery volume of the maximum stroke size in one stroke.

14. The system of one of the claims 9 to 13, wherein causing performance of each delivery event of the number of delivery events includes, in response to determining that (1) the delivery volume of the delivery event is greater than a delivery volume of a maximum stroke size and (2) a difference between the delivery volume of the delivery event and the delivery volume of the maximum stroke size is less than a threshold value, causing performance of the delivery event in one stroke with a stroke size greater than maximum stroke size.

15. A fluid delivery system comprising:

a reservoir including a plunger and barrel for storing fluid;

a drive system configured to linearly translate the plunger, the drive system including a motor;

a motor position sensor configured to measure positions of the motor;

one or more processors electrically coupled to the motor and the motor position sensor; and

one or more processor-readable storage media storing instructions which, when executed by the one or more processors, cause the one or more processors to perform operations including:

determining a requested delivery rate of a request for fluid delivery;

determining, based on a range that the requested delivery rate falls in, a corresponding number of delivery events per hour for the request;

determining, based on the requested delivery rate and the number of delivery events, a delivery volume of each delivery event of the number of delivery events and an event interval between adjacent deliver events of the number of delivery events; and

causing performance of the number of delivery events according to the delivery volume and the event interval.

**FIG. 1**

**FIG. 2**

EP 4 693 325 A1

300

310

330

320

**FIG. 3**

FIG. 4

**FIG. 5**

*FIG. 6*

700

Manufacturing calibration (e.g., to determine manufacturing motor voltage (MMV) and manufacturing duty cycle (MDC) under a target load, and manufacturing delivery rate (MDR))
710

Reservoir Setup 720

Determine a baseline coefficient of variation of rotation time (or delivery rate) with no load
722

Detect plunger location and insulin volume
724

Measure and compensate reservoir friction variation by adjusting motor drive voltage and/or duty cycle
726

Determine a baseline coefficient of variation of delivery rate at calibrated motor drive voltage and duty cycle and different stroke sizes
728

Fluid (e.g., insulin) Delivery 730

Delivery scheduling
732

Fluid delivery
734

Occlusion Detection
736

FIG. 7

800

```
┌─────────────────────────────────────────────┐
│         Set slide to home position          │
│                    810                      │
└─────────────────────────────────────────────┘
                       │
                       ▼
┌─────────────────────────────────────────────┐
│        Drive slide forward to a position     │◄──┐
│                    820                      │   │
└─────────────────────────────────────────────┘   │
                       │                           │
                       ▼                           │
┌─────────────────────────────────────────────┐   │
│ Determine motor drive voltage and motor drive│   │
│ duty cycle for producing a target stall load │   │
│                    830                      │   │
└─────────────────────────────────────────────┘   │
                       │                           │
                       ▼            840            │
        ◇ Difference between motor drive ◇──N──┐   │
        ◇ voltages at different positions ◇    │   │
        ◇ within a threshold?           ◇    ▼   │
                       │              ┌──────────┐│
                       Y              │Calibration││
                       ▼              │  fails   ││
┌─────────────────────────────────────┐ 845    ││
│      Determine motor voltage midpoint│└──────────┘│
│                    850              │            │
└─────────────────────────────────────┘            │
                       │            860             │
                       ▼                            │
        ◇ Motor voltage midpoint ◇──N──┐           │
        ◇ greater than a low threshold?◇  ▼         │
                       │        ┌──────────┐        │
                       Y        │Set min MMV│        │
                       ▼        │   865    │        │
┌─────────────────────────────┐└──────────┘        │
│Determine and store the final │      │             │
│midpoint motor drive voltage  │◄─────┘             │
│(as manufacturing motor voltage│                   │
│(MMV)) and motor drive duty    │                   │
│cycle (as the manufacturing    │                   │
│duty cycle (MDC))              │                   │
│              870             │                   │
└─────────────────────────────┘                   │
                       │                           │
                       ▼                           │
┌─────────────────────────────────────────────┐   │
│Determine and store manufacturing delivery    │   │
│rate at MMV and MDC with no load              │   │
│                    880                      │   │
└─────────────────────────────────────────────┘   │
```

*FIG. 8*

900

Drive motor steadily using manufacturing motor voltage and manufacturing duty cycle
910

Measure motor rotation time (MRT)
920

Save the MRT to a buffer
930

935
Buffer Full?

N

Y

Determine a coefficient of variation of rotation time
940

942
Number of steps > a threshold?

N

Determine a baseline coefficient of variation of rotation time
950

Y

Proceed to Plunger seek

*FIG. 9*

1000

Drive motor steadily using manufacturing motor voltage and manufacturing duty cycle
1010

Measure motor rotation time (MRT)
1020

1030
MRT > previous maximum rotation time (RTM)?

Y

N

MRT< RTM for a threshold number of consecutive rotations?
1032

N

Y

Determine and save an RTM to a buffer
1036

Reduce RTM
1034

1038
Buffer Full?

N

Y

Determine a coefficient of variation (CV) of RTM and a change of the CV of RTM with respect to the baseline CV of rotation time
1040

1042
Change of CV > a threshold?

N

Y

Plunger detected, stop motor
1050

Proceed to friction variation measurement and compensation

*FIG. 10*

1100

Set motor drive voltage and duty cycle to manufacturing motor voltage and
manufacturing duty cycle, respectively
1110

In each stroke of a plurality of strokes before a compensation buffer is full          1120

Drive the motor for a first number of steps
1122

Drive and determine step interval for each step of a second number of
steps
1124

Determine an instantaneous delivery rate of the current stroke based on the
determined step intervals
1126

Save the instantaneous delivery rate of the current stroke to a buffer
1128

Determine a pre-compensation delivery rate (PDR) based on the instantaneous delivery
rates saved in the buffer
1130

1132

Is the PDR lower
than the manufacturing delivery rate?

N → Compensation
complete
1102

Y

Ⓐ

*FIG. 11*

1100 (Cont'd)

A

Adjust motor drive voltage (e.g., increase by a voltage incremental step or increase based on extrapolation using MDR and PDR) and/or motor drive duty cycle
1140

In each stroke of a plurality of strokes before the compensation buffer is full   1150

Drive the motor for a first number of steps
1152

Drive and determine step interval for each step of a second number of steps
1154

Determine an instantaneous (current) delivery rate of the current stroke based on the determined step intervals
1156

Save the instantaneous delivery rate of the current stroke to a buffer
1158

Determine a reservoir compensation delivery rate (RCR) based on the instantaneous delivery rates stored in the buffer
1160

1162

Y ⟷ Is RCR lower than the manufacturing delivery rate? ⟶ N ⟶ Compensation complete
1102

*FIG. 11 (Cont'd)*

1200

Set motor drive voltage and duty cycle to the motor drive voltage and duty cycle learned
during reservoir compensation
1210

For each stroke size of a plurality of stroke sizes  1220

In each stroke of a plurality of strokes of the stroke size  1222

Drive the motor for a first number of steps
1224

Drive and determine step interval for each step of a second number
of steps
1226

Determine an instantaneous delivery rate of the current stroke based
on the determined step intervals
1228

Save the instantaneous delivery rate of the current stroke to a buffer
1230

Determine a coefficient of variation of the delivery rates as a baseline CV of delivery rate
(DBR)
1240

*FIG. 12*

1300

1302 Basal Delivery Request — Input 1

1304 Square Bolus Delivery Request — Input 2

1306 Regular Bolus Delivery Request — Input 3

1308 Closed loop Delivery Request — Input 4

**Delivery Request Queue (e.g., FIFO) 1310**

Send next request

Single Request Output

1320 Pass Request to Delivery Scheduler

1330 Delivery Complete? — Y / N

*FIG. 13*

1400

Determine remaining requested amount of fluid (e.g., insulin) to be delivered
1410

Determine a stroke size (e.g., number of steps) for the next stroke
1420

Control the motor to start to deliver insulin according to the stroke size
1430

1440

Is motor movement detected? — N → Sensor Error or Stall detected
1450

Y

Determine current delivery rate based on measured step intervals during one or more steps of the stroke
1460

Determine total drive steps and delivered volume in the stroke
1480

Perform occlusion detection based on measured current delivery rate
1470

*FIG. 14*

EP 4 693 325 A1

FIG. 15

Calculate remaining requested delivery (RDR) for a request
1510

1520
RDR≥0?

Determine total excess delivery
1522

Requested delivery completed
1505

1530
RDR ≥ min. stroke size?

Determine total unfulfilled delivery
1532

1540
RDR ≥ max stroke size?

1550
RDR ≤ Max stroke size + a theshold number?

Determine stroke size
1552

Set stroke size to max stroke size
1560

Enable insulin delivery in a stroke
1570

1500

1600

Receive a basal delivery request including a target basal rate (e.g., in units/hour)
1610

Determine a number of delivery events within a time period (e.g., every hour) and the delivery amount for each delivery event
1612

1614
Temp basal?

Determine event timer value
1616

Determine event timer value
1618

1620
Event timer = 0?
N

Y

Start event timer
1622

1624
Event timer value ≥ event interval ?
N

Y

1628
New basal rate?
N

Y

Reset event timer
1626

FIG. 16

1702

Enter desired temporary basal percentage (TEMPperB)

1700

1710
TEMPperB=0%?

Y

N

Calculate temporary basal rate
1720

1722
Temp basal rate ≥
0.05UPH??

N

Error

Y

Calculate the number of temp basal delivery events
1730

1740
number of temp basal
delivery events ≥ 4?

Y

Set minimum duration (e.g., 30 min)
Set duration increment (e.g., 15 min)
1742

N

1750
number of temp basal
delivery events ≥ 2?

Y

Set minimum duration (e.g., 30 min)
Set duration increment (e.g., 30 min)
1752

N

Set minimum duration (e.g., 60 min)
Set duration increment (e.g., 60 min)
1754

Setup complete

*FIG. 17*

1800

Receive bolus amount and duration of a square bolus request
1810

Determine a number of delivery events within the duration and the delivery amount for each delivery event
1820

Set delivery event interval and timers
1830

1840
Bolus timer expired?

Y → Delivery complete
1842

N

1850
Current event interval timer expired?

N

Y

Reset event timer
1860

FIG. 18

FIG. 19A

FIG. 19B

2000

Obtain a baseline coefficient of variation of delivery rate ( DBR, e.g., from block 1240)
2010

Obtain measured current delivery rate (CDR, e.g., from block 1460)
2012

2014

CDR ≥ previous minimum delivery rate (OSM)?

N

Y

No occlusion
2002

Determine and save an OSM to a buffer
2020

2016

CDR ≥ OSM for a threshold number of consecutive steps?

N

Y

2022

Buffer Full?

N

Increase OSM
2018

Y

Determine a coefficient of variation (CV) of delivery rate (BDR) based on the (minimum) delivery rates saved in the buffer
2024

Determine and save a previous rate change (PRC) of BDR with respect to DBR
2026

A

**FIG. 20**

2000 (Cont'd)

FIG. 20 (Cont'd)

2100

Obtain a baseline coefficient of variation of delivery rate ( DBR, e.g., from block 1240)
2010

Determine a maximum temperature OTM of the temperature measurement values in a
temperature buffer
2110

Store a temperature measurement value in the temperature buffer
2112

Increase the current index TBI of the temperature buffer
2114

Reset index TBI
to 0
2118

N ← Is TBI less than a
threshold TBS? 2116

Y

Obtain measured current delivery rate (CDR, e.g., from block 1460)
2012

. . .

*FIG. 21*

From 2024 →

Determine and save a previous rate change (PRC) of BDR with respect to DBR
2026

2100
(Cont'd)

2120
(OTM-PMIC_TEMP)>ODT?

Y
2122
POF=True?

N

N

2128
OTF=True?

N

Y

Set OTF=True
2124

Set DDC=0
2126

Calculate DDC
2130

Set OTF=False
2134

N

2132
DDC<ODD?

Y

2136
BDR>DBR?

N

Set TMF=1
2138

Y

N

2140
OTF=True?

Y

Set TMF=ODM
2142

Y

BDR > TMF*DBR?

N

2144

Determine a current rate change (CRC) of BDR with respect to TMF*DBR
2146

2042
CRC> gross occlusion threshold (GOT)?

2060
Pre-occlusion flag (POF)= true?

. . .

*FIG. 21 (Cont'd)*

. . .

*FIG. 22*

EP 4 693 325 A1

FIG. 23

EP 4 693 325 A1

# EUROPEAN SEARCH REPORT

**Application Number**

EP 25 19 4155

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2002/065509 A1 (LEBEL RONALD J [US] ET AL) 30 May 2002 (2002-05-30) | 1-3,9,10 | INV.<br>G16H20/17 |
| A | * the whole document * | 4-8,<br>11-15 | A61M5/142<br>A61M5/172 |
| A | US 2024/197983 A1 (BREINGAN KYLE [US]) 20 June 2024 (2024-06-20)<br>* the whole document * | 1-15 | ADD.<br>A61B5/00 |
| A | US 2020/197608 A1 (MAZLISH BRYAN [US] ET AL) 25 June 2020 (2020-06-25)<br>* paragraphs [0059], [0195]; figures 1, 2 * | 1-15 | |
| A | US 2020/342974 A1 (CHEN YAMEI [US] ET AL) 29 October 2020 (2020-10-29)<br>* paragraph [0185]; figures 1, 2, 8 * | 1-15 | |
| A | US 2023/201451 A1 (MONIRABBASI SALMAN [US] ET AL) 29 June 2023 (2023-06-29)<br>* the whole document * | 1-15 | |

**TECHNICAL FIELDS SEARCHED (IPC)**

G16H
A61B
A61M

The present search report has been drawn up for all claims

| Place of search | Date of completion of the search | Examiner |
|---|---|---|
| Munich | 20 November 2025 | Lorenz, Larissa |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 25 19 4155

This annex lists the patent family members relating to the patent documents cited in the above-mentioned European search report.
The members are as contained in the European Patent Office EDP file on
The European Patent Office is in no way liable for these particulars which are merely given for the purpose of information.

20-11-2025

| Patent document cited in search report | | Publication date | Patent family member(s) | | Publication date |
|---|---|---|---|---|---|
| US 2002065509 | A1 | 30-05-2002 | US | 6427088 B1 | 30-07-2002 |
| | | | US | 6668196 B1 | 23-12-2003 |
| | | | US | 2001041831 A1 | 15-11-2001 |
| | | | US | 2001041920 A1 | 15-11-2001 |
| | | | US | 2002016568 A1 | 07-02-2002 |
| | | | US | 2002019606 A1 | 14-02-2002 |
| | | | US | 2002049480 A1 | 25-04-2002 |
| | | | US | 2002058906 A1 | 16-05-2002 |
| | | | US | 2002065454 A1 | 30-05-2002 |
| | | | US | 2002065509 A1 | 30-05-2002 |
| | | | US | 2002065540 A1 | 30-05-2002 |
| | | | US | 2002173702 A1 | 21-11-2002 |
| | | | US | 2002173703 A1 | 21-11-2002 |
| | | | US | 2002173830 A1 | 21-11-2002 |
| | | | US | 2002198513 A1 | 26-12-2002 |
| | | | US | 2003009203 A1 | 09-01-2003 |
| | | | US | 2003028079 A1 | 06-02-2003 |
| | | | US | 2003028080 A1 | 06-02-2003 |
| | | | US | 2003028184 A1 | 06-02-2003 |
| | | | US | 2003050535 A1 | 13-03-2003 |
| | | | US | 2003055406 A1 | 20-03-2003 |
| | | | US | 2003065308 A1 | 03-04-2003 |
| | | | US | 2003065370 A1 | 03-04-2003 |
| | | | US | 2003069614 A1 | 10-04-2003 |
| | | | US | 2003176933 A1 | 18-09-2003 |
| | | | US | 2003212441 A1 | 13-11-2003 |
| | | | US | 2004193090 A1 | 30-09-2004 |
| | | | US | 2004210267 A1 | 21-10-2004 |
| | | | US | 2004225338 A1 | 11-11-2004 |
| | | | US | 2005010269 A1 | 13-01-2005 |
| | | | US | 2006173444 A1 | 03-08-2006 |
| | | | US | 2011125095 A1 | 26-05-2011 |
| | | | US | 2011270045 A1 | 03-11-2011 |
| | | | US | 2011275904 A1 | 10-11-2011 |
| | | | US | 2015057518 A1 | 26-02-2015 |
| US 2024197983 | A1 | 20-06-2024 | CN | 120379707 A | 25-07-2025 |
| | | | EP | 4637870 A1 | 29-10-2025 |
| | | | KR | 20250125400 A | 21-08-2025 |
| | | | US | 2024197983 A1 | 20-06-2024 |
| | | | WO | 2024137473 A1 | 27-06-2024 |
| US 2020197608 | A1 | 25-06-2020 | US | 2018200438 A1 | 19-07-2018 |
| | | | US | 2020197608 A1 | 25-06-2020 |
| | | | US | 2023173176 A1 | 08-06-2023 |

EPO FORM P0459

For more details about this annex : see Official Journal of the European Patent Office, No. 12/82

page 1 of 2

## ANNEX TO THE EUROPEAN SEARCH REPORT
## ON EUROPEAN PATENT APPLICATION NO.

EP 25 19 4155

This annex lists the patent family members relating to the patent documents cited in the above-mentioned European search report.
The members are as contained in the European Patent Office EDP file on
The European Patent Office is in no way liable for these particulars which are merely given for the purpose of information.

20-11-2025

| Patent document cited in search report | Publication date | Patent family member(s) | | Publication date |
|---|---|---|---|---|
| US 2020342974 A1 | 29-10-2020 | AU | 2018388898 A1 | 11-06-2020 |
| | | AU | 2022201374 A1 | 24-03-2022 |
| | | AU | 2024202178 A1 | 02-05-2024 |
| | | CA | 3085930 A1 | 27-06-2019 |
| | | CN | 111542884 A | 14-08-2020 |
| | | CN | 118217478 A | 21-06-2024 |
| | | EP | 3729446 A1 | 28-10-2020 |
| | | JP | 7133625 B2 | 08-09-2022 |
| | | JP | 7457072 B2 | 27-03-2024 |
| | | JP | 2021507395 A | 22-02-2021 |
| | | JP | 2022172230 A | 15-11-2022 |
| | | US | 2020342974 A1 | 29-10-2020 |
| | | US | 2024161900 A1 | 16-05-2024 |
| | | US | 2025329432 A1 | 23-10-2025 |
| | | WO | 2019125932 A1 | 27-06-2019 |
| US 2023201451 A1 | 29-06-2023 | AU | 2015355337 A1 | 22-06-2017 |
| | | AU | 2019200150 A1 | 31-01-2019 |
| | | AU | 2020204039 A1 | 09-07-2020 |
| | | CA | 2969217 A1 | 09-06-2016 |
| | | CA | 3096949 A1 | 09-06-2016 |
| | | CN | 106133731 A | 16-11-2016 |
| | | CN | 110101939 A | 09-08-2019 |
| | | DK | 3227804 T3 | 12-07-2021 |
| | | EP | 3227804 A1 | 11-10-2017 |
| | | EP | 3878493 A1 | 15-09-2021 |
| | | JP | 6391836 B2 | 19-09-2018 |
| | | JP | 2018508056 A | 22-03-2018 |
| | | KR | 20170085110 A | 21-07-2017 |
| | | KR | 20200045005 A | 29-04-2020 |
| | | US | 2016158438 A1 | 09-06-2016 |
| | | US | 2017189617 A1 | 06-07-2017 |
| | | US | 2019290844 A1 | 26-09-2019 |
| | | US | 2023201451 A1 | 29-06-2023 |
| | | WO | 2016089701 A1 | 09-06-2016 |

EPO FORM P0459

For more details about this annex : see Official Journal of the European Patent Office, No. 12/82

page 2 of 2

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63680325
- US 5485465 A
- US 7621893 B
- US 5248093 A
- US 8474332 B